# EUROPEAN PATENT APPLICATION

(11) **EP 4 538 279 A2**
(43) Date of publication of application: **16.04.2025**
(21) Application number: 25160777.6
(22) Date of filing: 30.10.2020
(51) Int. Cl.: C07F 7/02

(54) **REACTION AGENT FOR AMIDE REACTIONS AND AMIDE COMPOUND PRODUCTION METHOD USING SAME**

(30) Priority: 30.10.2019 JP 2019197786; 30.10.2019 JP 2019197776; 27.03.2020 JP 2020059087
(62) Divisional of application: 20882761.8
(71) Applicant: Chubu University Educational Foundation, Kasugai-shi Aichi 487-8501 (JP)
(72) Inventor: YAMAMOTO, Hisashi, Aichi (JP); MURAMATSU, Wataru, Aichi (JP); HATTORI, Tomohiro, Aichi (JP)
(74) Representative: Hasegawa, Kan

(57) **Abstract**

[Problem] To provide a novel means capable of generating highly stereoselective and/or highly efficient amidation reactions in a variety of substrates having a carboxyl group and an amino group, and capable of producing amide compounds.

[Solution] A reaction agent for amide reactions between carboxyl groups and amino groups and including a silane compound indicated by general formula (B).

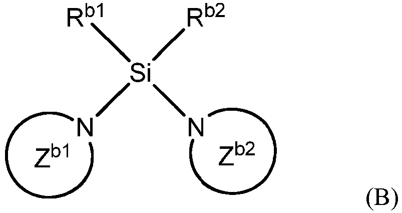

(In general formula (B), each substituent represents the definition described in the Claims.)

## Description

### FIELD

The present invention relates to a reaction agent for amide reaction and a method of producing an amide compound using the same.

### BACKGROUND

Conventionally, amide compounds represented by peptides have been used in a wide variety of fields, including pharmaceuticals, cosmetics, and functional foods. Development of synthetic methods thereof has been diligently pursued as an important research goal in synthetic chemistry (NPL 1 to 6). However, there are few truly effective catalysts or reactants other than carboxylic acid activators for the amidation reaction, which is the most important reaction in peptide synthesis. Therefore, it is unavoidable to use a reaction mode that forms by-products, and thus, peptide synthesis, which involves repeating multi-stage reactions, is extremely inefficient from the viewpoint of atom economy (atomic yield). The amount of by-products is large, and there are few effective purification means. As a result, the cost of disposal of by-products and purification constitutes most of the necessary costs for peptide synthesis, and is the largest obstacle to development in this field.

In peptide synthesis, which uses amino acids or derivatives thereof as starting materials, it is desirable for the amidation reaction to proceed with high stereoselectivity. Enzyme reactions in the body are examples of highly stereoselective amidation reactions. For example, in the body, peptides are synthesized with extremely high stereoselectivity through sophisticated use of enzymes and hydrogen bonds. However, enzyme reactions are not suitable for mass production, requiring enormous financial and time costs when applied to synthetic chemistry.

In synthetic chemistry, amidation reactions using catalysts have been examined, but in conventional means, the amide bond is formed primarily through the method of activating carboxylic acid, such that racemization occurs quickly, whereby synthesizing a peptide with high stereoselectivity and efficiency is difficult.

According to conventional methods, it is very difficult to link an additional amino acid or derivative to a peptide comprising a plurality of amino acids or derivatives thereof (chemical ligation) or link two or more peptides via amide bonds. As an amidation method for ligation to such peptides, there are known a method for ligation by using an amino acid having a sulfur atom to utilize the high reactivity of the sulfur atom (NPL 7) and a method for ligation by synthesizing an amino acid hydroxyamine to utilize the high reactivity of the hydroxyamine (NPL 8). However, in the former method, it is difficult to synthesize amino acids having a sulfur atom, and in the latter method, hydroxyamine synthesis involving several steps is necessary. Both methods are time-consuming and costly and have a disadvantage in efficiency.

The present inventors have developed, as techniques for synthesizing an amide compound in a highly chemoselective manner: a method of amidating a carboxylic acid/ester compound having a hydroxy group at the β-position in the presence of a specific metal catalyst (PTL 1); a method of using a hydroxyamino/imino compound as an amino acid precursor and amidating it in the presence of a specific metal catalyst, and then reducing them in the presence of a specific metal catalyst (PTL 2); and a method of amidating a carboxylic acid/ester compound in the presence of specific metal catalyst (PTL 3).

### CITATION LIST

### Patent Literature (PTL)

[PTL 1] WO 2017/204144 A
[PTL 2] WO 2018/199146 A
[PTL 3] WO 2018/199147 A

### Non-Patent Literature (NPL)

[NPL 1] Annu. Rev. Biophys. Biomol. Struct., 2005, 34, 91-118
[NPL 2] Tetrahedron, 2005, 6, 10827-10852
[NPL 3] Chem. Rev., 2007, 107, 5759-5812
[NPL 4] Chem. Rev., 2011, 111, 6557- 6602
[NPL 5] Org. Process Res. Dev., 2016, 20(2), 140-177
[NPL 6] Chem. Rev., 2016, 116, 12029-12122
[NPL 7] Science, 1992, 256, 221-225
[NPL 8] Angew. Chem. Int. Ed., 2006, 45, 1248-1252

### SUMMARY

### Problem to be Solved

However, there is still a demand to develop a new method that can produce amide compounds by generating amidation reaction in a highly stereoselective manner and/or efficiently for various reactants having carboxyl and amino groups.

Accordingly, an objective of the present invention is to provide a new method of producing amide compounds via amidation reaction in a highly stereoselective manner and/or efficiently using various reactants having carboxyl and amino groups.

### Means for Solving the Problem

As a result of intensive investigations, the present inventors have found that a silane compound having a specific structure has the effect of causing an amidation reaction between a carboxyl group and an amino group, and that such a silane compound can be used as a reaction agent alone or optionally in combination with another secondary silane compound, a Lewis acid catalysts, and/or a phosphorus compound. The present inventors have also found that with the use of such a silane compound, it becomes possible to produce an amide compound via an amidation reactions in a highly stereoselective manner and/or efficiently using various reactants having carboxyl and amino groups, thereby arriving at the present invention.

Thus, the present invention provides the following aspects.

### [Aspect 1]

A reaction agent for amide reaction between a carboxyl group and an amino group, comprising at least one silane compound selected from compounds represented by general formulae (A) and (B).

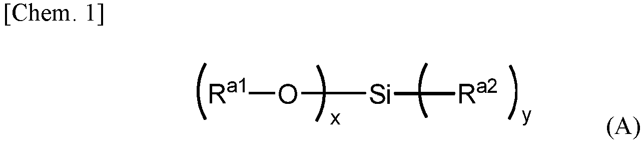

In general formula (A),
R^{a1} represents a monovalent hydrocarbon group substituted with one or more halogen atoms,
R^{a2} represents a hydrogen atom or a 5- to 7-membered heterocyclic group that contains at least one nitrogen atom as a ring-constituting atom and may have one or more substituents,
x represents an integer of 3 or 4, wherein x R^{a1}'s may be either identical to each other or different from each other, and
y represents an integer of 1 or 0, provided that x + y = 4 and that when y is 0, then R^{a2} is absent.

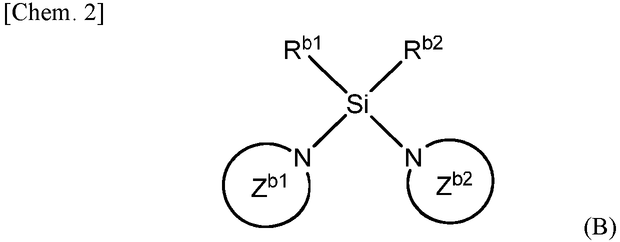

**In** general formula (B),
R^{b1} and R^{b2}, independently of each other, represent a hydrogen atom or an alkyl group or alkoxy group having 1 to 10 carbon atoms or an aryl group, aralkyl group, alkyl aryl group, aryloxy group, aralkyloxy group, or alkyl aryloxy group having 6 to 12 carbon atoms, wherein the alkyl, alkoxy, aryl, aralkyl, alkyl aryl, aryloxy, aralkyloxy, or alkyl aryloxy group may have one or more substituents, and
Z^{b1}and Z^{b2}, independently of each other, represent a 5- to 7-membered heterocyclic group that contains at least one nitrogen atom as a ring-constituting atom and may have one or more substituents.

### [Aspect 2]

A method of producing, from a compound represented by general formula (1-1) and a compound represented by general formula (1-2), an amide compound represented by general formula (1-3), comprising:
causing a reaction between the compound represented by general formula (1-1) and the compound represented by general formula (1-2) in the presence of a silane compound according to Aspect 1.

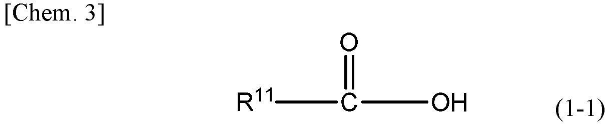

In general formula (1-1),
R¹¹ represents a monovalent hydrocarbon group or heterocyclic group that may have one or more substituents or a monovalent group formed by linking, optionally via a linking group, two or more multivalent hydrocarbon and/or heterocyclic groups that each may have one or more substituents.

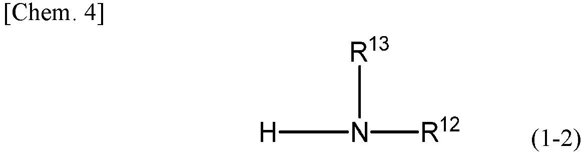

In general formula (1-2),
R¹² represents a monovalent hydrocarbon group or heterocyclic group that may have one or more substituents or a monovalent group formed by linking, optionally via a linking group, two or more multivalent hydrocarbon and/or heterocyclic groups that each may have one or more substituents, and
R¹³ represents a hydrogen atom, carboxyl group, or hydroxyl group, or a monovalent hydrocarbon group or heterocyclic group that may have one or more substituents and may be bound to the nitrogen atom via a linking group, or
R¹² and R¹³ may be bound to each other to form, together with the nitrogen atom to which R¹² and R¹³ bind, a hetero ring that may have one or more substituents.

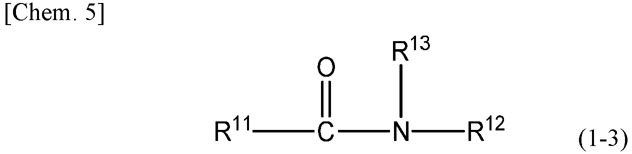

In general formula (1-3), each symbol represents the same definition as that of the same symbol in general formulae (1-1) and (1-2) above.

### [Aspect 3]

A method of producing, from a compound represented by general formula (2-1), an amide compound represented by general formula (2-2), comprising:
causing an intramolecular reaction in the compound represented by general formula (2-1) in the presence of a silane compound according to Aspect 1.

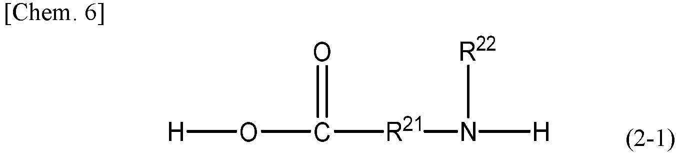

In general formula (2-1),
R²¹ represents a divalent hydrocarbon group or heterocyclic group that may have one or more substituents or a divalent group formed by linking, optionally via a linking group, two or more multivalent hydrocarbon and/or heterocyclic groups that each may have one or more substituents, and
R²² represents a hydrogen atom, carboxyl group, or hydroxyl group, or a monovalent hydrocarbon group or heterocyclic group that may have one or more substituents and may be bound to the nitrogen atom via a linking group, or
R²¹ and R²² may be bound to each other to form, together with the nitrogen atom to which R²¹ and R²² bind, a hetero ring that may have one or more substituents.

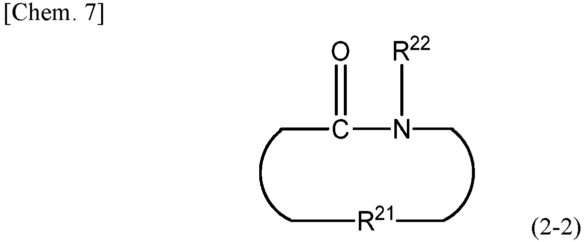

In general formula (2-2), each symbol represents the same definition as that of the same symbol in general formula (2-1) above.

### [Aspect 4]

A method of producing, from a compound represented by general formula (3-1) and a compound represented by general formula (3-2), an amide compound represented by general formula (3-3), comprising:
causing a reaction between the compound represented by general formula (3-1) and the compound represented by general formula (3-2) in the presence of a silane compound according to Aspect 1.

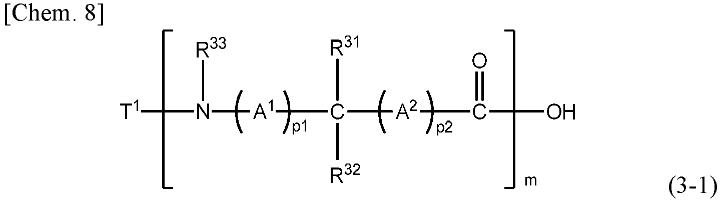

In general formula (3-1),
R³¹ and R³², independently of each other, represent a hydrogen atom, halogen atom, hydroxyl group, carboxyl group, nitro group, cyano group, thiol group, or, a monovalent hydrocarbon group or heterocyclic group that may have one or more substituents and may be bound to the carbon atom via a linking group, and
R³³ represents a hydrogen atom, carboxyl group, hydroxyl group, or, a monovalent hydrocarbon group or heterocyclic group that may have one or more substituents and may be bound to the nitrogen atom via a linking group, or
R³¹ and R³³ may be bound to each other to form, together with the carbon atom to which R³¹ binds and the nitrogen atom to which R³³ binds, a hetero ring that may have one or more substituents,
A¹ and A², independently of each other, represent a divalent aliphatic hydrocarbon group that may have one or more substituents having 1 to 3 carbon atoms,
T¹ represents a hydrogen atom or a monovalent substituent,
p1 and p2, independently of each other, represent an integer of 0 or 1, and
m represents an integer of equal to or greater than 1 corresponding to the number of the structure units parenthesized with [ ], provided that when m is equal to or greater than 2, then the two or more structure units in [ ] may be either identical to each other or different from each other.

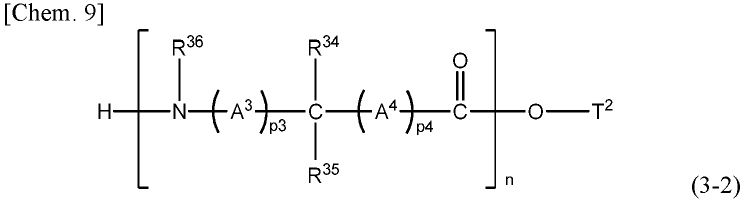

In general formula (3-2),
R³⁴ and R³⁵, independently of each other, represent a hydrogen atom, halogen atom, hydroxyl group, carboxyl group, nitro group, cyano group, thiol group, or, a monovalent hydrocarbon group or heterocyclic group that may have one or more substituents and may be bound to the carbon atom via a linking group,
R³⁶ represents a hydrogen atom, carboxyl group, hydroxyl group, or, a monovalent hydrocarbon group or heterocyclic group that may have one or more substituents and may be bound to the nitrogen atom via a linking group,
R³⁴ and R³⁶ may be bound to each other to form, together with the carbon atom to which R³⁴ binds and the nitrogen atom to which R³⁶ binds, a hetero ring that may have one or more substituents,
A³ and A⁴, independently of each other, represent a divalent aliphatic hydrocarbon group that may have one or more substituents having 1 to 3 carbon atoms,
T² represents a hydrogen atom or a monovalent substituent,
p3 and p4, independently of each other, represent an integer of 0 or 1, and
n represents an integer of equal to or greater than 1 corresponding to the number of the structure units parenthesized with [ ], provided that when n is equal to or greater than 2, then the two or more structure units in [ ] may be either identical to each other or different from each other.

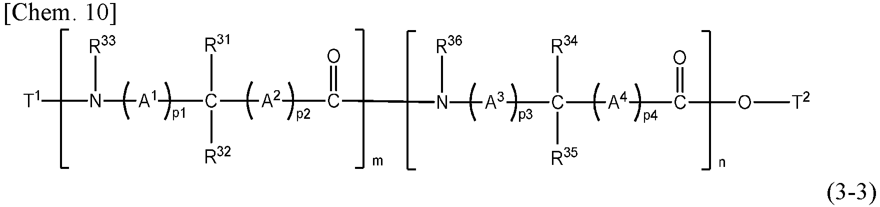

In general formula (3-3), each symbol represents the same definition as that of the same symbol in general formulae (3-1) and (3-2) above.

### [Aspect 5]

The method according to any one of Aspects 2 to 4, wherein the reaction is carried out in the presence of, in addition to the silane compound represented by general formula (A), a second silane compound which is different from the silane compound represented by general formula (A).

### [Aspect 6]

The method according to Aspect 5, wherein the second silane compound is at least one compound selected from compounds represented by general formulae (C1) to (C4).

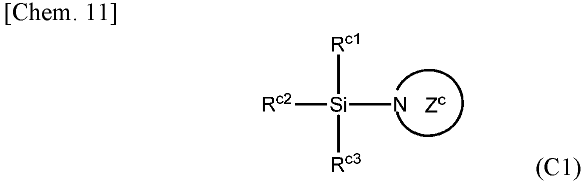

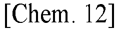

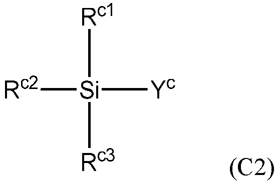

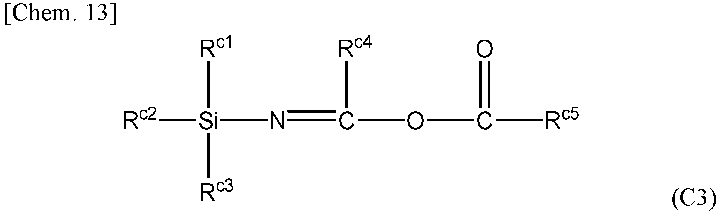

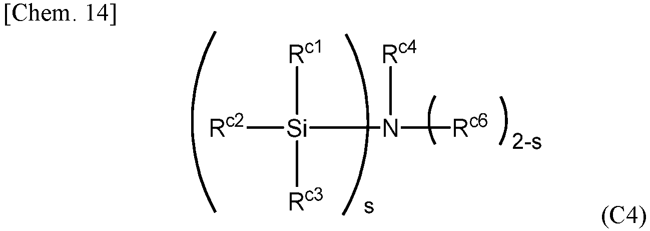

In general formulae (C1) to (C4),
R^{c1} to R^{c3}, independently of each other, represent a hydrogen atom or a linear- or branched-chain alkyl group or alkoxy group that contains 1 to 10 carbon atoms and may have one or more substituents, provided that 0 or 1 of R^{c1} to R^{c3} is a hydrogen atom,
R^{c4} and R^{c5}, independently of each other, represent a linear- or branched-chain alkyl group or alkoxy group that contains 1 to 10 carbon atoms and may have one or more substituents,
Z^{c} represents a 5- to 10-membered heterocyclic group that contains at least one nitrogen atom as a ring-constituting atom and may have one or more substituents,
Y^{c} represents a hydrogen atom or halogen group,
R^{c6} represents a linear- or branched-chain alkyl group, alkoxy group, or alkyl carbonyl group that contains 1 to 10 carbon atoms and may have one or more substituents, and
s represents an integer of 1 or 2, provided that when s is 2, then R^{c6} is absent.

### [Aspect 7]

The method according to any one of Aspects 2 to 6, wherein the reaction is in the presence of an aminosilane catalyst.

### [Aspect 8]

The method according to Aspect 7, wherein the aminosilane catalyst is at least one compound selected from compounds represented by general formula (D).

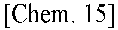

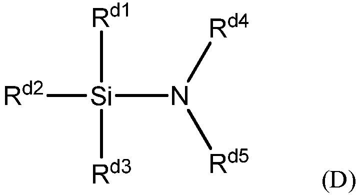

In general formula (D),
R^{d1} to R^{d3}, independently of each other, represent a hydrogen atom or a linear- or branched-chain alkyl group or alkoxy group that may have one or more substituents, provided that 0 or 1 of R^{d1} to R^{d3} is a hydrogen atom, and
R^{d4} and R^{d5}, independently of each other, represent a hydrogen atom or an alkyl group, aryl group, alkyl aryl group, or aryl alkyl group that may have one or more substituents, provided that 0 or 1 of R^{d4} and R^{d5} is a hydrogen atom.

### [Aspect 9]

The method according to any one of Aspects 2 to 8, wherein the reaction is carried out in the presence of a Lewis acid catalyst.

### [Aspect 10]

The method according to Aspect 9, wherein the Lewis acid catalyst is a metal compound containing at least one metal selected from the group consisting of titanium, zirconium, hafnium, tantalum, and niobium.

### [Aspect 11]

The method according to any one of Aspects 2 to 10, wherein the reaction is carried out in the presence of a phosphorus compound.

### [Aspect 12]

The method according to Aspect 11, wherein the phosphorus compound is either a phosphine compound or phosphate compound.

### [Aspect 13]

The method according to any one of Aspects 2 to 12, wherein the reaction is carried out as a batch reaction or a flow reaction.

### Effects of the Invention

The method according to the present invention allows for the production of an amide compound by causing an amidation reaction in the presence of a silane compound having a specific structure as a reaction agent, in a highly stereoselective manner and/or efficiently and using various reactants having carboxyl and amino groups

### EMBODIMENTS

The present invention is described hereinafter in detail with reference to specific embodiments thereof. However, the present invention is not limited to the following embodiments and can be carried out in any embodiment that does not deviate from the gist of the present invention.

All the patent publication, unexamined patent publications, and non-patent literature cited in the present disclosure are incorporated herein by reference in their entireties for any purpose.

### *Definition of Terms:

The term "amino acid" herein refers to a compound having a carboxyl group and an amino group. Unless otherwise specified, the type of an amino acid is not particularly limited. For example, from the viewpoint of optical isomerism, an amino acid may be a D-amino acid or an L-amino acid. From the viewpoint of the relative positions of the carboxyl group and the amino group, an amino acid may be any of an α-amino acid, β-amino acid, γ-amino acid, δ-amino acid, or ε-amino acid. Examples of amino acids include, but are not limited to, natural amino acids that make up proteins. Examples include valine, leucine, isoleucine, alanine, arginine, glutamine, lysine, aspartic acid, glutamic acid, proline, cysteine, threonine, methionine, histidine, phenylalanine, tyrosine, tryptophan, asparagine, glycine, and serine.

The term "peptide" herein refers to a compound comprising a plurality of amino acids linked together via peptide bonds. Unless otherwise specified, the plurality of amino acid units constituting a peptide may be the same type of amino acid unit or may consist of two or more types of amino acid units. The number of amino acids constituting a peptide is not restricted as long as it is two or more. Examples include 2 (also called "dipeptide"), 3 (also called "tripeptide"), 4 (also called "tetrapeptide"), 5 (also called "pentapeptide"), 6, 7, 8, 9, 10, 15, 20, 30, 40, 50, 100, or more.

The term "lactam" herein refers to a compound comprising a ring formed by an intramolecular amidation bond between a carboxyl group and an amino group in a single molecule, and the term "cyclic peptide" herein refers to a peptide comprising a ring formed by an intramolecular amidation bond between a carboxyl group and an amino group (for example, but not limited to, between a terminal carboxyl group and a terminal amino group) in a single peptide molecule.

The term "amino group" herein refers to a functional group represented by any formula of -NH₂, -NRH, and -NRR' (where R and R' each represent a substituent) obtained by removing hydrogen from ammonia, a primary amine, and a secondary amine, respectively.

Unless otherwise specified, a hydrocarbon group herein may be either aliphatic or aromatic. An aliphatic hydrocarbon group may be in the form of either a chain or a ring. A chain hydrocarbon group may be linear or branched. A cyclic hydrocarbon group may be monocyclic, bridged cyclic, or spirocyclic. The hydrocarbon group may be saturated or unsaturated. **In** other words, one, two, or more carbon-carbon double and/or triple bonds may be included. Specifically, "hydrocarbon group" represents a concept including an alkyl group, alkenyl group, alkynyl group, cycloalkyl group, cycloalkenyl group, cycloalkynyl group, aryl group, etc. Unless otherwise specified, one, two, or more hydrogen atoms of the hydrocarbon group may be replaced with any substituents and one, two, or more carbon atoms of the hydrocarbon group may be replaced with any heteroatoms corresponding to the valence thereof.

The term "hydrocarbon oxy group" herein refers to a group comprising an oxy group (-O-) linked via one bond thereof to the hydrocarbon group as defined above.

The term "hydrocarbon carbonyl group" herein refers to a group comprising a carbonyl group (-C(=O)-) linked via one bond thereof to the hydrocarbon group as defined above.

The term "hydrocarbon sulfonyl group" herein refers to a group comprising a sulfonyl group (-S(=O)₂-) linked via one bond thereof to the hydrocarbon group as defined above.

A heterocyclic group may be saturated or unsaturated. **In** other words, it may contain one, two, or more carbon-carbon double and/or triple bonds. A heterocyclic group may be monocyclic, bridged cyclic, or spirocyclic. The heteroatom included in the constituent atoms of the heterocyclic group is not particularly limited, examples thereof including nitrogen, oxygen, sulfur, phosphorus, and silicon.

The term "heterocyclic oxy group" herein refers to a group comprising an oxy group (-O-) linked via one bond thereof to the heterocyclic group as defined above.

The term "heterocyclic carbonyl group" herein refers to a group comprising a carbonyl group (-C(=O)-) linked via one bond thereof to the heterocyclic group as defined above.

The term "heterocyclic sulfonyl group" herein refers to a group comprising a sulfonyl group (-S(=O)₂-) linked via one bond thereof to the heterocyclic group as defined above.

Unless otherwise specified, the term "substituent" herein refers, independently of each other, to any substituent which is not particularly limited so long as the amidation step of the production method according to the present invention proceeds.

Examples include, but are not limited to, a halogen atom, hydroxyl group, carboxyl group, nitro group, cyano group, thiol group, sulfonic acid group, amino group, amide group, imino group, imide group, hydrocarbon group, heterocyclic group, hydrocarbon oxy group, hydrocarbon carbonyl group (acyl group), hydrocarbon oxycarbonyl group, hydrocarbon carbonyl oxy group, hydrocarbon substitution amino group, hydrocarbon substitution amino carbonyl group, hydrocarbon carbonyl substitution amino group, hydrocarbon substitution thiol group, hydrocarbon sulfonyl group, hydrocarbon oxysulfonyl group, hydrocarbon sulfonyl oxy group, heterocyclic oxy group, heterocyclic carbonyl group, heterocyclic oxycarbonyl group, heterocyclic carbonyl oxy group, heterocyclic amino group, heterocyclic amino carbonyl group, heterocyclic carbonyl substitution amino group, heterocyclic substitution thiol group, heterocyclic sulfonyl group, heterocyclic oxysulfonyl group, and heterocyclic sulfonyl oxy group. The term "substituents" also may include functional groups obtained by substituting any of the aforementioned functional groups with any of the aforementioned functional groups as long as the valence and physical properties thereof permit. When a functional group has one or more substituents, the number of the substituents is not particularly limited as long as the valence and physical properties thereof permit. When the functional group has two or more substituents, they may be identical to each other or different from each other.

The term Me herein refers to a methyl group, the term Et herein refers to a ethyl group, the term Pr herein refers to a propyl group, the term i-Pr herein refers to an isopropyl group, the term Bu herein refers to a butyl group, and the term t-Bu herein refers to a Tert-butyl group.

The term Ac herein refers to a acetyl group, the term acac herein refers to an acetylacetonate, the term Cp herein refers to a cyclopentadienyl, the term Tf herein refers to a trifluoromethane sulfonyl, the term Trt herein refers to a trityl group, the term THF herein refers to a tetrahydrofuran, the term DCM herein refers to a dichloromethane, and the term DMSO herein refers to a dimethyl sulfoxide.

Amino acids and residues thereof may herein be represented by three-letter abbreviations well known to a person skilled in the art. The three-letter abbreviations of major amino acids are shown in the following table.

**[Table A]**

| | |
|---|---|
| Ala | Alanine |
| Arg | Arginine |
| Asn | Asparagine |
| Asp | Aspartic acid |
| Cys | Cysteine |
| Gln | Glutamine |
| Glu | glutamic acid |
| Gly | Glycine |
| His | Histidine |
| Ile | Isoleucine |
| Leu | Leucine |
| Lys | Lysine |
| Met | Methionine |
| Phe | Phenylalanine |
| Phg | Phenylglycine |
| Pro | Proline |
| Ser | Serine |
| Thr | Threonine |
| Trp | Tryptophan |
| Tyr | Tyrosine |
| Val | Valine |

β-homoamino acids and residues thereof may herein be represented by "Ho" followed by three-letter abbreviations of corresponding β-amino acids.

The term "reaction agent" for an amide reaction between a carboxyl group and an amino group herein refers to an agent capable of causing or promoting an amide reaction between a carboxyl group and an amino group.

A statement including two or more words linked with a slash ("/") in the listing of the names of compounds or substituents encompass all compound or substituent names obtained by choosing one of the listed words alone, unless otherwise specified or unless the context clearly indicates otherwise. For example, the statement "(2-/3-/4-)methyl imidazole group" refers to a "2-methyl imidazole group, 3-methyl imidazole group, and 4-methyl imidazole group," and the statement "(mono/di/tri/tetra/penta/hexa/hepta/octa/nona)fluoro(butyl/butoxy) group" refers to a "monofluorobutyl group, difluorobutyl group, trifluorobutyl group, tetrafluorobutyl group, pentafluorobutyl group, hexafluorobutyl group, heptafluorobutyl group, octafluorobutyl group, nonafluorobutyl group, monofluorobutoxy group, difluorobutoxy group, trifluorobutoxy group, tetrafluorobutoxy group, pentafluorobutoxy group, hexafluorobutoxy group, heptafluorobutoxy group, octafluorobutoxy group, and nonafluorobutoxy group."

### *Reaction agent according to the present invention (silane compound (A) and/or silane compound (B)):

An aspect of the present invention relates to reaction agent for an amide reaction between a carboxyl group and an amino group (hereinafter also referred to as "reaction agent according to the present invention"), comprising at least one silane compound selected from a silane compound represented by general formula (A) (hereinafter also referred to as a "silane compound (A)") and a silane compound represented by general formula (B) (hereinafter also referred to as a "silane compound (B)").

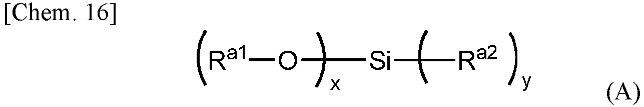

The definition of each symbol in general formula (A) is as follows.

R^{a1} represents a hydrogen or a monovalent hydrocarbon group substituted with one or more halogen atoms. The hydrocarbon group may be either aliphatic or aromatic, or may be a combination thereof. The aliphatic hydrocarbon group may be either saturated or unsaturated, and may be in the form of a ring or a chain. The chain aliphatic hydrocarbon group may be either a linear chain or a branched chain. The number of carbon atoms in the backbone of the hydrocarbon group is not limited, but may typically be from 1 to 10, preferably from 1 to 7, more preferably from 1 to 4.

The number of halogen atoms substituting the hydrocarbon group of R^{a1} is also not limited and may be any number of 1 or more, but may typically be from 1 to 20, preferably from 1 to 16, more preferably from 1 to 12, still more preferably from 1 to 8. The type of halogen atom is also not limited, but may preferably be selected from fluorine, chlorine, bromine, and iodine, more preferably from fluorine, chlorine, or bromine, still more preferably from fluorine or chlorine. When there are two or more halogen atoms, they may be either identical to each other or different from each other.

Preferred examples of the halogen-substituted hydrocarbon groups of R^{a1} include, but are not limited to, (linear- or branched-chain) haloalkyl groups represented by general formula CₘXₙH₍₂ₘ₊₁₋ₙ₎, where m represents any integer equal to or greater than 1, n represents any integer of from 1 to (2m+1), and X represents a halogen atom, provided that when n is 2 or more, the plurality of X's may be either identical to each other or different from each other. Examples of such haloalkyl groups include, but are not limited to: 1-fluoroethyl group, 1-chloroethyl group, 2-fluoroethyl group, 2-chloroethyl group, 1,1-difluoroethyl group, 1,1-dichloroethyl group, 1,2-difluoroethyl group, 1,2-dichloroethyl group, 2,2-difluoroethyl group, 2,2-dichloroethyl group, 1,1,1-trifluoroethyl group, 1,1,1-trichloroethyl group, 2,2,2-trifluoroethyl group, 2,2,2-trichloroethyl group, 2,2,3,3-tetrafluoro n-propyl group, 2,2,3,3-tetrachloro n-propyl group, 3,2,2,3,3-pentafluoro n-propyl group, 3,2,2,3,3-pentachloro n-propyl group, 1,1, 1,3,3,3-hexafluoro isopropyl group, and 1,1,1,3,3,3-hexachloro isopropyl group.

R^{a2} represents a hydrogen atom or a 5- to 10-membered (preferably 5-, 6-, or 10-membered) heterocyclic group that contains at least one (preferably from 2 to 4, more preferably 2 or 3) nitrogen atom as a ring-constituting atom and may have one or more substituents. Each of the substituents, if any, may be selected from those listed above, but may preferably be a halogen atom, more preferably a chlorine atom or a fluorine atom. Examples of the number of substituents are 10, 9, 8, 7, 6, 5, 4, 3, 2, 1, or 0. When there are two or more substituents, they may be either identical to each other or different from each other.

When R^{a2} is a nitrogen-containing heterocyclic group, examples thereof include, but are not limited to: pyrrole group, imidazole group, pyrazole group, triazolegroup (1,2,3-triazolegroup, 1,2,4-triazolegroup), piperidyl group, pyridinyl group, piperazinyl group, tetrazole group, indole group, and benzimidazole group. Preferred among these are imidazole group, pyrazole group, and triazolegroup.

x represents an integer of 3 or 4, and y represents an integer of 1 or 0, provided that x + y = 4. In other words, the number of the halogen substitution hydrocarbon groups of R^{a1} is 3 or 4, but may preferably be 3 (i.e., x may preferably be 3, and y may preferably be 1). x R^{a1}'s are may be either identical to each other or different from each other.

Preferred examples of the silane compound (A) include, but are not limited to: tris(1-fluoroethoxy)silane, tris(1-chloroethoxy)silane, tris(2-fluoroethoxy)silane, tris(2-chloroethoxy)silane, tris(1,1-difluoroethoxy)silane, tris(1,1-dichloroethoxy)silane, tris(1,2-difluoroethoxy)silane, tris(1,2-dichloroethoxy)silane, tris(2,2-difluoroethoxy)silane, tris(2,2-dichloroethoxy)silane, tris(1,1,1-trifluoroethoxy)silane, tris(1,1,1-trichloroethoxy)silane, tris(2,2,2-trifluoroethoxy)silane, tris(2,2,2-trichloroethoxy)silane, tris(2,2,3,3-tetrafluoro n-propoxy)silane, tris(2,2,3,3-tetrachloro n-propoxy)silane, tris(2,2,3,3,3-pentafluoro n-propoxy)silane, tris(2,2,3,3,3-pentachloro n-propoxy)silane, tris(1,1,1,3,3,3-hexafluoroisopropoxy)silane, tris(1,1,1,3,3,3-hexachloro isopropoxy)silane, tetrakis(1,1-difluoroethoxy)silane, tetrakis(1,1-dichloroethoxy)silane, tetrakis(2,2-difluoroethoxy)silane, tetrakis(2,2-dichloroethoxy)silane, tetrakis(1,1,1-trifluoroethoxy)silane, tetrakis(1,1,1-trichloroethoxy)silane, tetrakis(2,2,2-trifluoroethoxy)silane, tetrakis(2,2,2-trichloroethoxy)silane, tetrakis(2,2,3,3-tetrafluoro n-propoxy)silane, tetrakis(2,2,3,3-tetrachloro n-propoxy)silane, tetrakis(2,2,3,3,3-pentafluoro n-propoxy)silane, tetrakis(2,2,3,3,3-pentachloro n-propoxy)silane, tetrakis(1,1,1,3,3,3-hexafluoroisopropoxy)silane, and tetrakis(1,1,1,3,3,3-hexachloro isopropoxy)silane.

More preferred examples of the silane compound (A) include tris(2,2,2-trichloroethoxy)silane, tris(2,2,2-trifluoroethoxy)silane, tris(2,2,3,3-tetrafluoro n-propoxy)silane, and tris(1,1,1,3,3,3-hexafluoroisopropoxy)silane, and especially preferred among them are tris(2,2,2-trifluoroethoxy)silane and tris(1,1,1,3,3,3-hexafluoroisopropoxy)silane.

It is a novel finding previously unknown to the art that the silane compound (A) can function as a reaction agent causing an amidation reaction in one or more reactant compounds having a carboxyl group and/or an amino group, between the carboxyl group and the amino group thereof.

Among the silane compounds (A) mentioned above, those compounds that have 3 R^{a1}-O- groups (each including a halogen-substituted hydrocarbon group of R^{a1}), i.e., the compounds in which x is 3 and y is 1, are novel compounds previously unknown to the art. These novel compounds are also subject to an aspect of the present invention. Examples of such novel silane compounds (A) include tris(1,1,1,3,3,3-hexafluoroisopropoxy)silane, tris(2,2,3,3-tetrafluoro n-propoxy)silane, tris(2,2,2-trichloroethoxy)silane, and tris(2,2,2-trifluoroethoxy)silane.

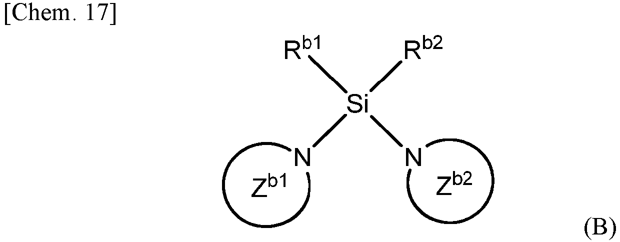

The definition of each symbol in general formula (B) is as follows.

R^{b1} and R^{b2}, independently of each other, represent a hydrogen atom or an alkyl group or alkoxy group having 1 to 10 carbon atoms or an aryl group, aralkyl group, alkyl aryl group, aryloxy group, aralkyloxy group, or alkyl aryloxy group having 6 to 12 carbon atoms, wherein the alkyl, alkoxy, aryl, aralkyl, alkyl aryl, aryloxy, aralkyloxy, or alkyl aryloxy group may have one or more substituents. Each of the substituents, if any, may be selected from those listed above, but may preferably be a halogen atom, more preferably a chlorine atom or a fluorine atom. Examples of the number of substituents are 10, 9, 8, 7, 6, 5, 4, 3, 2, 1, or 0. When there are two or more substituents, they may be either identical to each other or different from each other.

When each of R^{b1} and R^{b2} is an alkyl group that may have one or more substituents, preferred examples thereof include, but are not limited to: methyl group, ethyl group, propyl group (n-propyl group, isopropyl group), butyl group (n-butyl group, tert-butyl group, sec-butyl group, isobutyl group), pentyl group, hexyl group, heptyl group, octyl group, nonyl group, and decyl group, as well as any groups derived from these alkyl groups via substituition with one or more substituents such as chlorine and/or fluorine atoms. When each of R^{b1} and R^{b2} is an alkoxy group that may have one or more substituents, preferred examples thereof include, but are not limited to, any groups obtained by linking each of the examples of alkyl groups that may have one or more substituents mentioned above with an oxy group (-O-).

When each of R^{b1} and R^{b2} is an aryl group, aralkyl group, or alkyl aryl group that may have one or more substituents, preferred examples thereof include, but are not limited to: phenyl group, benzyl group, tolyl group, naphthyl group, and xylyl group, as well as any groups derived from these aryl/aralkyl/alkyl aryl groups via substituition with one or more substituents such as chlorine and/or fluorine atoms. When each of R^{b1} and R^{b2} is an aryloxy group, aralkyloxy group, or alkyl aryloxy group that may have one or more substituents, preferred examples thereof include, but are not limited to, any groups obtained by linking each of the examples of aryl group, aralkyl group, or alkyl aryl groups mentioned above with an oxy group (-O-).

Preferred examples of R^{b1} and R^{b2} include: methyl/methoxy group, ethyl/ethoxy group, propyl/propoxy group, butyl/butoxy group, (mono/di/tri)fluoro(methyl/methoxy) group, (mono/di/tri)chloro(methyl/methoxy) group, (mono/di/tri/tetra/penta)fluoro(ethyl/ethoxy) group, (mono/di/tri/tetra/penta)chloro(ethyl/ethoxy) group, (mono/di/tri/tetra/penta/hexa/hepta)fluoro(propyl/propoxy) group, (mono/di/tri/tetra/penta/hexa/hepta)chloro(propyl/propoxy) group, (mono/di/tri/tetra/penta/hexa/hepta/octa/nona)fluoro(butyl/butoxy) group, (mono/di/tri/tetra/penta/hexa/hepta/octa/nona)chloro(butyl/butoxy) group, and phenyl/phenoxy group.

Z^{b1} and Z^{b2}, independently of each other, represent a 5 to 10-membered (preferably 5-, 6-, or 10-membered) heterocyclic group that contains one or more (preferably 2 to 4, more preferably 2 or 3) nitrogen atoms and may have one or more substituents, ring-constituting atom. Each of the substituents, if any, may be selected from those listed above, but may preferably be selected from alkyl groups (e.g., linear or branched alkyl groups containing 1 to 10 carbon atoms; hereinafter also referred to as -R), alkoxy groups (-O-R), amino groups (-NH₂), alkylamino groups (-NHR), dialkylamino groups (-NR₂: two alkyl groups R's may be either identical to each other or different from each other), and thioalkyl groups (-SR), as well as any groups derived from these groups via substituition with one or more halogen atoms (e.g., bromine or chlorine atoms). Examples of the number of substituents are 10, 9, 8, 7, 6, 5, 4, 3, 2, 1, or 0. When there are two or more substituents, they may be either identical to each other or different from each other.

Examples of nitrogen-containing heterocyclic groups as Z^{b1} and Z^{b2} include, but are not limited to: pyrrole group, imidazole group, pyrazole group, triazolegroup (1,2,3-triazolegroup, 1,2,4-triazolegroup), piperidyl group, pyridinyl group, piperazinyl group,, tetrazole group, indole group, and benzimidazole group as well as any groups derived from these aryl/aralkyl/alkyl aryl groups via substituition with one or more substituents mentioned above, such as (2-/3-/4-/5-)methyl imidazole group and (2,3-/2,4-/2,5-)dimethyl imidazole group. Preferred among them include imidazole group, pyrazole group, triazolegroup, and 2-methyl imidazole group.

Preferred examples of the silane compound (B) include, but are not limited to: dimethyl diimidazole silane, diethyl diimidazole silane, methyl ethyl diimidazole silane, dipropyl diimidazole silane, methyl propyl diimidazole silane, dibutyl diimidazole silane, methyl butyl diimidazole silane, dimethoxy diimidazole silane, diethoxy diimidazole silane, dimethyl dipyrazole silane, diethyl dipyrazole silane, dimethoxy dipyrazole silane, dimethyl di(triazole) silane, diethyl di(triazole) silane, and dimethoxy di(triazole) silane, especially preferred among these being dimethyl diimidazole silane and dimethoxy diimidazole silane.

It is a novel finding previously unknown to the art that the silane compound (B) can function as a reaction agent causing an amidation reaction in one or more reactant compounds having a carboxyl group and/or an amino group, between the carboxyl group and the amino group thereof.

The production method according to the present invention may be carried out using, as a reaction agent, at least one silane compound (A) alone, at least one silane compound (B) alone, both at least one silane compound (A) and at least one silane compound (B), or a combination of at least one silane compound (A) and/or at least one silane compound (B) with at least one second silane compound explained below. In other words, the selection and combination of silane compounds as reaction agents are not restricted in any way, and can be used in any selection and combination.

### *Production method according to the present invention:

Another aspect of the present invention relates to a method of producing an amide compound method (hereinafter also referred to as "the production method according to the present invention") using the silane compound (A) and/or the silane compound (B) as reaction agents. The method includes using one or more compounds having a carboxyl group and/or an amino group as reactants, and causing an amidation reaction between the carboxyl group and the amino group. The production method of the present invention can be broadly classified into the following modes, depending on the types of the reactants used and the target compound produced by the amidation reaction.
(1) A method including using a first compound having a carboxyl group and a second compound having an amino group as reactants and producing a third compound (amide compound) in the presence of the silane compound(s) (A) and/or (B) described above, by causing an intermolecular amidation reaction between the carboxyl group of the first compound and the amino group of the second compound, thereby linking these compounds with an amide bond (hereinafter also referred to as "Embodiment (1)").
(2) A method including using a first compound having a carboxyl group and an amino group as a reactant and producing a second compound (lactam compound) in the presence of the silane compound(s) (A) and/or (B) described above, by causing an intramolecular amidation reaction (lactamization reaction) between the carboxyl group and the amino group of the first compound, thereby cyclizing the first compound with an amide bond (hereinafter also referred to as "Embodiment (2)").

In addition, among specific embodiments included in Embodiment (1) above, the following specific embodiment is especially interesting.

(3) A method including using an amino acid or a peptide as each of the first compound having a carboxyl group and the second compound having an amino group as reactants and producing a peptide compound as the third compound in the presence of the silane compound(s) (A) and/or (B) described above, by causing an intermolecular amidation reaction between the carboxyl group of the first compound and the amino group of the second compound, thereby linking these compounds with an amide bond (hereinafter also referred to as "Embodiment (3)").

Likewise, Embodiment (2) includes a specific embodiment in which the first compound is a peptide and the second compound (lactam compound) is a cyclic peptide.

The combinations of the reactants and the target compounds in Embodiments (1) to (3) are described below.

### *Embodiment (1):

Embodiment (1) relates to a method for using a compound having a carboxyl group represented by formula (1-1) (hereinafter also referred to as "compound (1-1)") and a compound having an amino group represented by formula (1-2) (hereinafter also referred to as "compound (1-2)") as reactants, and producing an amide compound represented by formula (1-3) by causing an intermolecular amidation reaction between the carboxyl group of compound (1-1) and the amino group of compound (1-2) in the presence of a silane compound (A) and/or a silane compound (B) mentioned above.

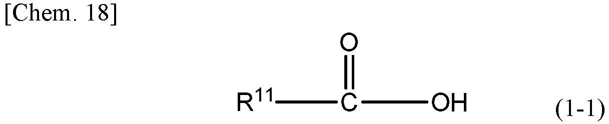

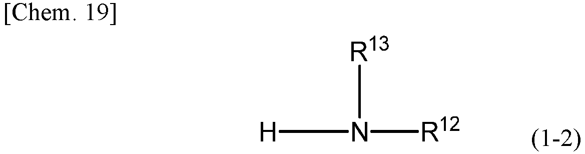

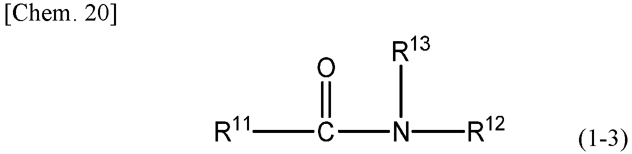

In other words, in this embodiment, the carboxyl and amino groups possessed by the different compounds (1-1) and (1-2) are linked via an amidation reaction to produce a novel amide compound (1-3).

The definition of each symbol in general formulae (1-1), (1-2), and (1-3) are as follows.

R¹¹ and R¹², independently of each other, represent (i) a monovalent hydrocarbon group or heterocyclic group that may have one or more substituents or (ii) a monovalent group formed by linking, optionally via a linking group, two or more multivalent hydrocarbon and/or heterocyclic groups that each may have one or more substituents. Roughly speaking, when each of R¹¹ and/or R¹² is (i), then the compound (1-1) and/or (1-2) may be a monomer or a low molecular compound, while when each of R¹¹ and/or R¹² is
(ii), then the compound (1-1) and/or (1-2) may be a polymer or a macromolecular compound.

When each of R¹¹ and R¹² is, independently of each other, (i) a monovalent hydrocarbon group or heterocyclic group that may have one or more substituents, the details thereof are as follows.

When each of R¹¹ and/or R¹² is a monovalent hydrocarbon group that may have one or more substituents, the number of carbon atoms of the hydrocarbon group (including its substituents, if any) is not particularly limited, but the upper limit thereof may be, for example, 40 or less, 30 or less, 20 or less, 16 or less, or 12 or less. The lower limit thereof depends on the type of the hydrocarbon group, but may be 1 or more for alkyl groups, 2 or more for alkenyl and alkynyl groups, and 3 or more, such as 4 or more, or 5 or more, for cycloalkyl groups. Examples of the number of atoms include, for example, 1, 2, 3, 4, 5, 6, 7, 8, 9, 10, 11, 12, 13, 14, 15, 16, 17, 18, 19, 20, 22, 24, 26, 28, 30, 32, 34, 36, 38, or 40.

When each of R¹¹ and/or R¹² is a monovalent heterocyclic group that may have one or more substituents, the total number of carbon atoms and hetero atoms of the heterocyclic group (including its substituents, if any) is not particularly limited, but the upper limit thereof may be, for example, 20 or less, 15 or less, 10 or less, 8 or less, or 6 or less. The lower limit thereof depends on the type of the heterocyclic structure, but may typically be 3 or more, for example 4 or more, or 5 or more. Examples of the number of atoms include, for example, 3, 4, 5, 6, 7, 8, 9, 10, 11, 12, 13, 14, 15, 16, 17, 18, 19, 20, 22, 24, 26, 28, 30, 32, 34, 36, 38, or 40.

Among them, each of R¹¹ and R¹² may preferably be, independently of each other, an amino group, alkyl group, alkenyl group, cycloalkyl group, alkoxy group, aryl group, aryloxy group, acyl group, heterocyclic group, or heterocyclic oxy group that may have one or more substituents.

Examples of R¹¹ and R¹² include, but are not limited to, the following.
*Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
*Cycloalkyl groups such as cyclopropyl group, cyclobutyl group, cyclopentyl group, cyclohexyl group, cycloheptyl group, bicyclooctyl group, and spirooctyl group;
* Aryl groups such as phenyl group, benzyl group, tolyl group, naphthyl group, and anthracenyl group;
*Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, and carbazolyl group; and
*Heterocyclic oxy groups such as furanyl oxy group, pyrrolyl oxy group, indolyl oxy group, and quinolyl oxy group.

Of the groups mentioned above, those having a carboxyl group may or may not have a protective group. When such a group with a carboxyl group has a protective group, the reaction selectivity with the carboxyl group on the right side of formula (1-1) is usually superior to that with the carboxyl groups present on the other substituents, although it may also depend on the reactivity of the compound (1-1) and the compound (1-2) used in the reaction.

When each of R¹¹ and R¹² is, independently of each other, (ii) a monovalent group formed by linking, optionally via a linking group, two or more multivalent hydrocarbon and/or heterocyclic groups that each may have one or more substituents, the details thereof are as follows.

Such multivalent hydrocarbon groups or heterocyclic groups include multivalent (e.g., di-, tri-, tetra-, or penta-valent, or even higher) hydrocarbon groups or heterocyclic groups obtained by removing one or more hydrogen atoms from the monovalent hydrocarbon or heterocyclic groups mentioned above. When two or more (e.g., two, three, four, five, or more) of these multivalent hydrocarbon or heterocyclic groups are linked together, they may be linked by direct bonds or with intervening linking groups. Such linking groups are not particularly limited, but may be selected, independently of each other, from the structures listed below (where, in the chemical formulae below, A represents a monovalent hydrocarbon group or a heterocyclic group, each of which may independently have one or more substituents. When two A's are present in the same group, they may be identical to each other or different from each other.).

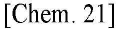

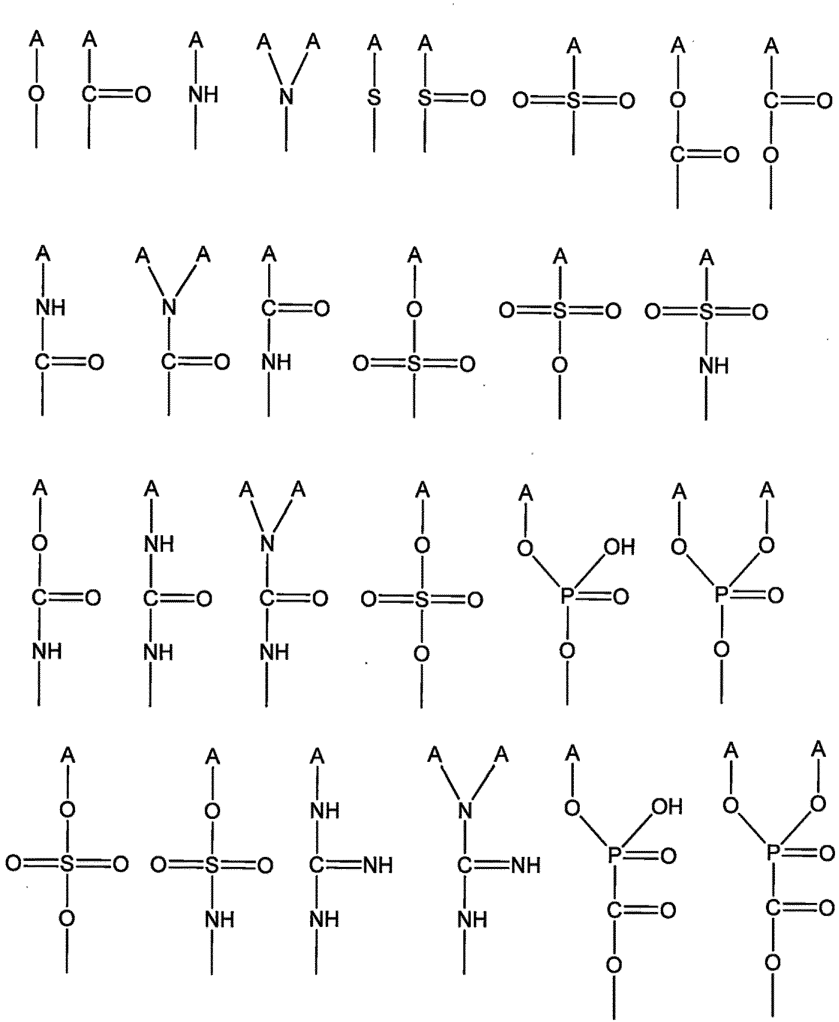

As mentioned above, when each of R¹¹ and/or R¹² is (ii) a monovalent group formed by linking, optionally via a linking group, two or more multivalent hydrocarbon and/or heterocyclic groups that each may have one or more substituents, then the compound (1-1) and/or (1-2) typically is a polymer or a macromolecular compound. Examples of such polymers and macromolecular compounds include, but are not limited to, the following.
*Peptides/proteins (which will be explained in Embodiment (3) below in details);
*Nucleic acids (e.g., DNA (deoxyribonucleic acid) and RNA (ribonucleic acid));
*Polysaccharides (e.g., cellulose, amylose, starch, chitin, and chitosan);
*Complex polysaccharides (e.g., lipopolysaccharides and glycoproteins);
*Lipids (e.g., simple lipids, phospholipids, and glycolipids);
*Natural and synthetic resins (e.g., phenol resins, silicone resins, epoxy resins, melamine resins, urea resins, unsaturated polyester resins, alkyd resins, polyvinyl chloride, polyethylene, polyethylene glycol, polypropylene, polystyrene, polyvinyl acetate, polylactate, polyester, polyurethane, polyamide (e.g., nylon), (meth)acrylic resin, polyvinyl chloride, polyvinylidene chloride, and natural and synthetic rubbers).

R¹³ represents a hydrogen atom, carboxyl group, or hydroxyl group, or a monovalent hydrocarbon group or heterocyclic group that may have one or more substituents. The details of the substituents, if any, are described above. Examples of the number of the substituents include 5, 4, 3, 2, 1, or 0.

When R¹³ is a monovalent hydrocarbon group or heterocyclic group that may have one or more substituents, there may be an intervening linking group between the hydrocarbon group or heterocyclic group and the nitrogen atom to which the hydrocarbon group or heterocyclic group binds. Such linking groups are not particularly limited, but may be selected, independently of each other, from the structures shown below (where, in the chemical formulae below, A represents a monovalent hydrocarbon group or a heterocyclic group, each of which may independently have one or more substituents. When two A's are present in the same group, they may be identical to each other or different from each other).

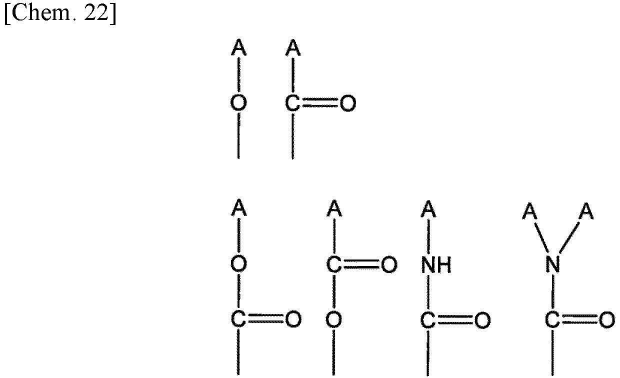

The upper limit of the number of the carbon atoms contained in each hydrocarbon group (including its substituents, if any) may be, for example, 20 or less, 15 or less, 10 or less, 8 or less, or 6 or less. The lower limit thereof depends on the type of the hydrocarbon group, but may be 1 or more for alkyl groups, 2 or more for alkenyl and alkynyl groups, and 3 or more, such as 4 or more, or 5 or more, for cycloalkyl groups. Examples of the number of atoms include, for example, 1, 2, 3, 4, 5, 6, 7, 8, 9, 10, 11, 12, 13, 14, 15, 16, 17, 18, 19, or 20.

The upper limit of the number of the carbon atoms and hetero atoms included in each heterocyclic group (including its substituents, if any) may be, for example, 20 or less, 15 or less, 10 or less, 8 or less, or 6 or less. The lower limit thereof depends on the type of the heterocyclic structure, but may be typically 3 or more, for example 4 or more, or 5 or more. Examples of the number of atoms include, for example, 3, 4, 5, 6, 7, 8, 9, 10, 11, 12, 13, 14, 15, 16, 17, 18, 19, or 20.

R¹² and R¹³ may be bound to each other to form, together with the nitrogen atom to which R¹² and R¹³ bind, a hetero ring that may have one or more substituents. The details of the substituents, if any, are described above. Examples of the number of such substituents include, for example, 5, 4, 3, 2, 1, or 0.

The upper limit of the total number of carbon atoms and hetero atoms included in each heterocyclic group (including its substituents, if any) may be, for example 20 or less, 15 or less, 10 or less, 8 or less, or 6 or less. The lower limit thereof depends on the type of the heterocyclic structure, but may be typically 3 or more, for example 4 or more, or 5 or more. Examples of the number of atoms include, for example, 3, 4, 5, 6, 7, 8, 9, 10, 11, 12, 13, 14, 15, 16, 17, 18, 19, or 20.

Examples of such hetero rings include, but are not limited to, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, and 2,5-dihydro-1,3-thiazolyl group.

### *Embodiment (2):

Embodiment (1) relates to a method for using a compound having a carboxyl group and an amino group represented by formula (2-1) (hereinafter also referred to as "compound (2-1)") as a reactant, and producing a lactam compound represented by formula (2-2) by causing an intramolecular amidation reaction between the carboxyl group and the amino group of compound (2-1) in the presence of a silane compound (A) and/or a silane compound (B) mentioned above.

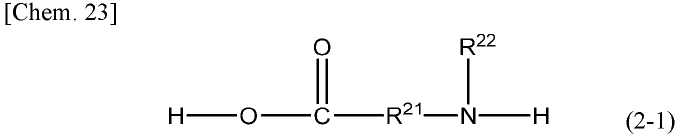

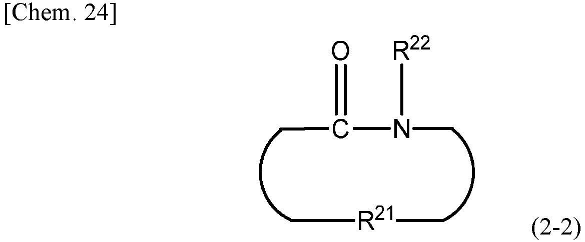

In other words, in this embodiment, the carboxyl and amino groups possessed by the same compound (2-1) are linked via an intramolecular amidation reaction to cause cyclization and produce a novel lactam compound (2-2).

The definition of each symbol in general formulae (2-1) and (2-2) are as follows.

R²¹ represents (i) a divalent hydrocarbon group or heterocyclic group that may have one or more substituents or (ii) a divalent group formed by linking, optionally via a linking group, two or more multivalent hydrocarbon and/or heterocyclic groups that each may have one or more substituents. Roughly speaking, when R²¹ is (i), then the compound (2-1) may be a monomer or a low molecular compound, while when R²¹ is (ii), then the compound (2-1) may be a polymer or a macromolecular compound.

When R²¹ is (i) a divalent hydrocarbon group or heterocyclic group that may have one or more substituents, examples thereof include divalent hydrocarbon or heterocyclic groups obtained by removing any hydrogen atom from the monovalent hydrocarbon or heterocyclic groups described for R¹¹ in formula (1-1) and R¹² in formula (1-2) above. Other details are the same as those described for R¹¹ in formula (1-1) and R¹² in formula (1-2) above.

On the other hand, when R²¹ is (ii) a divalent group formed by linking, optionally via a linking group, two or more multivalent hydrocarbon and/or heterocyclic groups that each may have one or more substituents, examples thereof include divalent groups obtained by removing any hydrogen atom from the monovalent groups including two or more multivalent hydrocarbon groups or heterocyclic groups linked together described for R¹¹ in formula (1-1) and R¹² in formula (1-2) above. Other details are the same as those described for R¹¹ in formula (1-1) and R¹² in formula (1-2) above.

R²² represents a hydrogen atom, carboxyl group, hydroxyl group, or a monovalent hydrocarbon group or heterocyclic group that may have one or more substituents. Details of R²² are the same as those described for R¹³ in formula (1-2) above.

R²¹ and R²² may be bound to each other to form, together with the nitrogen atom to which R²¹ and R²² bind, a hetero ring that may have one or more substituents. The details of such a hetero ring are the same as those described for the hetero ring formed by the bonding of R¹² and R¹³ in formula (1-2) above.

### *Embodiment (3):

Embodiment (3) relates to a method for using an amino acid or peptide represented by formula (3-1) (hereinafter also referred to as "compound (3-1)") and an amino acid or peptide represented by formula (3-2) (hereinafter also referred to as "compound (3-2)") as reactants, and producing a peptide represented by formula (3-3) by causing an intermolecular amidation reaction between the terminal carboxyl group of compound (3-1) and the terminal amino group of compound (3-2) in the presence of a silane compound (A) and/or a silane compound (B) mentioned above.

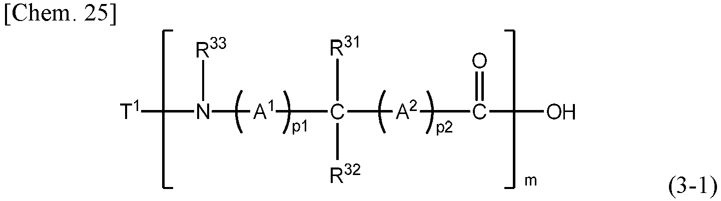

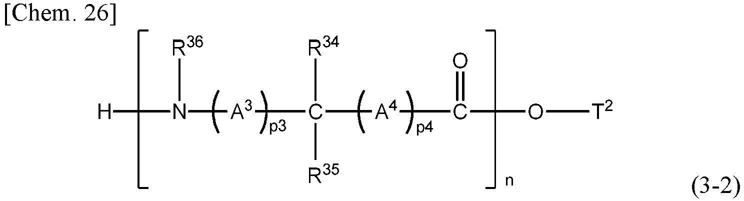

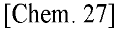

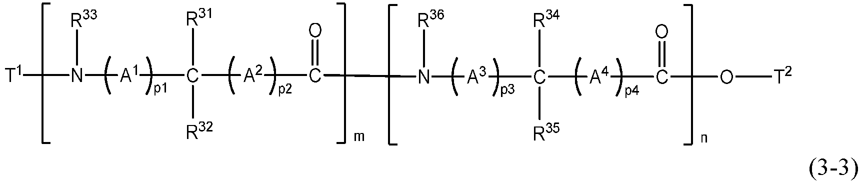

In other words, in this embodiment, the carboxyl and amino groups possessed by the different peptides (3-1) and (3-2) are linked via an amidation reaction to produce a novel peptide (3-3).

The definition of each symbol in general formulae (3-1), (3-2), and (3-3) are as follows.

R³¹, R³², R³⁴, and R³⁶, independently of each other, represent a hydrogen atom, halogen atom, hydroxyl group, carboxyl group, nitro group, cyano group, or thiol group, or a monovalent hydrocarbon group or heterocyclic group that may have one or more substituents. The details of the substituents, if any, are described above. Examples of the number of such substituents include, for example, 5, 4, 3, 2, 1, or 0.

When each of R³¹, R³², R³⁴, and/or R³⁶ is a monovalent hydrocarbon group or heterocyclic group that may have one or more substituents, then there may be an intervening linking group between the hydrocarbon group or heterocyclic group and the carbon atom to which the hydrocarbon group or heterocyclic group binds. Such linking groups are not limited, but may be selected, independently of each other, from the structures indicated below (where, in the chemical formulae below, A represents a monovalent hydrocarbon group or a heterocyclic group, each of which may independently have one or more substituents. When two A's are present in the same group, they may be identical to each other or different from each other).

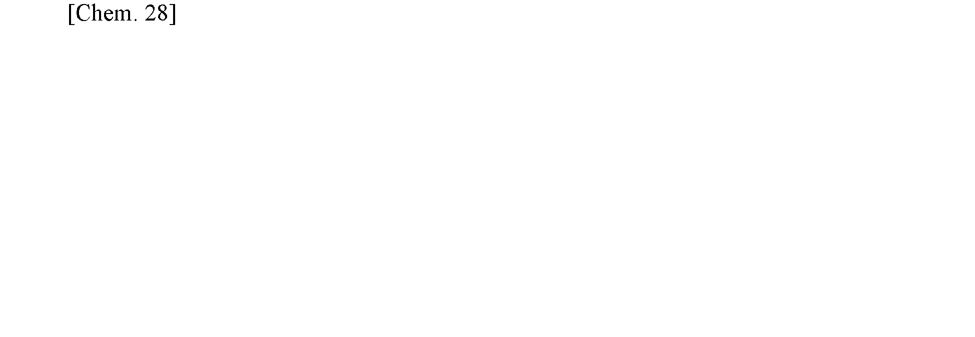

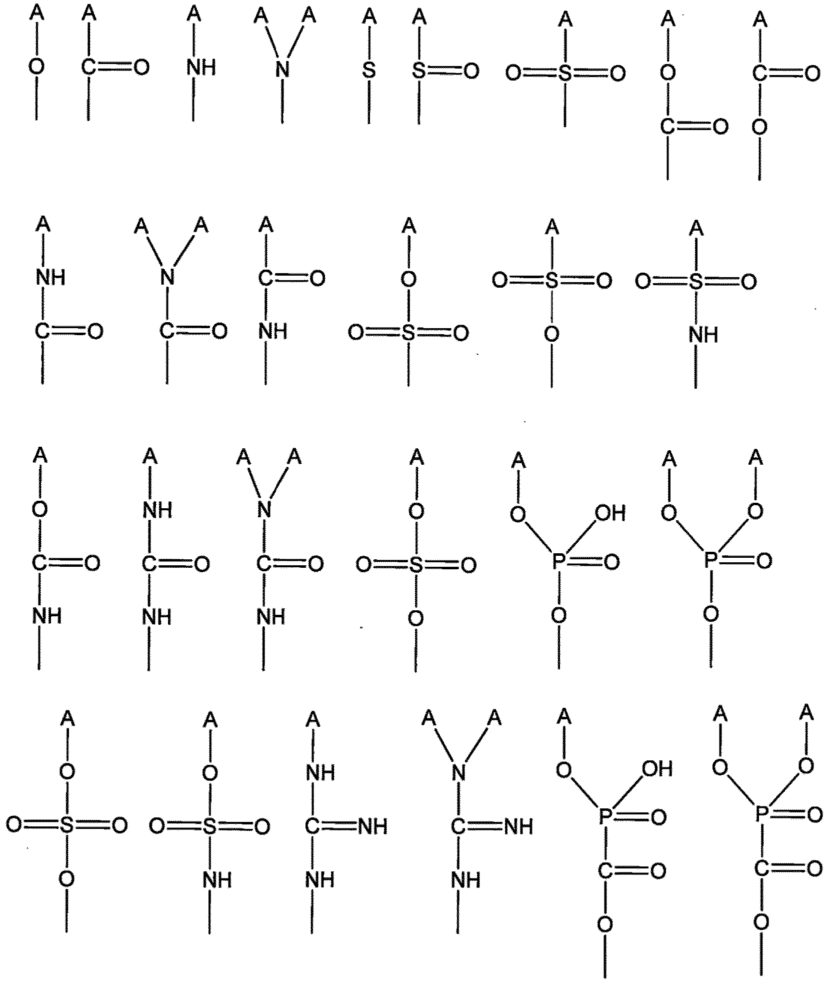

The number of carbon atoms contained in each hydrocarbon group (including its substituents, if any) is not particularly limited, but the upper limit thereof may be, for example, 20 or less, 15 or less, 10 or less, 8 or less, or 6 or less. The lower limit thereof depends on the type of the hydrocarbon group, but may be 1 or more for alkyl groups, 2 or more for alkenyl and alkynyl groups, and 3 or more, such as 4 or more, or 5 or more, for cycloalkyl groups. Examples of the number of atoms include, for example, 1, 2, 3, 4, 5, 6, 7, 8, 9, 10, 11, 12, 13, 14, 15, 16, 17, 18, 19, or 20.

The upper limit of the total number of carbon atoms and hetero atoms included in each heterocyclic group (including its substituents, if any) may be, for example 20 or less, 15 or less, 10 or less, 8 or less, or 6 or less. The lower limit thereof depends on the type of the heterocyclic structure, but may be typically 3 or more, for example 4 or more, or 5 or more. Examples of the number of atoms include, for example, 3, 4, 5, 6, 7, 8, 9, 10, 11, 12, 13, 14, 15, 16, 17, 18, 19, or 20.

Among them, each of R³¹, R³², R³⁴, and R³⁶ may preferably be selected, independently of each other, from hydrogen atom, hydroxyl group, thiol group, carboxyl group, nitro group, cyano group, or halogen atom, or, that may have one or more substituents, amino group, alkyl group, alkenyl group, cycloalkyl group, alkoxy group, aryl group, aryloxy group, acyl group, heterocyclic group, and heterocyclic oxy group.

Examples of R³¹, R³², R³⁴, and R³⁶ include, but are not limited to, the following.
*Hydrogen atom, hydroxyl group, thiol group, carboxyl group, nitro group, and cyano group;
*Halogen atoms such as fluorine atom, chlorine atom, bromine atom, and iodine atom;
*Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
*Alkenyl groups such as ethenyl group, propenyl group, allyl group, butenyl group, pentenyl group, hexenyl group, heptenyl group, and octenyl group;
*Alkylyl groups such as propargyl group;
*Cycloalkyl groups such as cyclopropyl group, cyclobutyl group, cyclopentyl group, cyclohexyl group, cycloheptyl group, bicyclooctyl group, and spirooctyl group;
*Alkoxy groups such as methoxy group, ethoxy group, propoxy group, butoxy group, sec-butoxy group, and tert-butoxy group;
* Aryl groups such as phenyl group, benzyl group, tolyl group, naphthyl group, and anthracenyl group;
*Aryloxy groups such as phenyloxy group, benzyloxy group, and naphthyloxy group;
*Acyl groups such as acetyl group, propionyl group, benzoyl group, p-methoxybenzoyl group, and cinnamoyl group;
*Unsubstituted amino group and substitution amino groups such as dimethyl amino group, benzyl amino group, and triphenylmethyl amino group;
*Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, carbazolyl group; and
*Heterocyclic oxy groups such as furanyl oxy group, pyrrolyl oxy group, indolyl oxy group, and quinolyl oxy group.

Of the groups mentioned above, those having a carboxyl group may or may not have a protective group. When such a group with a carboxyl group has a protective group, the reaction selectivity with the carboxyl group on the right side of formula (3-1) is usually superior to that with the carboxyl groups present on the other substituents, although it may also depend on the reactivity of the compound (3-1) and the compound (3-2) used in the reaction.

R³³ and R³⁶, independently of each other, represent a hydrogen atom, carboxyl group, or hydroxyl group, or, a monovalent hydrocarbon group or heterocyclic group that may have one or more substituents. The details of the substituents, if any, are described above. Examples of the number of such substituents include, for example, 5, 4, 3, 2, 1, or 0.

When each of R³³ and/or R³⁶ is a monovalent hydrocarbon group or heterocyclic group that may have one or more substituents, then there may be an intervening linking group between the hydrocarbon group or heterocyclic group and the carbon atom to which the hydrocarbon group or heterocyclic group binds. Such linking group are not limited, but may be selected, independently of each other, from the structures indicated below (where, in the chemical formulae below, A represents a monovalent hydrocarbon group or a heterocyclic group, each of which may independently have one or more substituents. When two A's are present in the same group, they may be identical to each other or different from each other.).

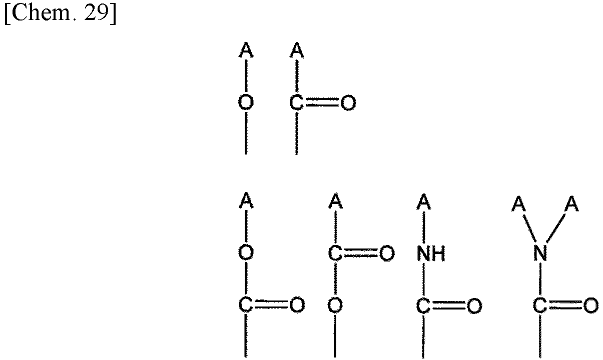

The upper limit of the number of carbon atoms hydrocarbon group (including its substituents, if any) may be, for example, 20 or less, 15 or less, 10 or less, 8 or less, or 6 or less. The lower limit thereof depends on the type of the hydrocarbon group, but may be 1 or more for alkyl groups, 2 or more for alkenyl and alkynyl groups, and 3 or more, such as 4 or more, or 5 or more, for cycloalkyl groups. Examples of the number of atoms include, for example, 1, 2, 3, 4, 5, 6, 7, 8, 9, 10, 11, 12, 13, 14, 15, 16, 17, 18, 19, or 20.

The upper limit of the total number of carbon atoms and hetero atoms of each heterocyclic group (including its substituents, if any) may be, for example, 20 or less, 15 or less, 10 or less, 8 or less, or 6 or less. The lower limit thereof depends on the type of the heterocyclic structure, but may typically be 3 or more, for example 4 or more, or 5 or more. Examples of the number of atoms include, for example, 3, 4, 5, 6, 7, 8, 9, 10, 11, 12, 13, 14, 15, 16, 17, 18, 19, or 20.

Among them, each of R³³ and R³⁶ may preferably be selected, independently of each other, from hydrogen atom, hydroxyl group, or carboxyl group, or, that may have one or more substituents, alkyl group, alkenyl group, cycloalkyl group, alkoxy group, aryl group, aryloxy group, acyl group, heterocyclic group, and heterocyclic oxy group.

Examples of R³³ and R³⁶ include, but are not limited to, the following.
*Hydrogen atom, hydroxyl group, and carboxyl group;
*Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
*Alkenyl groups such as ethenyl group, propenyl group, allyl group, butenyl group, pentenyl group, hexenyl group, heptenyl group, and octenyl group;
*Alkylyl groups such as propargyl group;
*Cycloalkyl groups such as cyclopropyl group, cyclobutyl group, cyclopentyl group, cyclohexyl group, cycloheptyl group, bicyclooctyl group, and spirooctyl group;
* Aryl groups such as phenyl group, benzyl group, tolyl group, naphthyl group, anthracenyl group; and
*Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, and carbazolyl group.

R³¹ and R³³ may be bound to each other to form, together with the carbon atom to which R³¹ binds and the nitrogen atom to which R³³ binds, a hetero ring that may have one or more substituents. Likewise, R³⁴ and R³⁶ may be bound to each other to form, together with the carbon atom to which R³⁴ binds and the nitrogen atom to which R³⁶ binds, a hetero ring that may have one or more substituents. The details of the substituents, if any, are described above. Examples of the number of such substituents include, for example, 5, 4, 3, 2, 1, or 0.

The upper limit of the total number of carbon atoms and hetero atoms of each heterocyclic group (including its substituents, if any) may be, for example, 20 or less, 15 or less, 10 or less, 8 or less, or 6 or less. The lower limit thereof depends on the type of the heterocyclic structure, but may typically be 3 or more, for example 4 or more, or 5 or more. Examples of the number of atoms include, for example, 3, 4, 5, 6, 7, 8, 9, 10, 11, 12, 13, 14, 15, 16, 17, 18, 19, or 20.

Examples of such hetero rings include, but are not limited to, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, and 2,5-dihydro-1,3-thiazolyl group.

A¹ to A⁴, independently of each other, represent a divalent aliphatic hydrocarbon group that may have one or more substituents having 1 to 3 carbon atoms. Examples include, but are not limited to, methylene group, ethylene group, propylene group, and isopropylene group, as well as any groups derived from these groups via substituition with one or more substituents. Examples of the number of such substituents include, for example, 3, 2, 1, or 0.

p1 to p4, independently of each other, represent an integer of 0 or 1.

m and n, independently of each other, represent an integer of equal to or greater than 1 corresponding to the number of the structure units parenthesized with [ ]. In other words, m represents the number of the amino acid residues parenthesized with [ ] in general formula (3-1). When m is 1, then the compound (3-1) is an amino acid, while when m is equal to or greater than 2, then the compound (3-1) is a peptide. Likewise, n represents the number of the amino acid residues parenthesized with [ ] in general formula (3-2). When n is 1, then the compound (3-2) is an amino acid, while n is equal to or greater than 2, then the compound (3-2) is a peptide. The upper limit of each of m and n is not particularly limited so long as the amidation step proceeds, but may preferably be, for example, 100 or less, 80 or less, 60 or less, 50 or less, 40 or less, 30 or less, 20 or less, 15 or less, 12 or less, or 10 or less. Examples of each of m and n, independently of each other, include 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, and 100.

Needless to say, when m is equal to or greater than 2, then each of R¹, R², R³, A¹, A², p1, and p2 in the structure parenthesized with [ ] may be either identical to each other or different from each other among the two or more amino acid residues. Likewise, when n is equal to or greater than 2, then each of R⁴, R⁵, R⁵, A³, A⁴, p3, and p4 in the structure parenthesized with [ ] may be either identical to each other or different from each other among the two or more amino acid residues. In other words, when each of the compound (3-1) and/or the compound (3-2) is a peptide, the two or more amino acid units constituting the peptide may be identical to each other or different from each other.

T¹ represents a hydrogen atom or a monovalent substituent. The type of the monovalent substituent is not particularly restricted, but may be selected from the examples of R³³ and R³⁶ mentioned above, as well as any protective groups for amino groups (hereinafter also referred to as PG¹). The protective group PG¹ for amino groups is not restricted as long as the amino group can be protected so that it does not react in the amidation process and can be deprotected and converted to an amino group after the reaction.

There are a wide variety of protective groups for amino groups, PG¹, known to the art. Examples thereof include monovalent hydrocarbon groups that may have one or more substituents and monovalent heterocyclic groups that may have one or more substituents. Preferred among these are monovalent hydrocarbon groups that may have one or more substituents. There may be any intervening linking group between the hydrocarbon group or heterocyclic group and the nitrogen atom of the amino group to be protected (the nitrogen atom in formula (3-1) to which PG¹ binds). Such a linking group is not particularly limited, but may be selected, independently of each other, from the linking groups listed below (where, in the chemical formulae below, A represents a monovalent hydrocarbon group or a heterocyclic group, each of which may independently have one or more substituents. When two A's are present in the same group, they may be identical to each other or different from each other.).

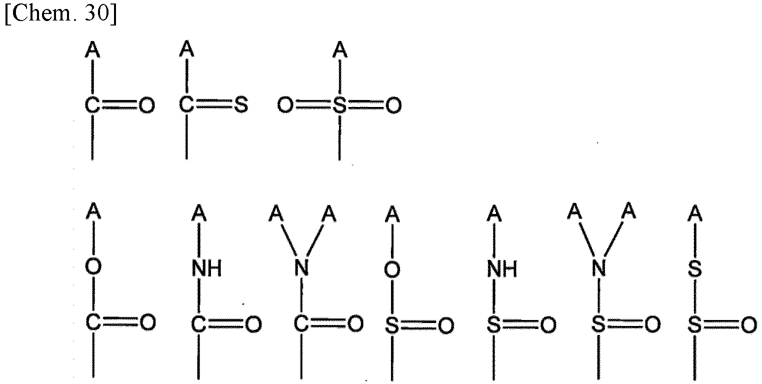

The number of carbon atoms in the protective group PG¹ may typically be 1 or more, or 3 or more, and typically 20 or less, or 15 or less.

Among them, the amino-protective group PG¹ may preferably be one or more selected from the group consisting of a monovalent hydrocarbon group, acyl group, hydrocarbon oxycarbonyl group, hydrocarbon sulfonyl group, and amide group that may have one or more substituents.

Specific examples of the amino-protective group PG¹ are listed below. Incidentally, an amino-protective group may be referred to either by the name of the functional group excluding the nitrogen atom of the amino group to which it binds or by the name of the group including the nitrogen atom to which it binds. The following list includes either or both of these names for each protective group.

Examples of unsubstituted or substituted hydrocarbon groups includes: alkyl groups such as methyl group, ethyl group, and propyl group; alkenyl groups such as ethenyl group, propenyl group, and allyl group; alkylyl groups such as propargyl group; cycloalkyl groups such as cyclopropyl group, cyclobutyl group, cyclopentyl group, and cyclohexyl group; aryl groups such as phenyl group, benzyl group, p-methoxybenzyl group, tolyl group, and triphenylmethyl group (Troc group); and substituted hydrocarbon groups such as cyanomethyl group. The number of carbon atoms may typically be 1 or more, or 3 or more, and typically 20 or less, or 15 or less.

Examples of unsubstituted or substituted acyl groups includes: benzoyl group (Bz), o-methoxybenzoyl group, 2,6-dimethoxy benzoyl group, p-methoxybenzoyl group (PMPCO), cinnamoyl group, and phthaloyl group (Phth).

Examples of unsubstituted or substituted hydrocarbon oxycarbonyl groups includes: tert-butoxycarbonyl group (Boc), benzyloxycarbonyl group (Cbz or Z), methoxycarbonyl group, ethoxycarbonyl group, 2-(trimethylsilyl)ethoxycarbonyl group, 2-phenyl ethoxycarbonyl group, 1-(1-adamanthyl)-1-methylethoxycarbonyl group, 1-(3,5-di-t-butylphenyl)-1-methylethoxycarbonyl group, vinyloxycarbonyl group, allyloxycarbonyl group (Alloc), N-hydroxypiperidinyloxycarbonyl group, p-methoxybenzyloxycarbonyl group, p-nitrobenzyloxycarbonyl group, 2-(1,3-dithianyl)methoxycarbonyl, m-nitrophenoxycarbonyl group, 3,5-dimethoxybenzyloxycarbonyl group, o-nitrobenzyloxycarbonyl group, 2,2,2-trichloroethoxycarbonyl group (Troc), and 9-fluorenylmethyloxycarbonyl group (Fmoc).

Examples of unsubstituted or substituted hydrocarbon sulfonyl groups includes: methane sulfonyl group (Ms), toluenesulfonyl group (Ts), and 2- or 4-nitro benzene sulfonyl (Ns) group.

Examples of unsubstituted or substituted amide groups includes: acetamide, o-(benzoyloxymethyl)benzamide, 2-[(t-butyl-diphenyl-siloxy)methyl]benzamide, 2-toluenesulfonamide, 4-toluenesulfonamide, 2-nitro benzene sulfonamide, 4-nitro benzene sulfonamide, tert-butylsulfinyl amide, 4-toluenesulfonamide, 2-(trimethylsilyl)ethane sulfonamide, and benzyl sulfonamide.

In terms of deprotection methods, the protective group PG¹ may be deprotected by, e.g., at least one of the following methods: deprotection by hydrogenation, deprotection by weak acid, deprotection by fluorine ion, deprotection by one-electron oxidizing agent, deprotection by hydrazine, and deprotection by oxygen.

Preferred examples of the amino protective group PG¹ include mesyl group (Ms), tert-butoxycarbonyl group (Boc), benzyl group (Bn or Bzl), benzyloxycarbonyl group (Cbz), benzoyl group (Bz), p-methoxybenzyl group (PMB), 2,2,2-trichloroethoxycarbonyl group (Troc), allyloxycarbonyl group (Alloc), 2,4-dinitrophenyl group (2,4-DNP), phthaloyl group (Phth), p-methoxybenzoyl group (PMPCO), cinnamoyl group, toluenesulfonyl group (Ts), 2- or 4-nitrobenzenesulfonyl group (Ns), cyanomethyl group, and 9-fluorenylmethyloxycarbonyl group (Fmoc). These protective groups are preferred because, as mentioned above, they can easily protect the amino group and can be removed under relatively mild conditions.

More preferable examples of the amino protective group PG¹ include mesyl group (Ms), tert-butoxycarbonyl group (Boc), benzyloxycarbonyl group (Cbz), benzyl group (Bn), p-methoxybenzyl group (PMB), 2,2,2-trichloroethoxycarbonyl group (Troc), allyloxycarbonyl group (Alloc), p-methoxybenzoyl group (PMPCO), benzoyl group (Bz), cyanomethyl group, cinnamoyl group, 2- or 4-nitrobenzenesulfonyl group (Ns), toluenesulfonyl group (Ts), phthaloyl group (Phth), 2,4-dinitrophenyl group (2,4-DNP), and 9-fluorenylmethyloxycarbonyl group (Fmoc).

Even more preferable examples of the amino protective group PG¹ include mesyl group (Ms), tert-butoxycarbonyl group (Boc), benzyloxycarbonyl group (Cbz), benzyl group (Bn), p-methoxybenzyl group (PMB), 2,2,2-trichloroethoxycarbonyl group (Troc), allyloxycarbonyl group (Alloc), p-methoxybenzoyl group (PMPCO), benzoyl group (Bz), cyanomethyl group, and cinnamoyl group.

T² represents a hydrogen atom or a monovalent substituent. The type of the monovalent substituent is not particularly limited, but may be selected from the examples of R³¹, R³², R³⁴, and R³⁶ mentioned above, as well as protective groups for carboxyl group (hereinafter also referred to as PG²). The carboxyl protective group PG² is not limited as long as the carboxyl group can be protected so that it does not react in the amidation process, and then can be deprotected and converted to a carboxyl group after the reaction.

Examples of the carboxyl protective group PG² include monovalent hydrocarbon groups or heterocyclic groups that may have one or more substituents. The details of the substituents, if any, are described above. Examples of the number of such substituents include, for example, 5, 4, 3, 2, 1, or 0.

The upper limit of the number of carbon atoms of each hydrocarbon group (including its substituents, if any) may be for example 20 or less, 15 or less, 10 or less, 8 or less, or 6 or less. The lower limit thereof depends on the type of the hydrocarbon group, but may be 1 or more for alkyl groups, 2 or more for alkenyl and alkynyl groups, and 3 or more, such as 4 or more, or 5 or more, for cycloalkyl groups. Examples of the number of atoms include, for example, 1, 2, 3, 4, 5, 6, 7, 8, 9, 10, 11, 12, 13, 14, 15, 16, 17, 18, 19, or 20.

The upper limit of the total number of carbon atoms and hetero atoms of each heterocyclic group (including its substituents, if any) may be, for example, 20 or less, 15 or less, 10 or less, 8 or less, or 6 or less. The lower limit thereof depends on the type of the heterocyclic structure, but may typically be 3 or more, for example 4 or more, or 5 or more. Examples of the number of atoms include, for example, 3, 4, 5, 6, 7, 8, 9, 10, 11, 12, 13, 14, 15, 16, 17, 18, 19, or 20.

Examples of the carboxyl protective group PG² include, but are not limited to, the following.
*Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
*Alkenyl groups such as ethenyl group, propenyl group, allyl group, butenyl group, pentenyl group, hexenyl group, heptenyl group, and octenyl group;
*Alkylyl groups such as propargyl group;
*Cycloalkyl groups such as cyclopropyl group, cyclobutyl group, cyclopentyl group, cyclohexyl group, cycloheptyl group, bicyclooctyl group, and spirooctyl group;
* Aryl groups such as phenyl group, benzyl group, tolyl group, naphthyl group, anthracenyl group;
*Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, and carbazolyl group; and
*Silicon-based protective groups such as trimethylsilyl (TMS) group, triethylsilyl (TES) group, triisopropylsilyl (TIPS) group, tri(tert-butyl)silyl (TBS) group, and tert-butyldiphenylsilyl (TBDPS) group.

In Embodiment (3), it is preferable to adjust the reaction procedure as described below in such a manner that (a) the first amino acid or peptide (3-1) is first contacted with the silane compound (A) and/or (B), (b) then with the second silane compound (if optionally used), and (c) finally with the second amino acid or peptide (3-2). This makes it possible to selectively form an amide bond between the terminal carboxyl group of the compound (3-1) on the right side of the formula and the terminal amino group of the compound (3-2) on the left side of the formula and link them together, even when any amino groups or any carboxyl groups present in the reactant compounds (3-1) and (3-2) that should not be subject to amidation reaction (e.g., the amino group of the compound (3-1) on the left side of the formula, or the carboxyl group on the right in the compound (3-2) on the right side of the formula) are not protected with protective groups PG¹ or PG². Therefore, when this reaction procedure is employed, it is preferred that at least either of T¹ and T² is a hydrogen atom, and it is more preferred that both of T¹ and T² are hydrogen atoms.

With regard to the compound (3-2), the amino group on the left side of general formula (3-2) may form a salt with an acid. **In** this case, examples of the acid include, but are not limited to' aliphatic carboxylic acids with 1 to 5 carbon atoms, such as acetic acid and propionic acid; and trifluoroacetic acid, hydrochloric acid, hydrobromic acid, sulfuric acid, nitric acid, phosphoric acid, boric acid, and sulfonic acid.

Embodiment (3) is deemed a specific embodiment of Embodiment (1) in which the compound (1-1) and the compound (1-2) are both amino acids or peptides and the compound (1-3) is a peptide. Likewise, Embodiment (2) has a specific embodiment in which the compound (2-1) is an amino acid or peptide and the compound (2-3) is a cyclic peptide. The details of such a specific embodiment are clear to those skilled in the art, taking into account the details of Embodiments (2) and (3) explained above.

In all of these embodiments, some or all of the reactant compounds may be linked or immobilized to a substrate, resin, or other carrier at any of their substituents. **In** this case, the type of the substrate, resin, or other carrier is not limited. Any known substrate, resin, or other carrier can be used as long as it does not substantially interfere with the amide reaction in the production method according to the present invention and does not depart from the purpose of the present invention. There are no restrictions the manner of linking and immobilizing the reactant compound to the substrate, resin, or other carrier, although it is preferable to form a covalent bond between any substituent of the reactant compound and any substituent present on the substrate, resin, or other carrier. There are also no restrictions on the type of each substituent and the method of forming a covalent bond. Any known substituent and covalent bond formation method can be used as long as it does not substantially interfere with the amide bond reaction in the production method according to the present invention and does not depart from the purpose of the present invention. When the reactant compounds are amino acids or peptides, such as in the case of Embodiment (3), the reactant compound may be linked and immobilized to a substrate, resin, or other carrier by covalent bonding using a carboxyl or amino group of the reactant compound (other than the carboxyl or amino group being the target of the amide reaction). Such a mode can be regarded as a variation of the embodiment in which the carboxyl or amino group of the reactant compound (other than the carboxyl or amino group being the target of the amide reaction) is protected by introducing a protective group.

### *Amount(s) of the reactant compound(s) used:

The amounts of the reactant compounds used in the production method according to the present invention depend on the embodiment of the production method according to the present invention, but are generally as follows.

In the case of Embodiment (1), the amount ratio between the compound (1-1) and the compound (1-2) is not particularly limited, but may be determined as follows. The molar ratio of the compound (1-2) to 1 mol of the compound (1-1) may be typically 0.1 mol or more, for example 0.2 mol or more, 0.3 mol or more, or 0.5 mol or more, and may be typically 20 mol or less, for example 10 mol or less, 8 mol or less, 6 mol or less, 5 mol or less, or 2 mol or less. It is preferable to use the compound (1-1) in a higher amount than that of the compound (1-2) in order to increase the efficiency of the reaction. Specifically, the molar ratio of the compound (1-2) to 1 mol of the compound (1-1) may be 0.5 or less. Needless to say, it is necessary to use at least 1 mol of each of the reactant compounds (1-1) and (1-2) for 1 mol of the target compound (1-3) to be produced.

In the case of Embodiment (3), the amount ratio between the compound (3-1) and the compound (3-2) is the same as in Embodiment (1). Specifically, although the ratio in Embodiment (3) is not particularly limited to, the molar ratio of the compound (3-2) to 1 mol of the compound (3-1) may typically be 0.1 mol or more, for example 0.2 mol or more, 0.3 mol or more, or 0.5 mol or more, and also may be typically 20 mol or less, for example 10 mol or less, 8 mol or less, 6 mol or less, 5 mol or less, or 2 mol or less. It is preferable to use the compound (3-1) in a higher amount than that of the compound (3-2) in order to increase the efficiency of the reaction. Specifically, the molar ratio of the compound (3-2) to 1 mol of the compound (3-1) may be 0.5 or less. Needless to say, it is necessary to use at least 1 mol of each of the reactant compounds (3-1) and (3-2) for 1 mol of the target compound (3-3) to be produced.

On the other hand, in the case of Embodiment (2), such an adjustment of the amount ratio is unnecessary since only one reactant is used, i.e., the compound (2-1). Needless to say, it is necessary to use at least 1 mol of the reactant compound (2-1) for 1 mol of the target compound (2-2) to be produced.

### *Amount(s) of the silane compound(s) (A) and/or (B) (reaction agent according to the

### present invention) used:

The amount(s) of the silane compound(s) (A) and/or (B) (the reaction agent according to the present invention) is not particularly limited, so long as the desired amidation reaction between a carboxyl group and an amino group can be induced through the implementation of the production method according to the present invention. For example, for 1 mol of each reactant compound, each of the silane compound(s) (A) and/or (B) may be used in an amount of typically 0.1 mol or more, for example 0.2 mol or more, 0.3 mol or more, 0.5 mol or more, or 1 mol or more. On the other hand, there is no restriction on the upper limit of the amount of silane compound (A) and/or (B) used. For example, for 1 mol of each reactant compound, each of the silane compound(s) (A) and/or (B) may be used in an amount of typically 20 mol or less, for example, 10 mol or less, 8 mol or less, 6 mol or less, or 5 mol or less, or 2 mol or less for reaction efficiency. When two or more silane compounds (A) and/or (B) are used, the total amount of the two or more silane compounds (A) and/or (B) may preferably satisfy the range mentioned above.

### *Second silane compound:

In the production method according to the present invention, in addition to the reactant compounds and the silane compound(s) (A) and/or (B) (reactants of the invention), a second silane compound may be used in the production method of the invention. Various advantages may be obtained by carrying out the reaction in the presence of the second silane compound in the reaction system. For example, in Embodiment (3), the reaction procedure can be adjusted as described below in such a manner that (a) the first amino acid or peptide (3-1) is first contacted with the silane compound (A) and/or (B), (b) then with the second silane compound (if optionally used), and (c) finally with the second amino acid or peptide (3-2). This makes it possible to selectively form an amide bond between the terminal carboxyl group of the compound (3-1) on the right side of the formula and the terminal amino group of the compound (3-2) on the left side of the formula and link them together, even when any amino groups or any carboxyl groups present in the reactant compounds (3-1) and (3-2) that should not be subject to amidation reaction (e.g., the amino group of the compound (3-1) on the left side of the formula, or the carboxyl group on the right in the compound (3-2) on the right side of the formula) are not protected with protective groups PG¹ or PG².

When the second silane compound is used in the production method according to the present invention, the type of the second silane compound is not particularly limited, but may preferably be one or more silane compounds selected from compounds represented by general formulae (C1) to (C4) (hereinafter also referred to as "silane compound (C)").

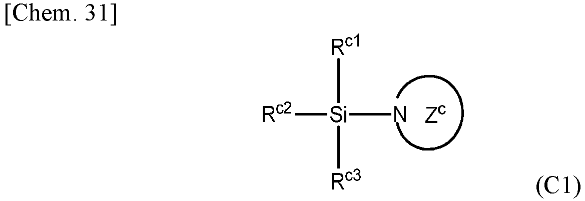

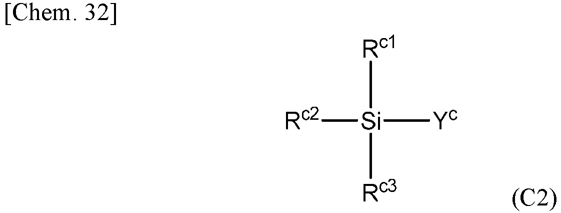

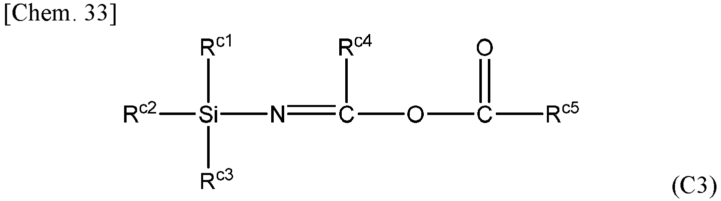

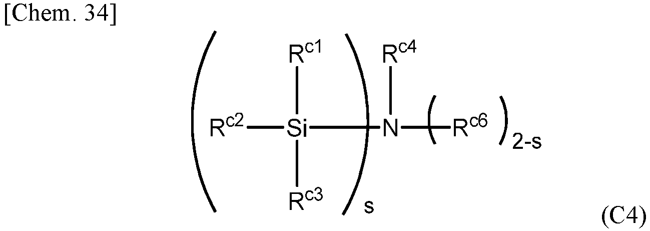

The definition of each symbol in general formulae (C1) to (C4) are as follows.

R^{c1} to R^{c3}, independently of each other, represent a hydrogen atom or a linear- or branched-chain alkyl group or alkoxy group that contains 1 to 10 (preferably 1 to 5, more preferably 1 to 3) carbon atoms and may have one or more substituents, provided that at least 2 of R^{c1} to R^{c3} are an alkyl group that may have one or more substituents or alkoxy group. In other words, 0 or 1 of R^{c1} to R^{c3} is a hydrogen atom

R^{c4} and R^{c5}, independently of each other, represent a linear- or branched-chain alkyl group or alkoxy group that contains 1 to 10 (preferably 1 to 5, more preferably 1 to 3) carbon atoms and may have one or more substituents.

When each of R^{c1} to R^{c5} is an alkyl group or alkoxy group has one or more substituents, the types of the substituents (if any) are as explained above, among which halogen atoms are preferred. Examples of the number of such substituents include, for example, 5, 4, 3, 2, 1, or 0.

Z^{c} represents a 5- to 10-membered (preferably 5-, 6-, or 10-membered) heterocyclic group that contains one or more (preferably 2 to 4, more preferably 2 or 3) nitrogen atoms as ring-constituting atoms and may have one or more substituents When the heterocyclic group has one or more substituents, the details of the substituents, if any, are as explained above, preferred among which include alkyl groups (e.g., linear- or branched-chain alkyl groups having 1 to 10 carbon atoms; hereinafter also referred to as -R), alkoxy group (-OR), amino group (-NH₂), alkylamino group (-NHR), dialkylamino group (-NR₂: where the two alkyl groups R may be either identical to each other or different from each other), and thioalkyl group (-SR), as well as any groups derived from these groups via substitution with one or more halogen atoms (e.g., bromine or chlorine atom). Examples of the number of such substituents include, for example, 10, 9, 8, 7, 6, 5, 4, 3, 2, 1, or 0. When there are two or more substituents, they may be either identical to each other or different from each other.

Examples of the nitrogen-containing heterocyclic group of Z^{c} include, but are not limited to: pyrrole group, imidazole group, pyrazole group, triazolegroup (1,2,3-triazolegroup, 1,2,4-triazolegroup), piperidyl group, pyridinyl group, piperazinyl group, tetrazole group, indole group, and benzimidazole group, as well as any groups derived from these groups via substitution with one or more substituents mentioned above, such as (2-/3-/4-/5-)methylimidazole group and (2,3-/2,4-/2,5-)dimethylimidazole group. Preferred among them are imidazole group, pyrazole group, triazolegroup, 2-methyl imidazole group.

Y^{c} represents hydrogen atom or halogen group such as chlorine atom or bromine atom.

R^{c6} represents a linear- or branched-chain alkyl group, alkoxy group, or alkyl carbonyl group that contains 1 to 10 (preferably 1 to 5, more preferably 1 to 3) carbon atoms and may have one or more substituents. When this group has one or more substituents, the types of the substituents (if any) are as explained above, among which halogen atoms are preferred. Examples of the number of such substituents include, for example, 5, 4, 3, 2, 1, or 0.

s represents an integer of 1 or 2. When s is 2, then R^{c6} is absent.

Examples of the compound represented by general formula (C1) include 1-(trimethylsilyl)imidazole (TMSIM), dimethylethylsilylimidazole (DMESI), dimethylisopropylsilylimidazole (DMIPSI), 1-(tert-butyldimethylsilyl)imidazole (TBSIM), 1-(trimethylsilyl)triazole, 1-(tert-butyldimethylsilyl)triazole, dimethylsilylimidazole, and dimethylsilyl(2-methyl)imidazole. Preferred among these include 1-(trimethylsilyl)imidazole (TMSIM) and 1-(tert-butyldimethylsilyl)imidazole (TBSIM).

Examples of the compound represented by general formula (C2) include trimethylbromosilane (TMBS) and trimethylchlorosilane (TMCS). Preferred among these include trimethylbromosilane (TMBS).

Examples of the compound represented by general formula (C3) include N-methyl - N-trimethylsilyltrifluoroacetamide (MSTFA), N,O-bis(trimethylsilyl)trifluoroacetamide (BSTFA), and N,O-bis(trimethylsilyl)acetamide (BSA). Preferred among these include N-methyl -N-trimethylsilyltrifluoroacetamide (MSTFA).

Examples of the compound represented by general formula (C4) include N-(trimethylsilyl)dimethylamine (TMSDMA), N-(tert-butyldimethylsilyl)-N-methyl-trifluoroacetamide (MTBSTFA), and hexamethyldisilazane (HMDS). Preferred among these include N-(trimethylsilyl)dimethylamine (TMSDMA) and N-(tertbutyldimethylsilyl)-N-methyl trifluoroacetamide (MTBSTFA).

The silane compounds (C) mentioned above may be used either singly or in combination of any two or more.

The amount of the second silane compound used is not particularly limited so long as it can induce the desired amidation reaction between carboxyl and amino groups through the implementation of the manufacturing method. For example, for 1 mol of each reactant compound, the second silane compound may be used in an amount of typically 0.1 mol or more, for example 0.2 mol or more, 0.3 mol or more, 0.5 mol or more, or 1 mol or more. The upper limit of the amount of the second silane compound used is also not particularly limited, but for 1 mol of each reactant compound, the second silane compound may be used in an amount of typically 20 mol or less, for example 10 mol or less, 8 mol or less, 6 mol or less, or 5 mol or less, or 2 mol or less in terms of reaction efficiency. When two or more second silane compounds are used together, the total amount of the two or more second silane compounds may satisfy the range mentioned above.

### *Amino silane catalyst:

According to the production method according to the present invention, the reaction system may contain, in addition to the reactant compound and the silane compound(s) (A) and/or (B) (the reaction agent(s) according to the present invention), as well as the second silane compound optionally used, an aminosilane compound having a specific structure as a catalyst. Various advantages may be obtained by carrying out the reaction in the presence of such an aminosilane catalyst in the reaction system, such as an increase in reaction rate, a reduction in the amount of the reactant compound(s) used, and a reduction in the amount(s) of the silane compound(s) (A) and/or (B) (the reaction agent(s) according to the present invention) used.

When the aminosilane compound is used as a catalyst in the production method according to the present invention, the type of the aminosilane compound is not particularly limited, but may preferably be a compound represented by formula (D).

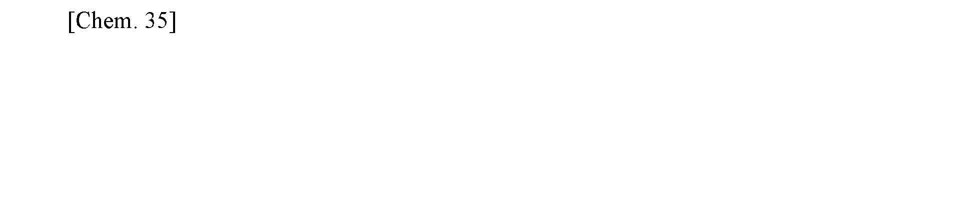

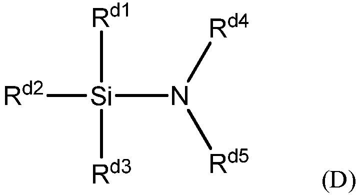

In formula (D), R^{d1} to R^{d3}, independently of each other, represent a hydrogen atom, or, a linear- or branched-chain alkyl group or alkoxy group that may have one or more substituents, provided that, at least 2 (preferably 3) of R^{c1} to R^{c3} are alkyl groups that may have one or more substituents or alkoxy group. In other words, 0 or 1 (preferably 0) of R^{d1} to R^{d3} is a hydrogen atom. When each of R^{d1} to R^{d3} is an alkyl group or alkoxy group, the number of carbon atoms is not particularly limited, but may preferably be 1 to 10, especially 1 to 7, more particularly 1 to 5. Each of R^{d1} to R^{d3} may be either an alkyl group or an alkoxy group, but may preferably be an alkoxy group. The alkyl group or alkoxy group may or may not have any substituent, but may preferably have at least one substituent. The type of the substituent (if any) is not particularly limited, and may be any substituent selected from the examples mentioned above, although it may preferably have at least one or more halogen atoms as substituents. When each alkyl group or alkoxy group has one or more halogen atoms as substituents, the number of the halogen atoms is not limited and may be 1 or more, although it may preferably be typically 1 to 20, particularly 1 to 16, more particularly 1 to 12, especially 1 to 8. The types of the halogen atoms are not limited, although they may preferably be selected typically from fluorine, chlorine, bromine, or iodine, more preferably from fluorine, chlorine, or bromine, still more preferably from fluorine or chlorine. When the number of halogen atoms is two or more, the two or more may be either identical to each other or different from each other. In other words, R^{d1} to R^{d3} may preferably be, but are not limited to, haloalkoxy groups.

In formula (D), R^{d4} and R^{d5}, independently of each other, represent a hydrogen atom, or, an alkyl group, aryl group, alkyl aryl group, or aryl alkyl group that may have one or more substituents, provided that at least one of R^{d4} and R^{d5} is an alkyl group, aryl group, alkyl aryl group, or aryl alkyl group that may have one or more substituents. In other words, 0 or 1 of R^{d4} and R^{d5} is a hydrogen atom. When each of R^{d4} and R^{d5} is an alkyl group, aryl group, alkyl aryl group, or aryl alkyl group, the number of carbon atoms is not particularly limited, but in the case of an alkyl group, the number of carbon atoms may preferably be 1 to 10, more preferably 1 to 7, still more preferably 1 to 5. In the case of an aryl group, the number of carbon atoms may preferably be 6 to 12, more preferably 6 to 10. In the case of an alkyl aryl group or aryl alkyl group, the number of carbon atoms may preferably be 7 to 20, more preferably 7 to 16, still more preferably 7 to 13. The number of each of the alkyl and aryl groups constituting an alkylaryl or arylalkyl group may be one, two or more, respectively. When an alkylaryl group or arylalkyl group contains two or more alkyl and/or aryl groups, they may be identical to each other or different from each other. The alkyl group, aryl group, alkyl aryl group, or aryl alkyl group may or may not have a substituent. The type of the substituent (if any) is not particularly limited, and may be any substituent selected from the examples mentioned above, but it may preferably have at least one or more halogen atoms and/or alkoxy groups as substituents. When the alkyl group, aryl group, alkyl aryl group, and/or aryl alkyl groups have at least one halogen atom as substituents, the number of the halogen atoms is not limited and may be 1 or more, although it may preferably be typically 1 to 20, particularly 1 to 16, more particularly 1 to 12, especially 1 to 8. The types of the halogen atoms are not limited, although they may preferably be selected typically from fluorine, chlorine, bromine, or iodine, more preferably from fluorine, chlorine, or bromine, still more preferably from fluorine or chlorine. When the number of halogen atoms is two or more, the two or more may be either identical to each other or different from each other. When the alkyl group, aryl group, alkyl aryl group, and/or aryl alkyl groups have at least one, the number of the alkoxy groups is not limited and may be 1 or more, although it may preferably be typically 1 to 10, more preferably 1 to 5, still more preferably 1 to 3. The types of the alkoxy groups are not limited, although they may preferably be selected typically from alkoxy groups having 1 to 10, preferably 1 to 7, more preferably 1 to 4. When the number of alkoxy groups is two or more, they may be either identical to each other or different from each other.

Examples of the aminosilane compound represented by formula (D) include, but are not limited to, various aminosilane compounds used as catalysts in Example Groups F and G below, and more specifically, the aminosilane compounds listed in the tables below.

**[Table B1]**

| Entry | Aminosilane catalysts |
|---|---|
| 1 | 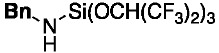 |
| 2 | 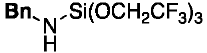 |
| 3 | 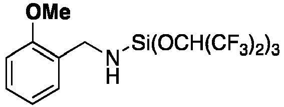 |
| 4 | 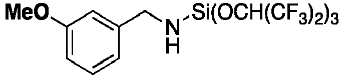 |
| 5 | 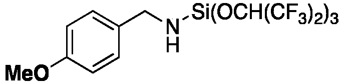 |
| 6 | 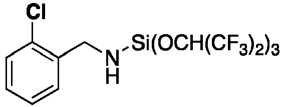 |
| 7 | 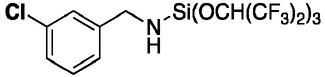 |
| 8 | 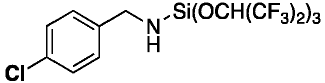 |
| 9 | 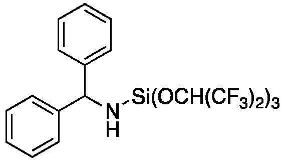 |
| 10 | 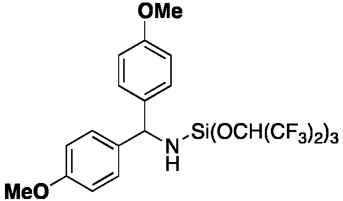 |

**[Table B2]**

| Entry | Aminosilane catalysts |
|---|---|
| 11 | 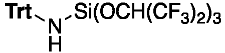 |
| 12 | 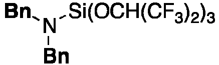 |
| 13 | 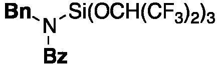 |
| 14 | 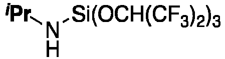 |
| 15 | 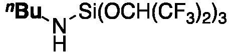 |
| 16 | 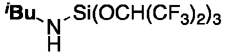 |
| 17 | 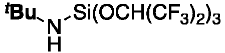 |
| 18 | 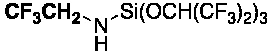 |
| 19 | 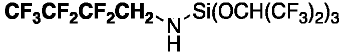 |
| 20 | 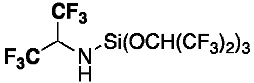 |
| 21 | 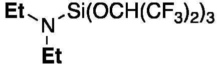 |
| 22 | 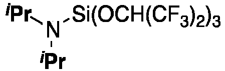 |

**[Table B3]**

| Entry | Aminosilane catalysts |
|---|---|
| 23 | 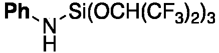 |
| 24 | 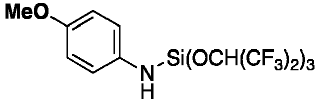 |
| 25 | 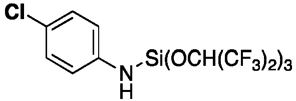 |
| 26 | 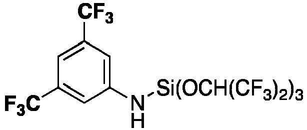 |
| 27 | 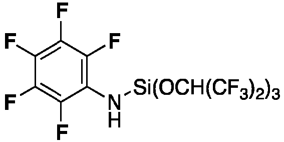 |
| 28 | 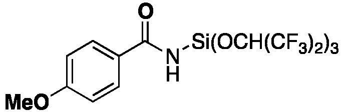 |
| 29 | 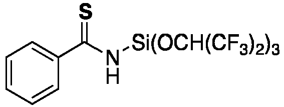 |
| 30 | 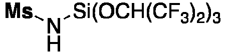 |
| 31 | 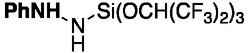 |
| 32 | 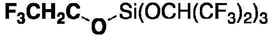 |
| 33 | 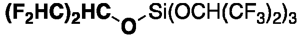 |
| 34 | 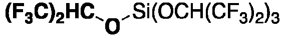 |

The aminosilane catalysts mentioned above may be used either singly or in combination of any two or more at any ratios.

The amount of the aminosilane catalyst used is not particularly limited so long as the desired amidation reaction between carboxyl and amino groups can be induced through the implementation of the production method according to the present invention. For example, when the amount of each reactant compound used is considered 100 mol%, the amount of the aminosilane catalyst may preferably be 0.1 mol% or more, for example 0.2 mol% or more, or 0.3 mol% or more, and may preferably be 30 mol% or less, for example 20 mol% or less, or 15 mol% or less. When two or more aminosilane catalysts are used together, the total amount of the two or more aminosilane catalysts may preferably satisfy the range mentioned above.

### *Lewis acid catalyst:

In the production method according to the present invention, the reaction system may contain a Lewis acid catalyst, in addition to the reactant compound(s) and the silane compound(s) (A) and/or (B) (the reaction agent(s) according to the present invention), as well as the second silane compound and/or the aminosilane catalyst optionally used. Various advantages may be obtained by carrying out a reaction in the presence of a Lewis acid catalyst in the reaction system, such as improved reaction yield and stereoselectivity. On the other hand, however, if a Lewis acid catalyst is used, it may be necessary to separate and remove the Lewis acid catalyst from the reaction product. Therefore, it is preferable to determine whether or not to use a Lewis acid catalyst in consideration of the purpose of using the production method according to the present invention.

When a Lewis acid catalyst is used in the production method according to the present invention, the type of catalyst is not limited, but it may preferably be a metal compound that functions as a Lewis acid.

Examples of metal elements constituting the metal compound include various metals belonging to groups 2 through 15 of the periodic table. Examples of such metal elements include boron, magnesium, aluminum, gallium, indium, silicon, calcium, lead, bismuth, mercury, transition metals, and lanthanoid elements. Examples of transition metals include scandium, titanium, vanadium, chromium, manganese, iron, cobalt, nickel, copper, zinc, yttrium, zirconium, niobium, molybdenum, technetium, ruthenium, rhodium, palladium, tin, silver, cadmium, hafnium, tantalum, tungsten, rhenium, osmium, iridium, platinum, gold, and thallium. Examples of lanthanoid elements include lanthanum, cerium, neodymium, samarium, europium, gadolinium, holmium, erbium, thulium, ytterbium. From the viewpoint of excellent reaction acceleration and highly stereoselective production of amide compounds, the metal element may preferably be one or more selected from titanium, zirconium, hafnium, tantalum, niobium, boron, vanadium, tungsten, neodymium, iron, lead, cobalt, copper, silver, palladium, tin, and thallium, more preferably one or more selected from titanium, zirconium, hafnium, tantalum, and niobium. The metal compound may contain one, two or more metal atoms. If the metal compound contains two or more metal atoms, the two or more metal atoms may be either of the same metal element or of different metal elements.

Ligands constituting the metal compound may be selected according to the type of the metal element. Examples of ligands include: substituted or unsubstituted linear- or branched-chain alkoxy groups containing 1 to 10 carbon atoms, such as methoxy group, ethoxy group, propoxy group, butoxy group, trifluoroethoxy group, and trichloroethoxy group; halogen atoms such as fluorine atom, chlorine atom, bromine atom, iodine atom; aryloxy groups having 1 to 10 carbon atoms; acetylacetonate group (acac), acetoxy group (AcO), trifluoromethane sullfonate group (TfO); substituted or unsubstituted linear- or branched-chain alkyl groups having 1 to 10 carbon atoms; phenyl group, oxygen atom, sulfur atom, group -SR (where R represents a substituent exemplified by substituted or unsubstituted hydrocarbon groups containing 1 to 20 carbon atoms), group -NRR' (where R and R', independently of each other, represent a hydrogen atom or a substituent exemplified by substituted or unsubstituted hydrocarbon groups containing 1 to 20 carbon atoms), and cyclopentadienyl (Cp) group.

Preferred metal compounds among these are titanium compounds, zirconium compounds, hafnium compounds, tantalum compounds, or niobium compounds. Examples of these metal compounds are indicated below.

Examples of titanium compounds include those represented by TiX¹₄ (where 4 X^{1,}s, independently of each other, represent any of the ligands exemplified above, provided that 4 X^{1,}s may be the same type of ligand or different from each other.). When X¹ is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X¹ is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X¹ is a halogen atom, it may preferably be a chlorine atom or a bromine atom. Preferred examples of titanium compounds include Ti(OMe)₄, Ti(OEt)₄, Ti(OPr)₄, Ti(Oi-Pr)₄, Ti(OBu)₄, Ti(Ot-Bu)₄, Ti(OCH₂CH(Et)Bu)₄, CpTiCl₃, Cp₂TiCl₂, Cp₂Ti(OTf)₂, (i-PrO)₂TiCl₂, and (i-PrO)₃TiCl.

Examples of zirconium compounds include those represented by ZrX²₄ (where 4 X^{2,}s, independently of each other, represent any of the ligands exemplified above, provided that 4 X^{2,}s may be the same type of ligand or different from each other.). When X² is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X² is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X² is a halogen atom, it may preferably be a chlorine atom or a bromine atom. Preferred examples of zirconium compounds include Zr(OMe)₄, Zr(OEt)₄, Zr(OPr)₄, Zr(Oi-Pr)₄, Zr(OBu)₄, Zr(Ot-Bu)₄, Zr(OCH₂CH(Et)Bu)₄, CpZrCl₃, Cp₂ZrCl₂, Cp₂Zr(OTf)₂, (i-PrO)₂ZrCl₂, and (i-PrO)₃ZrCl.

Examples of hafnium compounds include those represented by HfX³₄ (where 4 X^{3,}s, independently of each other, represent any of the ligands exemplified above, provided that 4 X^{3,} s may be the same type of ligand or different from each other.). When X³ is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X³ is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X³ is a halogen atom, it may preferably be a chlorine atom or a bromine atom. Preferred examples of hafnium compounds include HfCp₂Cl₂, HfCpCl₃, and HfCl₄.

Examples of tantalum compounds include those represented by TaX⁴₅ (where 5 X⁴'s, independently of each other, represent any of the ligands exemplified above, provided that 5 X⁴'s may be the same type of ligand or different from each other.). When X⁴ is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X⁴ is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X⁴ is a halogen atom, it may preferably be a chlorine atom or a bromine atom. Preferred examples of tantalum compounds include tantalum alkoxide compounds (e.g., compounds in which X⁴ is an alkoxy group) such as Ta(OMe)₅, Ta(OEt)₅, Ta(OBu)s, Ta(NMe₂)₅, Ta(acac)(OEt)₄, TaCl₅, TaCl₄(THF), and TaBr₅. Other examples are those in which X⁴ is an oxygen, such as Ta₂O₅.

Examples of niobium compounds include those represented by NbX⁵₅ (where 5 X^{5,}s, independently of each other, represent any of the ligands exemplified above, provided that 5 X⁵' s may be the same type of ligand or different from each other.). When X⁵ is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X⁵ is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X⁵ is a halogen atom, it may preferably be a chlorine atom or a bromine atom. Preferred examples of niobium compounds include niobium alkoxide compounds (e.g., compounds in which X⁵ is an alkoxy group) such as NbCl₄(THF), NbCl₅, Nb(OMe)₅, and Nb(OEt)₅. Other examples are those in which X⁵ is an oxygen, such as Nb₂O₅.

Preferred metal compounds as Lewis acid catalysts in the production method according to the present invention also depend on the type of the reactant compound(s).

For example, in the case of Embodiment (3) where the compound (3-1) and the compound (3-2) are both amino acids (i.e., m and n are both 1), then a tantalum or niobium compound is preferred as a Lewis acid catalyst.

On the other hand, in the case of Embodiment (3) where at least either the compound (3-1) and the compound (3-2) is a peptide (i.e., at least either m and n is equal to or greater than 2), then the Lewis acid catalyst may preferably be a titanium compound, zirconium compound, or hafnium compound, more preferably a titanium compound. The reason for this is not clear, but presumably because titanium catalysts are suitable for activation in a 7-membered ring including the peptide bond as the starting functional group, due to the small atomic radius of the titanium metal, which has little effect on the steric hindrance of the peptide chain.

These Lewis acid catalysts may be used either singly or in combination of any two or more.

The amount of the Lewis acid catalyst used is not limited so long as the desired amidation reaction between carboxyl and amino groups can be induced through the implementation of the production method according to the present invention. For example, when the amount of each reactant compound used is considered 100 mol%, the amount of the Lewis acid catalyst may preferably be 0.1 mol% or more, for example 0.2 mol% or more, or 0.3 mol% or more, and may preferably be 30 mol% or less, for example 20 mol% or less, or 15 mol% or less.

The Lewis acid catalyst may be loaded on a carrier. There are no particular restrictions on the carrier on which the Lewis acid catalyst is to be loaded, and any known carrier can be used. Also, any known method can be used to load the Lewis acid catalyst on the carri er.

### *Phosphorus compound:

In the production method according to the present invention, the reaction system may contain a phosphorus compound, in addition to the reactant compound(s) and the silane compound(s) (A) and/or (B) (the reaction agent(s) according to the present invention), as well as the second silane compound and/or the aminosilane catalyst and/or the Lewis acid catalyst optionally used. Various advantages may be obtained by conducting the reaction with a phosphorus compound in the reaction system, such as improved reaction yield and stereoselectivity.

When a phosphorus compound is used in the production method according to the present invention, the type of the phosphorus compound used is not particularly limited, but it may preferably be a trivalent phosphorus compound such as a phosphine compound or a phosphate compound.

Preferred phosphine compounds include those represented by general formula R₃P or (RO)₃P (where R, independently of each other, represents an aliphatic or aromatic hydrocarbon group or heterocyclic group that may have one or more substituents. preferably an alkyl group having 1 to 10 carbon atoms or an aryl group having 6 to 12 carbon atoms, the substituents (if any) being preferably, independently of each other, selected form alkyl groups or alkoxy groups having 1 to 5 carbon atoms and halogen atoms). Examples of such phosphine compounds include trimethyl phosphine, triethyl phosphine, tripropyl phosphine, trimethyloxy phosphine, triethyloxy phosphine, tripropyloxy and phosphine, triphenyl phosphine, trinaphthyl phosphine, and triphenyloxy phosphine, as well as compounds derived from these compounds via substitution with one or more substituents such as methyl group, methoxy group, and fluoro group, including tris(4-methylphenyl)phosphine, tris(4-methoxyphenyl)phosphine, tris(4-fluorophenyl)phosphine, tris(4-methylphenyloxy)phosphine, tris(4-methoxyphenyloxy)phosphine, and tris(4-fluorophenyloxy)phosphine.

Preferred phosphate compounds include those represented by general formula (RO)₃PO (where R, independently of each other, represents an aliphatic or aromatic hydrocarbon group or heterocyclic group that may have one or more substituents. preferably an alkyl group having 1 to 10 carbon atoms or an aryl group having 6 to 12 carbon atoms, the substituents (if any) being preferably, independently of each other, selected form alkyl groups or alkoxy groups having 1 to 5 carbon atoms and halogen atoms). Examples of such phosphine compounds include trimethyl phosphate, triethyl phosphate, tripropyl phosphate, trimethyloxy phosphate, triethyloxy phosphate, tripropyloxy phosphate, triphenyl phosphate, trinaphthyl phosphate, and triphenyloxy phosphate, as well as compounds derived from these compounds via substitution with one or more substituents such as methyl group, methoxy group, and fluoro group, including tris(4-methylphenyl)phosphate, tris(4-methoxyphenyl)phosphate, tris(4-fluorophenyl)phosphate, tris(4-methylphenyloxy)phosphate, tris(4-methoxyphenyloxy)phosphate, and tris(4-fluorophenyloxy)phosphate.

It is also possible to use multivalent phosphine compounds or multivalent phosphate compounds, which can be obtained by linking two or more molecules of any of the phosphine compounds or phosphate compounds mentioned above. Examples of such multivalent phosphine compounds or multivalent phosphate compounds include 2,2'-bis(diphenylphosphino)-1,1'-binaphthyl (BINAP) and 5,5'-bis(diphenylphosphino)-4,4'-bi-1,3-benzodioxole (SEGPHOS).

When a phosphorus compound is used in the production method according to the present invention, the among of the phosphorus compound used is not particularly limited, although when the amount of the reactant compound is considered to be 100 mol%, the amount of the phosphorus compound may be 0.1 mol% or more, and 20 mol% or less, or 10 mol% or less.

### *Other ingredients:

In the production method according to the present invention, the reaction system may contain one or more other ingredients, in addition to the reactant compound(s) and the silane compound(s) (A) and/or (B) (the reaction agent(s) according to the present invention), as well as the second silane compound and/or the aminosilane catalyst and/or the Lewis acid catalyst and/or the phosphorus compound optionally used. Various advantages may be obtained by conducting the reaction with a phosphorus compound in the reaction system, such as improved reaction yield and stereoselectivity.

For example, from the viewpoint of increasing reaction efficiency, a reaction accelerator may be coexisted during the amidation reaction. Examples of reaction accelerators that can be used include combination agents of cesium fluoride and imidazole or its derivatives, various bases, boron compounds, triethylamine, and various known metal catalysts such as Pd, Rh, Co, Ni, Mg, etc. Although the reaction accelerator is not essential for the amidation reaction, coexistence of the reaction accelerator in the amidation reaction may markedly accelerate the reaction rate. These reaction accelerators may be used either singly or in combination of any two or more at any ratios.

When a combination of cesium fluoride and imidazole is used as a reaction accelerator, there are no restrictions as to the amount of imidazole used, but when the amount of each reactant compound is considered to be 100 mol%, the amount of imidazole used may typically be 0.1 mol% or more, for example 0.2 mol% or more, or 0.3 mol% or more, and may typically be 30 mol% or less, for example 20 mol% or less, or 15 mol% or less. Likewise, there are no restrictions as to the amount of cesium fluoride used, but when the amount of each reactant compound is considered to be 100 mol%, the amount of cesium fluoride used may typically be 0.1 mol% or more, for example 0.2 mol% or more, or 0.3 mol% or more, and may typically be 30 mol% or less, for example 20 mol% or less, or 15 mol% or less. The reaction acceleration effect of the coexistence of imidazole and cesium fluoride is effective in both reactions using the silane compound (A) and the silane compound (B), but is particularly effective in reactions using the silane compound (A).

When a base is used as a reaction accelerator, the type of the base is not particularly limited, but examples thereof include amines having 1 to 3 linear- or branched-chain alkyl groups having 1 to 10 carbon atoms, such as triethylamine (Et₃N), diisopropylamine (i-Pr₂NH), and diisopropyl ethylamine (i-Pr₂EtN). There are no restrictions as to the amount of the base used is not particularly limited, but when the amount of each reactant compound is considered to be 100 mol%, the amount of the base used may be 0.1 mol% or more, or 5 mol% or more, and may be 120 mol% or less, or 100 mol% or less.

When a boron compound is used as a reaction accelerator, the type of the boron compound is not particularly limited, but examples thereof include mono/di/tri {(fluoro)alkyl}boranes having 1 to 3 linear- or branched-chain alkyl groups having 1 to 10 carbon atoms or haloalkyl groups derived from such alkyl groups via substitution with 1 to 20 halogen atoms. Examples include, but are not limited to, trispentafluoro borane. There are no restrictions as to the amount of the boron compound used is not particularly limited, but when the amount of each reactant compound is considered to be 100 mol%, the amount of the boron compound may be 0.05 mol% or more, or 0.1 mol% or more, and may be 10 mol% or less, or 3 mol% or less.

Although not bound by theory, the mechanism of action of the reaction accelerator mentioned above is estimated as follows. The silane compound(s) (A) and/or (B), as well as the second silane compound optionally used, is deemed to be activated by the reaction accelerator coexisting in the reaction system, resulting in the acceleration of the amidation reaction.

For the details of such reaction accelerators, various publications can be referred to, such as Blandez et al., Catalysis Science & Technology, 2015, Vol. 5, pp.2167-2173; Bonar-Law et al., Tetrahedron Letters, 1990, Vol.31, No.46, pp.6725-6728; Corru et al., Journal of Organometallic Chemistry, 1976, Vol.114, pp.135-144; Davis et al., Tetrahedron Letters, 1995, Vol.36, No.18, pp.3269-3272; Dunne et al., Journal of the American Chemical Society, 2011, Vol 133, No.42, pp.16782-16785;Horner et al., Journal of Organometallic Chemistry, 1985, Vol.282, pp.155-174.

### *Reaction procedure:

Amidation by the production method according to the present invention can be carried out by contacting the reactant compound(s) with the silane compound(s) (A) and/or (B), and optionally with the second silane compound, the aminosilane catalyst, the Lewis acid catalyst, the phosphorus compound, and/or the other ingredients if used. The order of contacting these components is not limited, and all may be mixed simultaneously or sequentially in any order. Specific examples include, but are not limited to, the following procedures.

For example, if there are no other carboxyl groups, amino groups, and/or other reactive groups than the carboxyl and amino groups being the target of the desired amidation reaction, or if such other carboxyl groups, amino groups, or other reactive groups are protected with protective groups, the order of contact is not particularly limited. However, from the standpoint of reaction efficiency, it is preferred to mix the reactant compounds and the silane compound(s) (A) and/or (B) simultaneously, optionally with the second silane compound, the amino silane catalyst, the Lewis acid catalyst, the phosphorus compound, and/or the other components. When each of other carboxyl groups and/or amino groups than the target carboxyl and amino groups is protected with a protective group, it would be necessary to carry out a protection step to introduce the protective group before the amidation reaction, and also a deprotection step to remove the protective group after the amidation reaction.

On the other hand, when, as in the case of Embodiment (3), the terminal carboxyl group of the first amino acid or peptide (3-1) is reacted with the terminal carboxyl group of the second amino acid or peptide (3-2), the reaction may preferably be carried out in the following order: (a) the first amino acid or peptide (3-1) is contacted with the silane compound(s) (A) and/or (B); (b) the second silane compound optionally used is then brought into contact; and (c) the second amino acid or peptide (3-2) is then brought into contact. This procedure allows selective amidation reaction to occur between the terminal carboxyl group of the first amino acid or peptide (3-1) and the terminal amino group of the second amino acid or peptide (3-2) without protecting other carboxyl and/or amino groups of each reactant compound that are not subject to the amidation reaction. This reaction procedure is highly desirable from the standpoint of efficiency since it eliminates the need for a protection step to introduce a protecting group before the amidation reaction and a deprotection step to remove the protecting group after the amidation reaction. In this reaction procedure, the timing of addition of optionally used components (such as the aminosilane catalyst, the Lewis acid catalyst, the phosphorus compound, and/or the other ingredients) is not restricted and can be added at any stage.

The silane compound(s) (A) and/or (B), as well as the second silane compound optionally used, may be either added to the reaction system directly as the final compound or may be generated in the reaction system by reacting raw material compounds for the desired compound in the system. For example, when a silyl imidazole compound (e.g., dimethylsilyl(2-methyl)imidazole) is used as the second silane compound, the desired silylimidazole compound can be synthesized by adding a corresponding silyl halide (e.g., silyl chloride) and a corresponding imidazole compound (e.g., 2-methylimidazole) in the system and causing them to react each other. In this case, since the amount of hydrogen halide (e.g., hydrochloric acid) generated is equivalent to the amount of the silyl imidazole compound, it is necessary to use two or more equivalents of the imidazole compound to the silyl halide. A person skilled in the art can select and implement various methods for the in-system synthesis of such silane compounds based on technical common knowledge.

From the viewpoint of increasing reaction efficiency, amidation may be carried out in an organic solvent. The type of the organic solvent is not particularly limited, examples thereof including: aromatic hydrocarbons such as toluene and xylene; pentane; ethers such as petroleum ether, 1-methyltetrahydrofuran (1-MeTHF), diisopropylether (i-Pr₂O), diethylether (Et₂O), cyclopentylmethylether (CPME): esters such as ethyl acetate (AcOEt); and organic acids such as acetic acid. These organic solvents may be used either singly or in combination of any two or more.

The concentration of each reactant compound in the reaction system is not restricted, but from the viewpoint of increasing reaction efficiency, it may be between 2 volume% and 70 volume%.

### *Reaction conditions:

The conditions for the amidation reaction in the production method according to the present invention are not limited as long as the reaction proceeds, although examples of the conditions for each reaction step are described below.

When the reactant compound(s) is mixed with the silane compound(s) (A) and/or (B) simultaneously, along with optionally-used components such as the second silane compound, the aminosilane catalyst, the Lewis acid catalyst, the phosphorus compound, and/or the other ingredients, the reaction condition are not limited as long as the reaction proceeds, examples thereof being as follows.

The reaction temperature is not limited as long as the reaction proceeds, although it may typically be 0°C or more, particularly 10°C or more, especially 20°C or more, and also may typically be 100°C or less, particularly 80°C or less, especially 60°C or less. In this regard, the production method according to the present invention is advantageous because the amidation reaction sufficiently proceeds even under a mild condition, e.g., at a temperature of 60°C or less.

The reaction pressure is not limited as long as the reaction proceeds, and the reaction may be carried out under reduced, normal, or pressurized pressure, but may typically be carried out under normal pressure.

The reaction atmosphere is also not limited as long as the reaction proceeds, but the reaction may be carried out under an atmosphere of inert gas such as argon or nitrogen.

The reaction time is also not limited as long as the reaction proceeds. However, in order for the reaction to proceed sufficiently and efficiently, the reaction time may be 10 minutes or more, particularly 20 minutes or more, or 30 minutes or more, and may be 80 hours or less, particularly 60 hours or less, or 50 hours or less. When the amino silane catalyst is added to the reaction system, then the reaction time can be reduced significantly.

On the other hand, in the case of Embodiment (3) in which the reaction is carried out in such an order as to omit the protection step and the deprotection step, i.e., in such a manner that (a) the first amino acid or peptide (3-1) is contacted with the silane compound(s) (A) and/or (B); (b) the second silane compound optionally used is then brought into contact; and (c) the second amino acid or peptide (3-2) is then brought into contact, the reaction conditions for each of steps (a) to (c) are not limited as long as the reaction proceeds, examples thereof being as follows.

The reaction temperature at step (a) is not limited as long as the reaction proceeds, although it may typically be 0°C or more, particularly 10°C or more, especially 20°C or more, and also may typically be 100°C or less, particularly 80°C or less, especially 60°C or less.

The reaction time at step (a) is not limited as long as the reaction proceeds. However, in order for the reaction to proceed sufficiently and efficiently, the reaction time may be 10 minutes or more, particularly 20 minutes or more, or 30 minutes or more, and also may be 10 hours or less, particularly 5 hours or less, or 3 hours or less. When the amino silane catalyst is added to the reaction system, then the reaction time can be reduced significantly.

The reaction temperature at step (b) is not limited as long as the reaction proceeds, although it may typically be 0°C or more, particularly 10°C or more, especially 20°C or more, and also may typically be 100°C or less, particularly 80°C or less, especially 60°C or less.

The reaction time at step (b) is not limited as long as the reaction proceeds. However, in order for the reaction to proceed sufficiently and efficiently, the reaction time may be 10 minutes or more, particularly 20 minutes or more, or 30 minutes or more, and also may be 10 hours or less, particularly 5 hours or less, or 3 hours or less.

The reaction temperature at step (c) is not limited as long as the reaction proceeds, although it may typically be 0°C or more, particularly 10°C or more, especially 20°C or more, and also may typically be 100°C or less, particularly 80°C or less, especially 60°C or less.

The reaction time at step (c) is not limited as long as the reaction proceeds. However, in order for the reaction to proceed sufficiently and efficiently, the reaction time may be 10 minutes or more, particularly 20 minutes or more, or 30 minutes or more, and also may be 80 hours or less, particularly 60 hours or less, or 50 hours or less. When the amino silane catalyst is added to the reaction system, then the reaction time can be reduced significantly.

The reaction pressure and atmosphere at steps (a) to (c) are not limited as long as the reaction proceeds, although the reaction may typically be carried out under normal pressure and under an atmosphere of inert gas such as argon or nitrogen.

In all of these embodiments, the production method according to the present invention (or, if the method includes two or more steps, each of the steps) may be carried out in a sequential manner (batch) or in a continuous manner (flow). Details of specific procedures for implementing a sequential method (batch method) and a continuous method (flow method) are well known in the art.

### *Post-processing, etc. (e.g., purification and recovery):

The production method according to the present invention may include the step of carrying out various post-processing onto the amido compound produced via the amidation reaction.

For example, the amide compound formed may be isolated and purified according to conventional methods such as column chromatography, recrystallization, etc.

When T¹ is the protective group PG¹ and/or T² is the protective group PG², the amino group protected by PG¹ and/or the carboxyl group protected by PG² of the amide compound produced may be deprotected, optionally after post-processing steps such as isolation and purification.

The method of deprotecting the amino group protected by PG¹ is not particularly restricted, and various methods can be used depending on the type of protecting group PG¹. Examples include deprotection by hydrogenation, deprotection by weak acids, deprotection by fluorine ions, deprotection by one-electron oxidants, deprotection by hydrazine, and deprotection by oxygen. The deprotection by hydrogenation may be carried out by, e.g.; (a) a method of causing deprotection in the presence of hydrogen gas using a metal catalyst such as palladium, palladium-carbon, palladium hydroxide, palladium-carbon hydroxide, etc., as a reduction catalyst; and (b) a method of causing deprotection in the presence of a metal catalyst such as palladium, palladium-carbon, palladium hydroxide, palladium-carbon hydroxide, etc., using a hydrotreating reductant such as sodium borohydride, lithium aluminum hydride, lithium borohydride, diborane, etc.

The method of deprotecting the carboxyl group protected by PG² is not particularly restricted, and various methods can be used depending on the type of protecting group PG². Examples include deprotection by hydrogenation, deprotection by bases, and deprotection by weak acids. In the case of deprotection with a base, a strong base such as lithium hydroxide, sodium hydroxide, potassium hydroxide, etc. can be used.

After conducting the amidation reaction by the production method according to the present invention, the resulting amide compound (if necessary, after deprotection) can be subjected to the production method according to the present invention again as a new reactant compound and linked to another reactant compound by an amide bond.

For example, in the case of Embodiment (3), a peptide obtained via an amidation reaction through the production method according to the present invention can be subjected to the production method according to the present invention to thereby cause an amidation reaction between the terminal amino or carboxyl group of the peptide and the terminal carboxyl or amino group of another amino acid or peptide to produce a novel peptide. Thus, the production method according to the present invention can be repeated in sequence, whereby a peptide having any amino acid sequence can be synthesized in principle.

Alternatively, after an amidation reaction is carried out via the production method according to the present invention, the resulting peptide may be bound to another amino acid using any other amidation methods. An example of such other amidation methods is the method described in WO 2018/199147 A (PTL 3), which was also developed by the present inventors. The method described in WO 2018/199147 A is a method for forming an amide bond between the carboxyl group of a first amino acid or peptide and the amino group of a second amino acid in the presence of a metal catalyst such as a specific tantalum or niobium compound. Specifically, in the case of Embodiment (3), the carboxyl protective group PG² of the compound (3-2) may be selected from the protective groups that can react in the presence of a metal catalyst such as a certain tantalum or niobium compound used in the method described in WO 2018/199147 A. This compound (3-2) may be used in the production method according to the present invention to thereby produce the compound (3-3), which may be subjected to the method described in WO 2018/199147 A, thereby linking the compound (3-3) with another amino acid via amidation reaction.

### EXAMPLES

The present invention will be described in more detail below with reference to examples. However, the present invention should in no way be bound by the following examples, and can be implemented in any form within the scope that does not depart from the purpose of the invention.

Amide compounds were produced by the production method according to the present invention, in a manner described in each of the following examples.

In the following examples, unless otherwise stated, diastereomeric and enantiomeric ratios were determined by ¹H-NMR analysis (measuring instrument: JEOL 400SS, JEOL 400 MHz, solvent: CDCl₃).

### [Synthesis Example Group: Synthesis of Trialkoxy Silanes (Silane Compounds (A))]

### *General synthesis procedure:

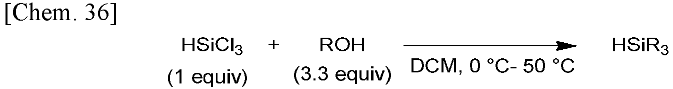

A two-necked round-bottom flask, heated and dried in a glove box under argon atmosphere, was equipped with a stirrer (samarium-cobalt), reflux cooler, and vacuum adapter. The flask was evacuated and refilled three times with nitrogen (N₂) gas, then filled with dry dichloromethane (16 mL) and trichlorosilane (HSiCl₃, 5.4 g, 40 mmol) and cooled to 0°C in an ice bath. Next, the alcohol corresponding to the alkoxy group of the trialkoxysilane to be synthesized (ROH, 3.3 equivalents, 132 mmol) was added dropwise and stirred at room temperature for 1 hour, then heated to 50°C and refluxed for 3 hours. After completion of the reaction, the solvent and unreacted alcohol were removed and distilled under reduced pressure and nitrogen (N₂) gas atmosphere (70-80°C/ 30 mmHg), the desired trialkoxysilane (HSiR₃) was obtained.

In the reaction formula above, R represents a halogen substitution alkoxy group.

### *Synthesis Example 1: synthesis of tris(2,2,2-trifluoroethoxy)silane

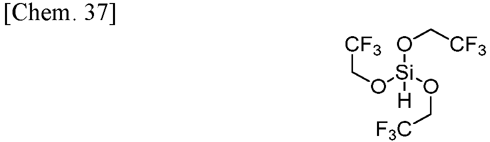

The reaction was carried out in accordance with the General Synthesis Procedure using 2,2,2-trifluoroethanol (CF₃CH₂OH) as an alcohol, followed by distillation at 70°C/30mmHg, whereby the title compound tris(2,2,2-trifluoroethoxy)silane ((CF₃CH₂O)₃SiH) was isolated as colorless liquid. The yield was 75%.

¹H NMR (400 MHz, CDCl₃, 24 °C): δ 4.48 (s, 1H), 4.14 (q, 6H, J = 8.3 Hz); ¹³C{¹H} NMR (100 MHz, CDCl₃, 24 °C): δ 123.6 (q, ¹J(C-F) = 276 Hz), 61.3 (q, ²J(C-F) = 37 Hz); ²⁹Si NMR (80 MHz, CDCl₃, 24 °C): -59.0.

### *Synthesis Example 2: synthesis of tris((1,1,1,3,3,3-hexafluoropropan-2-yl)oxy)silane

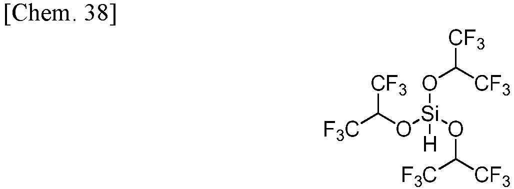

The reaction was carried out in accordance with the General Synthesis Procedure using 1,1,1,3,3,3-hexafluoro-2-propanol ((CF₃)₂CH₂OH) as an alcohol, followed by distillation at 70°C/30mmHg, whereby the title compound tris((1,1,1,3,3,3-hexafluoropropan-2-yl)oxy)silane (((CF₃)₂CH₂O)₃SiH) was isolated as colorless liquid. The yield was 60%.

¹H NMR (400 MHz, CDCl₃, 24 °C): δ 4.76 (s, 1H), 4.63-4.60 (m, 3H); ¹³C{¹H} NMR (100 MHz, CDCl₃, 24 °C): δ 120.6 (q, ¹J(C-F) = 280 Hz), 70.3 (quin, ²J(C-F) = 35 Hz); ²⁹Si NMR (80 MHz, CDCl₃, 24 °C): -62.8.

### *Synthesis Example 3: synthesis of tris(2,2,2-trichloroethoxy)silane

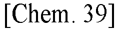

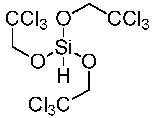

The reaction was carried out in accordance with the General Synthesis Procedure using 2,2,2-trichloroethanol (CCl₃CH₂OH) as an alcohol, without being followed by distillation, whereby the title compound tris(2,2,2-trichloroethoxy)silane ((CCl₃CH₂O)₃SiH) was isolated as colorless liquid. The yield was 94%.

¹H NMR (400 MHz, CDCl₃, 24 °C): δ 4.80 (s, 1H), 4.42 (s, 6H); ¹³C{¹H} NMR (100 MHz, CDCl₃, 24 °C): δ 97.6, 75.7; ²⁹Si NMR (80 MHz, CDCl₃, 24 °C): -61.3.

### *Synthesis Example 4: synthesis of tris(2,2,3,3-tetrafluoropropoxy)silane

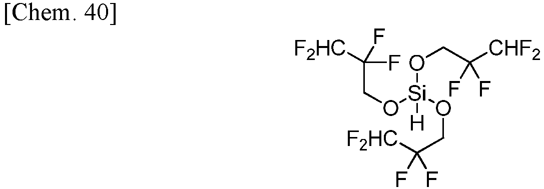

The reaction was carried out in accordance with the General Synthesis Procedure using 2,2,3,3-tetrafluoro-1-propanol (CF₂HCF₂CH₂OH) as an alcohol, followed by distillation at 120°C/30mmHg, whereby the title compound tris(2,2,3,3-tetrafluoropropoxy)silane ((CF₂HCF₂CH₂0)₃SiH) was isolated as colorless liquid. The yield was 71%.

¹H NMR (400 MHz, CDCl₃, 24 °C): δ 6.02-5.75 (m, 3H), 4.44 (s, 1H), 4.20-4.13 (m, 6H); ¹³C{¹H} NMR (100 MHz, CDCl₃, 24 °C): δ 114.7 (tt, ¹J(C-F) = 248 Hz, ²J(C-F) = 26.7 Hz), 109.4 (tt, ¹J(C-F) = 214 Hz, ²J(C-F) = 31.4 Hz), 60.5 (t, ²J(C-F) = 29.5 Hz); ²⁹Si NMR (80 MHz, CDCl₃, 24 °C): -57.9.

### [Example Group A: Amidation Reaction between Carbonic Acid Compound and Amino Compound using Silane Compound (A)]

### *Example a1: synthesis of N-(4-methoxybenzyl)propionamide

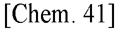

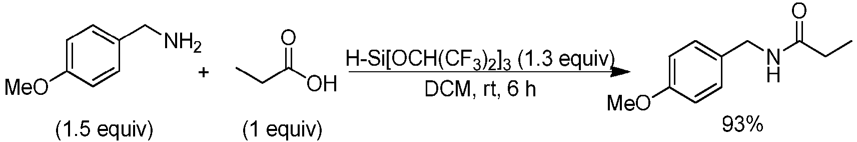

A 5.0-mL screw-cap vial heated and dried in a glove box was charged, under an argon atmosphere, with a stirrer (samarium-cobalt), propanoic acid ( 0.5 mmol, 1 equivalent), and 4-methoxybenzylamine (0.75 mmol, 1.5 mmol). This mixture was combined with HSi[OCH(CF₃)₂]₃ (0.65 mmol, 1.3 equivalents) (generation of hydrogen gas was observed.), and then mixed and dissolved in dry DCM (0.5 mL, 1M), and the vial was sealed. The vial was removed from the glove box and stirred vigorously for 6 hours at room temperature under an argon atmosphere. The progress of the reaction was monitored by TLC analysis. Once the reaction completed, and the reaction mixture diluted with CHCl₃ (3.0 mL) and transferred by a pipette to a silica gel column, and the vial and pipette used were washed with CHCl₃ (5.0mL). The resulting reaction product was purified with flash column chromatography using 0 to 40 % AcOEt in hexane, whereby N-(4-methoxybenzyl)propionamide was obtained as a white solid. The yield was 93%.

### *Example a2: synthesis of N-benzyl -N-methyl propionamide

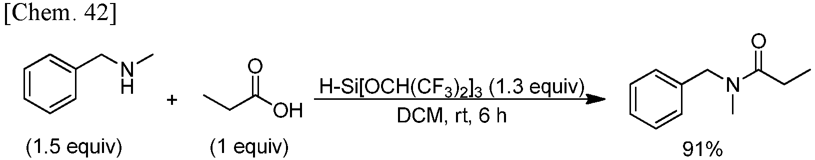

The reaction was carried out in the same manner as in Example a1 except that N-methyl benzylamine (0.75mmol, 1.5 equivalents) was used instead of 4-methoxybenzylamine, whereby N-benzyl -N-methyl propionamide was obtained as a white solid. The yield was 91%.

### [Example Group B: Intramolecular Amidation Reaction of Amino Carbonic Acid Compound using Silane Compound (A)]

### *Example b1: synthesis of (S)-tert-butyl (2-oxoazepan-3-yl)carbamate

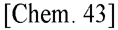

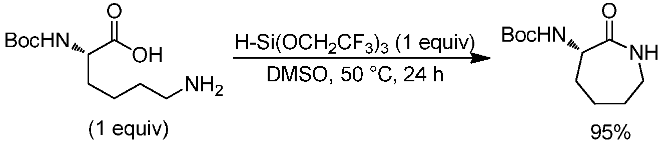

A 5.0-mL screw-cap vial heated and dried in a glove box was charged, under an argon atmosphere, with a stirrer (samarium-cobalt), Boc-Lys-OH(0.5mmol, 1 equivalent), and HSi(OCH₂CF₃)₃(0.5mmol, 1 equivalent), and then mixed and dissolved in dry DMSO (0.5mL, 1M), and the vial was sealed. The vial was removed from the glove box, placed in an oil bath heated to 50°C, and then stirred vigorously for 24 hours. The progress of the reaction was monitored by TLC analysis. Once the reaction completed, and the reaction mixture diluted with CHCl₃ (3.0mL), and transferred by a pipette to a silica gel column. The vial and the pipette used were washed with CHCl₃ (5.0mL). The resulting reaction product was purified with flash column chromatography using 0 to 40 % AcOEt in hexane, whereby (S)-tert-butyl (2-oxoazepan-3-yl)carbamate was obtained as a white solid. The yield was 95%.

### [Example Group C: Amidation Reaction between two Amino Acid Compounds using Silane Compound (A) (without Protective Group)]

### *Example c1: synthesis of H-L-Phe-L-Ala-Ot-Bu (1)

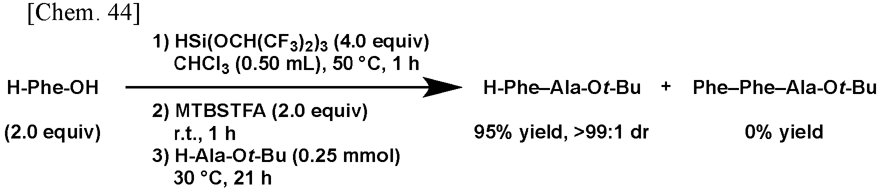

A 5-mL screw-cap vial heated and dried in a glove box was charged, under an argon atmosphere, with a stirrer (samarium-cobalt), L-phenylalanine (H-L-Phe-OH, 82.6mg, 0.5mmol), tris(hexafluoroisopropoxy)silane (HSi(OCH(CF₃)₂)₃, 530.2mg, 1.0mmol), and chloroform (0.5 mL), and sealed with a septum and a screw cap. The reaction vial was removed from the glove box, placed in an oil bath heated to 50°C, then stirred vigorously for 1 hour. The screw cap was removed, and use a microsyringe on top of the septum to remove the N-tert-butyldimethylsilyl-N-methyltrifluoroacetamide (MTBSTFA, 12 0.7 mg, 0.5 mg, 0.5 mg (mmol) was added, capped with a screw cap, and stirred vigorously for 1 hour at room temperature. The screw cap was removed, N-tert-butyldimethylsilyl-N-methyl trifluoroacetamide (MTBSTFA, 120.7mg, 0.5mmol) was added through the septum using a microsyringe, and the vial was sealed with the screw cap again, and was stirred vigorously at room temperature for 1 hour. The screw cap was removed again, L-alanine - tert-butyl ester (H-L-Ala-Ot-Bu, 36.3mg, 0.25mmol) was added through the septum using a microsyringe, and the vial was sealed with the screw cap again, and stirred vigorously for 21 hours in an oil bath heated to 30°C. The reaction vial was removed from the oil bath and left to stand until it reached to room temperature, and the reaction mixture was diluted with a 1% methanol/chloroform mixture (3.0 mL), and the product was transferred to a previously prepared silica gel column (1% methanol/chloroform mixture) using a Pasteur pipette. This procedure was repeated three more times, and the screw cap, septum, and the Pasteur pipette used was washed with the same mixture. The reaction product was purified with silica gel column chromatography (1-5% methanol/chloroform mixture), whereby the desired dipeptide (H-L-Phe-L-Ala-Ot-Bu, 69.2mg, 95%, >99:1dr) was obtained as a white solid.

### *Example c2: synthesis of H-L-Phe-L-Ala-Ot-Bu (2)

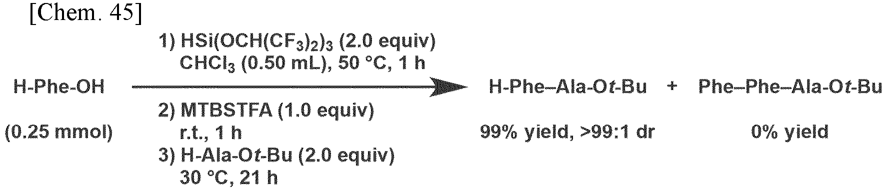

A 5-mL screw-cap vial heated and dried in a glove box was charged, under an argon atmosphere, with a stirrer (samarium-cobalt), L-phenylalanine (H-L-Phe-OH, 41.3mg, 0.25mmol), tris(hexafluoroisopropoxy)silane (HSi(OCH(CF₃)₂)₃, 265.1mg, 0.5mmol), and chloroform (0.5 mL), and sealed with a septum and a screw cap. The reaction vial was removed from the glove box, placed in an oil bath heated to 50°C, and then stirred vigorously for 1 hours. The screw cap was removed, through the septum using a microsyringe N-tert-butyldimethylsilyl-N-methyl trifluoroacetamide (MTBSTFA, 60.3mg, 0.25mmol) was added, and the vial was sealed with the screw cap again, and was stirred vigorously at room temperature for 1 hour. The screw cap was removed again, through the septum using a microsyringe L-alanine -tert-butyl ester (H-L-Ala-Ot-Bu, 72.6mg, 0.5mmol) was added, and the vial was sealed with the screw cap again, in an oil bath heated to 30°C stirred vigorously for 21 hours. The reaction vial was removed from the oil bath and left to stand until it reached to room temperature, and the reaction mixture was diluted with a 1% methanol/chloroform mixture (3.0 mL), and the product was transferred to a previously prepared silica gel column (1% methanol/chloroform mixture) using a Pasteur pipette. This procedure was repeated three more times, and the screw cap, septum, and the Pasteur pipette used was washed with the same mixture. The reaction product was purified with silica gel column chromatography (1-5% methanol/chloroform mixture), whereby the desired dipeptide (H-L-Phe-L-Ala-Ot-Bu, 72.7mg, 99%, >99:1dr) was obtained as a white solid.

### *Example c3: synthesis of H-L-Phe-L-Ser(t-Bu)-Ot-Bu

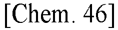

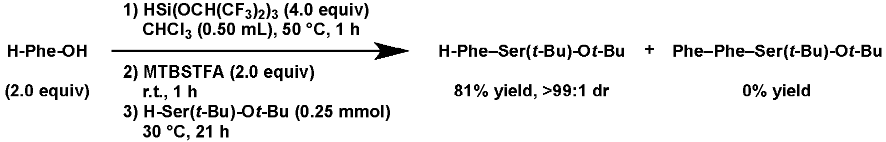

A 5-mL screw-cap vial heated and dried in a glove box was charged, under an argon atmosphere, with a stirrer (samarium-cobalt), L-phenylalanine (H-L-Phe-OH, 82.6mg, 0.5mmol), tris(hexafluoroisopropoxy)silane (HSi(OCH(CF₃)₂)₃, 530.2mg, 1.0mmol), and chloroform (0.5 mL), and sealed with a septum and a screw cap. The reaction vial was removed from the glove box, placed in an oil bath heated to 50°C, and then stirred vigorously for 1 hours. The screw cap was removed, through the septum using a microsyringe N-tert-butyldimethylsilyl-N-methyl trifluoroacetamide (MTBSTFA, 120.7mg, 0.5mmol) was added, and the vial was sealed with the screw cap again, and was stirred vigorously at room temperature for 1 hour. The screw cap was removed again, through the septum using a microsyringe L-serine -tert-butyl ester (H-L-Ser(t-Bu)-Ot-Bu, 54.3mg, 0.25mmol) was added, and the vial was sealed with the screw cap again, in an oil bath heated to 30°C stirred vigorously for 21 hours. The reaction vial was removed from the oil bath and left to stand until it reached to room temperature, and the reaction mixture was diluted with a 1% methanol/chloroform mixture (3.0 mL), and the product was transferred to a previously prepared silica gel column (1% methanol/chloroform mixture) using a Pasteur pipette. This procedure was repeated three more times, and the screw cap, septum, and the Pasteur pipette used was washed with the same mixture. The reaction product was purified with silica gel column chromatography (1-5% methanol/chloroform mixture), whereby the desired dipeptide (H-L-Phe-L-Ser(t-Bu)-Ot-Bu, 73.6mg, 81%, >99:1dr) was obtained as a colorless, gum-like compound.

### *Example c4: synthesis of H-L-Phe-L-Val-Ot-Bu (1)

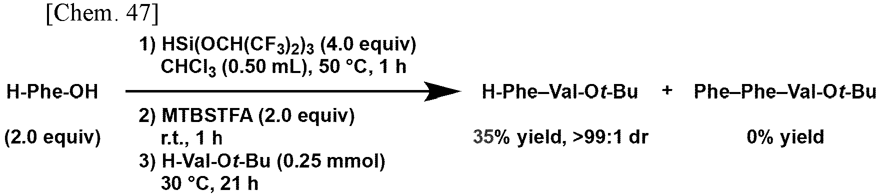

A 5-mL screw-cap vial heated and dried in a glove box was charged, under an argon atmosphere, with a stirrer (samarium-cobalt), L-phenylalanine (H-L-Phe-OH, 82.6mg, 0.5mmol), tris(hexafluoroisopropoxy)silane (HSi(OCH(CF₃)₂)₃, 530.2mg, 1.0mmol), and chloroform (0.5 mL), and sealed with a septum and a screw cap. The reaction vial was removed from the glove box, placed in an oil bath heated to 50°C, and then stirred vigorously for 1 hours. The screw cap was removed, through the septum using a microsyringe N-tert-butyldimethylsilyl-N-methyl trifluoroacetamide (MTBSTFA, 120.7mg, 0.5mmol) was added, and the vial was sealed with the screw cap again, and was stirred vigorously at room temperature for 1 hour. The screw cap was removed again, through the septum using a microsyringe L-valine-tert-butyl ester (H-L-Val-Ot-Bu, 43.3mg, 0.25mmol) was added, and the vial was sealed with the screw cap again, in an oil bath heated to 30°C stirred vigorously for 21 hours. The reaction vial was removed from the oil bath and left to stand until it reached to room temperature, and the reaction mixture was diluted with a 1% methanol/chloroform mixture (3.0 mL), and the product was transferred to a previously prepared silica gel column (1% methanol/chloroform mixture) using a Pasteur pipette. This procedure was repeated three more times, and the screw cap, septum, and the Pasteur pipette used was washed with the same mixture. The reaction product was purified with silica gel column chromatography (1-5% methanol/chloroform mixture), whereby the desired dipeptide (H-L-Phe-L-Val-Ot-Bu, 28.0mg, 35%, >99:1dr) was obtained as a colorless, gum-like compound.

### *Example c5: synthesis of H-L-Phe-L-Val-Ot-Bu (2)

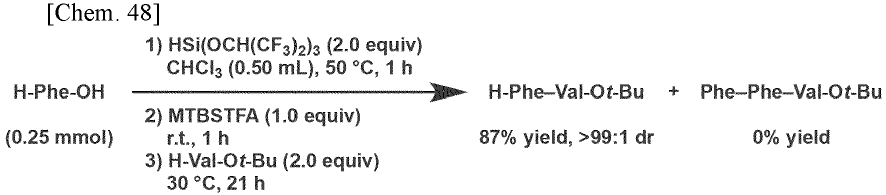

A 5-mL screw-cap vial heated and dried in a glove box was charged, under an argon atmosphere, with a stirrer (samarium-cobalt), L-phenylalanine (H-L-Phe-OH, 41.3mg, 0.25mmol), tris(hexafluoroisopropoxy)silane (HSi(OCH(CF₃)₂)₃, 265.1mg, 0.5mmol), and chloroform (0.5 mL), and sealed with a septum and a screw cap. The reaction vial was removed from the glove box, placed in an oil bath heated to 50°C, and then stirred vigorously for 1 hours. The screw cap was removed, through the septum using a microsyringe N-tert-butyldimethylsilyl-N-methyl trifluoroacetamide (MTBSTFA, 60.3mg, 0.25mmol) was added, and the vial was sealed with the screw cap again, and was stirred vigorously at room temperature for 1 hour. The screw cap was removed again, through the septum using a microsyringe L-valine-tert-butyl ester (H-L-Val-Ot-Bu, 86.6mg, 0.5mmol) was added, and the vial was sealed with the screw cap again, in an oil bath heated to 30°C stirred vigorously for 21 hours. The reaction vial was removed from the oil bath and left to stand until it reached to room temperature, and the reaction mixture was diluted with a 1% methanol/chloroform mixture (3.0 mL), and the product was transferred to a previously prepared silica gel column (1% methanol/chloroform mixture) using a Pasteur pipette. This procedure was repeated three more times, and the screw cap, septum, and the Pasteur pipette used was washed with the same mixture. The reaction product was purified with silica gel column chromatography (1-5% methanol/chloroform mixture), whereby the desired dipeptide (H-L-Phe-L-Val-Ot-Bu, 69.3mg, 87%, >99:1dr) was obtained as a colorless, gum-like compound.

### *Example c6: synthesis of H-L-Ala-L-Ala-Ot-Bu (1)

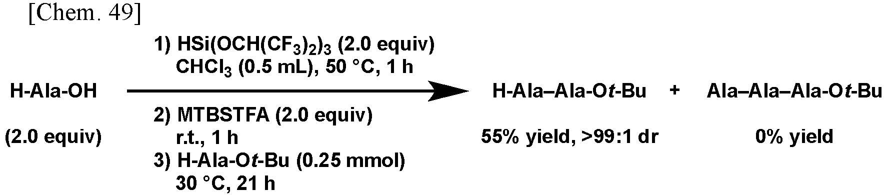

A 5-mL screw-cap vial heated and dried in a glove box was charged, under an argon atmosphere, with a stirrer (samarium-cobalt), L-alanine (H-L-Ala-OH, 44.5mg, 0.5mmol), tris(hexafluoroisopropoxy)silane (HSi(OCH(CF₃)₂)₃, 265.1mg, 0.5mmol), and chloroform (0.5 mL), and sealed with a septum and a screw cap. The reaction vial was removed from the glove box, placed in an oil bath heated to 50°C, and then stirred vigorously for 1 hours. The screw cap was removed, through the septum using a microsyringe N-tert-butyldimethylsilyl-N-methyl trifluoroacetamide (MTBSTFA, 120.7mg, 0.5mmol) was added, and the vial was sealed with the screw cap again, and was stirred vigorously at room temperature for 1 hour. The screw cap was removed again, through the septum using a microsyringe L-alanine -tert-butyl ester (H-L-Ala-Ot-Bu, 36.3mg, 0.25mmol) was added, and the vial was sealed with the screw cap again, in an oil bath heated to 30°C stirred vigorously for 21 hours. The reaction vial was removed from the oil bath and left to stand until it reached to room temperature, and the reaction mixture was diluted with a 1% methanol/chloroform mixture (3.0 mL), and the product was transferred to a previously prepared silica gel column (1% methanol/chloroform mixture) using a Pasteur pipette. This procedure was repeated three more times, and the screw cap, septum, and the Pasteur pipette used was washed with the same mixture. The reaction product was purified with silica gel column chromatography (5 to 30%methanol/chloroform mixture), whereby the desired dipeptide (H-L-Ala-L-Ala-Ot-Bu, 30.0mg, 55%, >99:1dr) was obtained as a colorless, gum-like compound.

### *Example c7: synthesis of H-L-Ala-L-Ala-Ot-Bu (2)

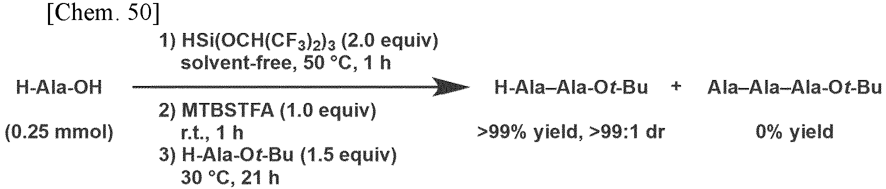

A 5-mL screw-cap vial heated and dried in a glove box was charged, under an argon atmosphere, with a stirrer (samarium-cobalt), L-alanine (H-L-Ala-OH, 22.3mg, 0.25mmol), tris(hexafluoroisopropoxy)silane (HSi(OCH(CF₃)₂)₃, 265.1mg, 0.5mmol), and sealed with a septum and a screw cap. The reaction vial was removed from the glove box, placed in an oil bath heated to 50°C, and then stirred vigorously for 1 hours. The screw cap was removed, through the septum using a microsyringe N-tert-butyldimethylsilyl-N-methyl trifluoroacetamide (MTBSTFA, 60.3mg, 0.25mmol) was added, and the vial was sealed with the screw cap again, and was stirred vigorously at room temperature for 1 hour. The screw cap was removed again, through the septum using a microsyringe L-alanine -tert-butyl ester (H-L-Ala-Ot-Bu, 54.5mg, 0.375mmol) was added, and the vial was sealed with the screw cap again, in an oil bath heated to 30°C stirred vigorously for 21 hours. The reaction vial was removed from the oil bath and left to stand until it reached to room temperature, and the reaction mixture was diluted with a 1% methanol/chloroform mixture (3.0 mL), and the product was transferred to a previously prepared silica gel column (1% methanol/chloroform mixture) using a Pasteur pipette. This procedure was repeated three more times, and the screw cap, septum, and the Pasteur pipette used was washed with the same mixture. The reaction product was purified with silica gel column chromatography (5 to 30%methanol/chloroform mixture), whereby the desired dipeptide (H-L-Ala-L-Ala-Ot-Bu, 42.1mg, 78%, >99:1dr) was obtained as a colorless, gum-like compound.

### *Example c8: synthesis of H-L-Ile-L-Ala-Ot-Bu

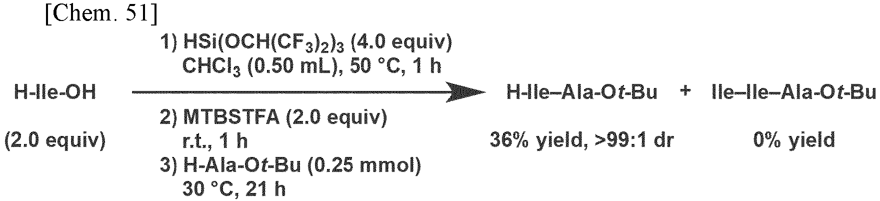

A 5-mL screw-cap vial heated and dried in a glove box was charged, under an argon atmosphere, with a stirrer (samarium-cobalt), L- 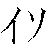 leucine (H-L-Ile-OH, 65.6mg, 0.5mmol), tris(hexafluoroisopropoxy)silane (HSi(OCH(CF₃)₂)₃, 530.2mg, 1.0mmol), and chloroform (0.5 mL), and sealed with a septum and a screw cap. The reaction vial was removed from the glove box, placed in an oil bath heated to 50°C, and then stirred vigorously for 1 hours. The screw cap was removed, through the septum using a microsyringe N-tert-butyldimethylsilyl-N-methyl trifluoroacetamide (MTBSTFA, 120.7mg, 0.5mmol) was added, and the vial was sealed with the screw cap again, and was stirred vigorously at room temperature for 1 hour. The screw cap was removed again, through the septum using a microsyringe L-alanine -tert-butyl ester (H-L-Ala-Ot-Bu, 36.3mg, 0.25mmol) was added, and the vial was sealed with the screw cap again, in an oil bath heated to 30°C stirred vigorously for 21 hours. The reaction vial was removed from the oil bath and left to stand until it reached to room temperature, and the reaction mixture was diluted with a 1% methanol/chloroform mixture (3.0 mL), and the product was transferred to a previously prepared silica gel column (1% methanol/chloroform mixture) using a Pasteur pipette. This procedure was repeated three more times, and the screw cap, septum, and the Pasteur pipette used was washed with the same mixture. silica gel column chromatography (1 to 10%methanol /chloroform mixture ) 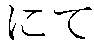 the reaction product was purified, desired dipeptide (H-L-Ile-L-Ala-Ot-Bu, 23.0mg, 36%, >99: 1dr) was obtained as a colorless, gum-like compound.

### *Example c9: synthesis of H-L-Phe-L-Ala-Ot-Bu

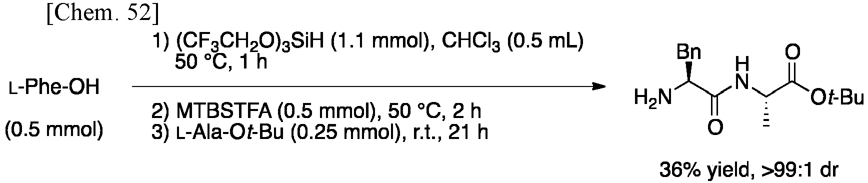

A 5-mL screw-cap vial heated and dried in a glove box was charged, under an argon atmosphere, with a stirrer (samarium-cobalt), L-phenylalanine (H-L-Phe-OH, 82.6mg, 0.5mmol), tristrifluoroethoxy silane (HSi(OCH₂CF₃)₃, 358.8mg, 1.0mmol), and chloroform (0.5 mL), and sealed with a septum and a screw cap. The reaction vial was removed from the glove box, placed in an oil bath heated to 50°C, and then stirred vigorously for 1 hours. The screw cap was removed, through the septum using a microsyringe N-tert-butyldimethylsilyl-N-methyl trifluoroacetamide (MTBSTFA, 120.7mg, 0.5mmol) was added, and the vial was sealed with the screw cap again, in an oil bath heated to 50°C stirred vigorously for 2 hours The screw cap was removed again, through the septum using a microsyringe L-alanine -tert-butyl ester (H-L-Ala-Ot-Bu, 36.3mg, 0.25mmol) was added, and the vial was sealed with the screw cap again, and stirred vigorously at room temperature for 21 hours. The reaction vial was removed from the oil bath and left to stand until it reached to room temperature, and the reaction mixture was diluted with a 1% methanol/chloroform mixture (3.0 mL), and the product was transferred to a previously prepared silica gel column (1% methanol/chloroform mixture) using a Pasteur pipette. This procedure was repeated three more times, and the screw cap, septum, and the Pasteur pipette used was washed with the same mixture. The reaction product was purified with silica gel column chromatography (1-5% methanol/chloroform mixture), whereby the desired dipeptide (H-L-Phe-L-Ala-Ot-Bu, 26.2mg, 36%, >99:1dr) was obtained as a white solid.

### [Example Group D: Amidation Reactions between two Amino Acid Compounds using Silane Compound (A) (with Protective Group)]

### *General Synthesis Procedure:

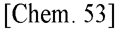

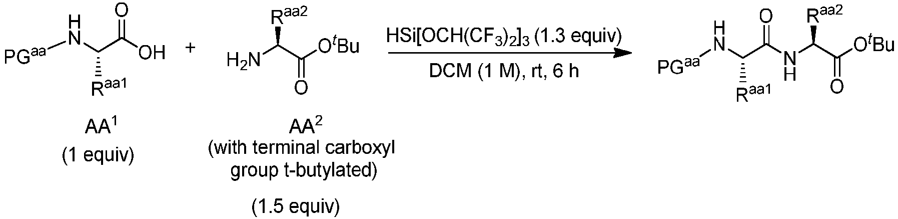

A 5.0-mL screw-cap vial heated and dried in a glove box was charged, under an argon atmosphere, with a stirrer (samarium-cobalt), a first amino acid AA¹ with its terminal amino group protected (0.5mmol, 1 equivalent), and a second amino acid AA² with its terminal carboxyl group tert-butylated (0.75mmol, 1.5 equivalents). This mixture was combined with tris(1,1,1,3,3,3-hexafluoroisopropoxy)silane (HSi[OCH(CF₃)₂]₃, 0.65mmol, 1.3 equivalents) (generation of hydrogen gas was observed.), and then mixed and dissolved in dry DCM (0.5 mL, 1M), and the vial was sealed. The vial was removed from the glove box and stirred vigorously for 6 hours at room temperature under an argon atmosphere. The reaction was monitored with TLC analysis. Once the reaction completed, and the reaction mixture diluted with CHCl₃ (3.0mL), and transferred by a pipette to a silica gel column. The vial and pipette used were washed with CHCl₃ (5.0mL). The reaction mixture was purified with flash column chromatography using 10 to 100% AcOEt in hexane or 0 to 10% MeOH in CHCl₃ as an eluate, whereby an analytically pure product was obtained.

In the reaction formula above, R^{aa1} represents the side chain of the first amino acid AA¹, R^{aa2} represents the side chain of the second amino acid AA², and PG^{aa} represents the terminal amino protective group of the first amino acid AA¹.

### *Example d1: synthesis of Boc-L-Ala-L-Ala-OtBu

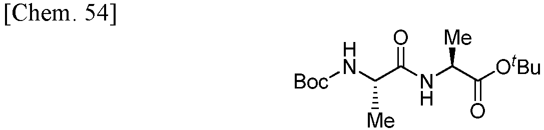

The reaction was carried out in accordance with the General Synthesis Procedure, using Boc-L-Ala as the first amino acid AA¹ and L-Ala-OtBu as the second amino acid AA², whereby the title compound Boc-L-Ala-L-Ala-OtBu was isolated as colorless liquid. The yield was 97%, and the diastereomer ratio was dr>99: 1.
R_{f} = 0.48 (40% AcOEt in hexane); [α]_{D}²⁵ = -20.0 (c 1.40, CHCl₃).

### *Example d2: synthesis of Bzo-L-Ala-L-Ala-OtBu

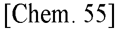

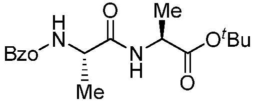

The reaction was carried out in accordance with the General Synthesis Procedure, using Bzo-L-Ala as the first amino acid AA¹ and L-Ala-OtBu as the second amino acid AA², whereby the title compound Bzo-L-Ala-L-Ala-OtBu was isolated as a white solid. The yield was 98%, and the diastereomer ratio was dr>99: 1. R_{f} = 0.60 (70% AcOEt in hexane).

¹H NMR (400 MHz, CDCl₃, 24 °C): δ 7.81-7.79 (m, 2H), 7.51-7.47 (m, 1H), 7.43-7.39 (m, 2H), 7.02 (br d, 1H, J = 6.9 Hz), 6.84 (br d, 1H, J = 5.9 Hz), 4.77 (quin, 1H, J = 7.1 Hz), 4.44 (quin, 1H, J = 7.1 Hz), 1.51 (d, 3H, J = 6.9 Hz), 1.47 (s, 9H), 1.37 (d, 3H, J = 7.1 Hz); ¹³C{¹H} NMR (100 MHz, CDCl₃, 24 °C): δ 172.0, 171.8, 167.0, 133.9, 13.7, 128.6, 127.2, 82.0, 49.5, 48.9, 28.0, 19.2, 18.3.

### *Example d3: synthesis of Boc-Gly-L-Ala-OtBu

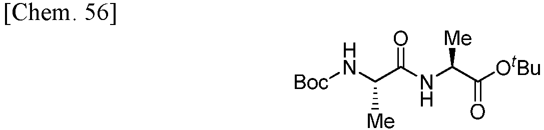

The reaction was carried out in accordance with the General Synthesis Procedure, using Boc-Gly as the first amino acid AA¹ and L-Ala-OtBu as the second amino acid AA², whereby the title compound Boc-Gly-L-Ala-OtBu was isolated as colorless liquid. The yield was 99%, and the diastereomer ratio was er>99: 1.

### *Example d4: synthesis of Boc-L-Val-L-Ala-OtBu

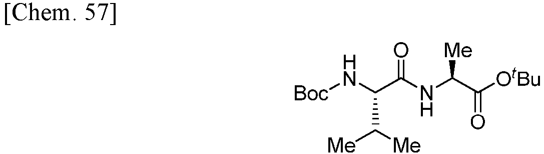

The reaction was carried out in accordance with the General Synthesis Procedure, using Boc-L-Val as the first amino acid AA¹ and L-Ala-OtBu as the second amino acid AA², whereby the title compound Boc-L-Val-L-Ala-OtBu was isolated as a white solid. The yield was 80%, and the diastereomer ratio was dr>99: 1.
R_{f}= 0.68 (40% AcOEt in hexane); [α]_{D}²⁵ = -12.8 (c 1.01, CHCl₃).

¹H NMR (400 MHz, CDCl₃, 24 °C): δ 6.40 (br s, 1H), 7.66 (br d, 1H, J = 6.4 Hz), 4.44 (quin, 1H, J = 7.1 Hz), 3.92 (br t, 1H, J = 7.8 Hz), 2.16-2.09 (m, 1H), 1.45 (s, 9H), 1.43 (s, 9H), 1.36 (d, 3 H, J = 7.1 Hz), 0.96 (d, 3H, J = 6.8 Hz), 0.90 (d, 3H, J = 6.8 Hz); ¹³C{¹H} NMR (100 MHz, CDCl₃, 24 °C): δ 172.0, 170.9, 155.9, 82.1, 79.9, 59.9, 48.7, 31.2, 28.4, 28.0, 19.3, 18.7, 17.7

### *Example d5: synthesis of Boc-L-Ile-L-Ala-OtBu

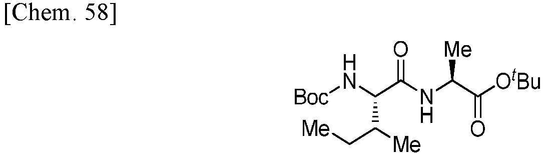

The reaction was carried out in accordance with the General Synthesis Procedure, using Boc-L-Ile as the first amino acid AA¹ and L-Ala-OtBu as the second amino acid AA², whereby the title compound Boc-L-Ile-L-Ala-OtBu was isolated as a white solid. The yield was 77%, and the diastereomer ratio was dr>99: 1.
R_{f}= 0.57 (40% AcOEt in hexane); [α]_{D}²⁵ = -12.6 (c 1.03, CHCl₃).

¹H NMR (400 MHz, CDCl₃, 24 °C): δ 6.45 (br s, 1H), 5.08 (br s, 1H), 4.42 (quin, 1H, J = 7.1 Hz), 3.94 (br t, 1H, J = 7.1 Hz), 2.00-1.86 (m, 1H), 1.59-1.47 (m, 1H), 1.44 (s, 9H), 1.42 (s, 9H), 1.35 (d, 3H, J = 7.1 Hz), 1.17-1.06 (m, 1H), 0.92 (d, 3H, J = 6.8 Hz), 0.89 (t, 3H, J = 7.5 Hz); ¹³C{¹H} NMR (100 MHz, CDCl₃, 24 °C): δ 171.9, 171.0, 155.8, 82.0, 79.9, 59.3, 48.7, 37.5, 28.4, 28.0, 24.8, 18.6, 15.6, 11.6.

### *Example d6: synthesis of Boc-L-Leu-L-Ala-OtBu

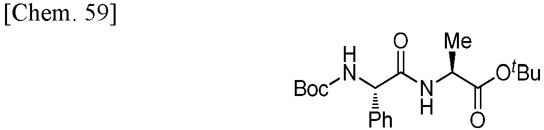

The reaction was carried out in accordance with the General Synthesis Procedure, using Boc-L-Leu as the first amino acid AA¹ and L-Ala-OtBu as the second amino acid AA², whereby the title compound Boc-L-Leu-L-Ala-OtBu was isolated as a white solid. The yield was 79%, and the diastereomer ratio was dr>99: 1.
R_{f}= 0.66 (50% AcOEt in hexane); [α]_{D}²⁵ = +92.4 (c 1.06, CHCl₃).

¹H NMR (400 MHz, CDCl₃, 24 °C): δ 7.36-7.30 (m, 5H), 6.31 (br d, 1H, J = 6.6 Hz), 5.74 (br s, 1H), 5.12 (br s, 1H), 4.38 (quin, 1H, J = 7.1 Hz), 1.40 (s, 9H), 1.38 (s, 9H), 1.37 (d, 3H, J = 7.5 Hz); ¹³C{¹H} NMR (100 MHz, CDCl₃, 24 °C): δ 171.5, 169.4, 155.2, 138.2, 129.1, 128.4, 27.3, 82.2, 80.1, 58.7, 49.1, 28.4, 27.9, 18.6.

### *Example d7: synthesis of Boc-L-Phe-L-Ala-OtBu

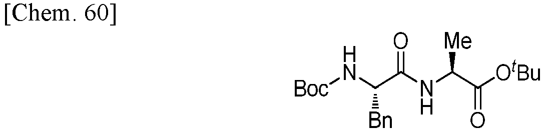

The reaction was carried out in accordance with the General Synthesis Procedure, using Boc-L-Phe as the first amino acid AA¹ and L-Ala-OtBu as the second amino acid AA², whereby the title compound Boc-L-Phe-L-Ala-OtBu was isolated as a white solid. The yield was 96%, and the diastereomer ratio was dr>99: 1.
R_{f}= 0.68 (50% AcOEt in hexane); [α]_{D}²⁵ = +17.7 (c 1.07, CHCl₃).

¹H NMR (400 MHz, CDCl₃, 24 °C): δ 7.28-7.18 (m, 5H), 6.44 (br d, 1H, J = 6.4 Hz), 4.99 (br s, 1H), 4.40-4.33 (m, 2H), 3.11-3.02 (m, 2H), 1.43 (s, 9H), 1.40 (s, 9H), 1.31 (d, 3H, J = 7.1 Hz); ¹³C{¹H} NMR (100 MHz, CDCl₃, 24 °C): δ 171.7, 170.6, 155.4, 136.6, 129.5, 128.7, 127.0, 82.1, 80.2, 55.7, 48.8, 38.6, 28.3, 28.0, 18.7.

### *Example d8: synthesis of Boc-L-Tyr(tBu)-L-Ala-OtBu

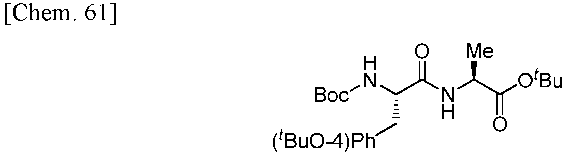

The reaction was carried out in accordance with the General Synthesis Procedure, using Boc-L-Tyr(^{t}Bu) as the first amino acid AA¹ and L-Ala-OtBu as the second amino acid AA², whereby the title compound Boc-L-Tyr(^{t}Bu)-L-Ala-OtBu was isolated as a white solid. The yield was 93%, and the diastereomer ratio was dr>99: 1.
R_{f}= 0.65 (40% AcOEt in hexane).

¹H NMR (400 MHz, CDCl₃, 24 °C): δ 7.08 (d, 2H, J = 8.5 Hz), 6.90 (d, 2H, J = 8.2 Hz), 6.44 (br d, 1H, J = 6.4 Hz), 4.95 (br s, 1H), 4.39-4.31 (m, 2H), 3.06-2.97 (m, 2H), 1.43 (s, 9H), 1.40 (s, 9H), 1.31 (s, 9H), 1.30 (d, 3H, J = 7.7 Hz); ¹³C{¹H} NMR (100 MHz, CDCl₃, 24 °C): δ 171.7, 170.7, 155.4, 154.4, 131.4, 129.9, 124.3, 82.0, 80.1, 78.4, 55.7, 48.8, 37.8, 28.9, 28.3, 28.0, 18.6.

### *Example d9: synthesis of Boc-L-Ser(^{t}Bu)-L-Ala-OtBu

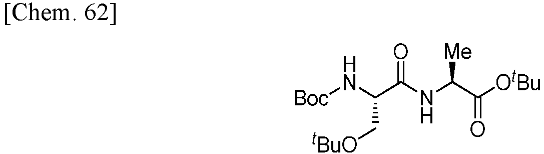

The reaction was carried out in accordance with the General Synthesis Procedure, using Boc-L-Ser(^{t}Bu) as the first amino acid AA¹ and L-Ala-OtBu as the second amino acid AA², whereby the title compound Boc-L-Ser(^{t}Bu)-L-Ala-OtBu was isolated as a white solid. The yield was 99%, and the diastereomer ratio was dr>99: 1.
R_{f} = 0.66 (40% AcOEt in hexane).

¹H NMR (400 MHz, CDCl₃, 24 °C): δ 7.28 (br s, 1H), 5.43 (br s, 1H), 4.41 (quin, 1H, J = 7.1 Hz), 4.15 (br s, 1H), 3.78-3.76 (m, 1H), 3.38-3.34 (m, 1H), 1.45 (s, 9H), 1.45 (s, 9H), 1.35 (d, 3H, J = 7.1 Hz), 1.19 (s, 9H); ¹³C{¹H} NMR (100 MHz, CDCl₃, 24 °C): δ 171.9, 170.1, 155.5, 81.8, 80.0, 74.0, 61.9, 54.3, 48.9, 28.4, 28.0, 27.4, 18.8.

### *Example d10: synthesis of Boc-L-Thr(^{t}Bu)-L-Ala-OtBu

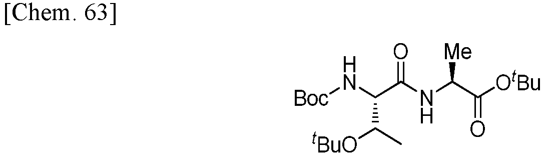

The reaction was carried out in accordance with the General Synthesis Procedure, using Boc-L-Thr(^{t}Bu) as the first amino acid AA¹ and L-Ala-OtBu as the second amino acid AA², whereby the title compound Boc-L-Thr(^{t}Bu)-L-Ala-OtBu was isolated as a white solid. The yield was 88%, and the diastereomer ratio was dr>99: 1.
R_{f}= 0.58 (40% AcOEt in hexane).

¹H NMR (400 MHz, CDCl₃, 24 °C): δ 7.52 (br d, 1H, J = 6.0 Hz), 5.57 (br d, 1H, J = 5.5 Hz), 4.42-4.32 (m, 1H), 4.12-4.06 (m, 2H), 1.44 (s, 9H), 1.43 (s, 9H), 1.34 (d, 3H, J = 7.1 Hz), 1.23 (s, 9H), 1.08 (d, 3H, J = 6.2 Hz); ¹³C{¹H} NMR (100 MHz, CDCl₃, 24 °C): δ 171.8, 169.7, 155.7, 81.8, 79.7, 75.1, 67.0, 58.6, 49.0, 28.46, 28.42, 28.0, 18.5, 17.7.

### *Example d11: synthesis of Boc-L-Cys(Bzl)-L-Ala-OtBu

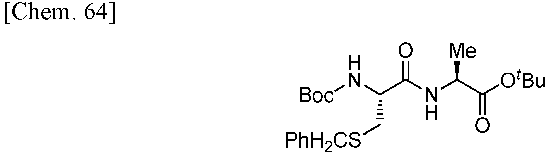

The reaction was carried out in accordance with the General Synthesis Procedure, using Boc-L-Cys(Bzl) as the first amino acid AA¹ and L-Ala-OtBu as the second amino acid AA², whereby the title compound Boc-L-Cys(Bzl)-L-Ala-OtBu was isolated as a white solid. The yield was 92%, and the diastereomer ratio was dr>99: 1.
R_{f}= 0.64 (40% AcOEt in hexane).

¹H NMR (400 MHz, CDCl₃, 24 °C): δ 7.35-7.21 (m, 5H), 6.91 (br d, 1H, J = 5.5 Hz), 5.36 (br s, 1H), 4.42 (quin, 1H, J = 7.1 Hz), 4.27 (br s, 1H), 3.74 (s, 2H), 2.87 (dd, 1H, J = 14.2, 5.8 Hz), 2.74 (dd, 1H, J = 14.0, 6.6 Hz), 1.45 (s, 18H), 1.37 (d, 3H, J = 7.1 Hz); ¹³C{¹H} NMR (100 MHz, CDCl₃, 24 °C): δ 171.6, 170.0, 155.3, 137.9, 129.1, 128.6, 127.2, 82.1, 80.3, 53.7, 49.0, 36.5, 33.8, 28.3, 28.0, 18.6.

### *Example d12: synthesis of Boc-L-Met-L-Ala-OtBu

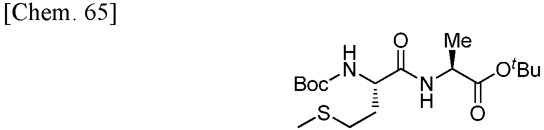

The reaction was carried out in accordance with the General Synthesis Procedure, using Boc-L-Met as the first amino acid AA¹ and L-Ala-OtBu as the second amino acid AA², whereby the title compound Boc-L-Met-L-Ala-OtBu was isolated as a white solid. The yield was 93%, and the diastereomer ratio was dr>99: 1.
R_{f}= 0.76 (40% AcOEt in hexane).

¹H NMR (400 MHz, CDCl₃, 24 °C): δ 6.63 (br d, 1H, J = 7.3 Hz), 5.20 (br d, 1H, J = 7.1 Hz), 4.42 (quin, 1H, J = 7.1 Hz), 4.27 (q, 1H, J = 6.7 Hz), 2.58 (t, 2H, J = 7.3 Hz), 2.11 (s, 3H), 2.09-2.02 (m, 1H), 1.97-1.88 (m, 1H), 1.45 (s, 9H), 1.43 (s, 9H), 1.36 (d, 3H, J = 7.1 Hz); ¹³C{¹H} NMR (100 MHz, CDCl₃, 24 °C): δ 171.8, 170.9, 155.5, 82.0, 80.0, 53.3, 48.8, 32.0, 30.1, 28.4, 28.0, 18.4, 15.2.

### *Example d13: synthesis of Boc-L-Asp(^{t}Bu)-L-Ala-OtBu

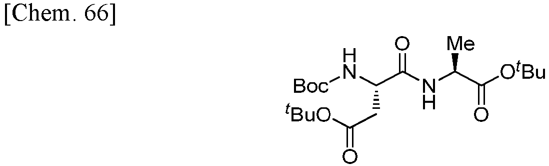

The reaction was carried out in accordance with the General Synthesis Procedure, using Boc-L-Asp(^{t}Bu) as the first amino acid AA¹ and L-Ala-OtBu as the second amino acid AA², whereby the title compound Boc-L-Asp(^{t}Bu)-L-Ala-OtBu was isolated as a white solid. The yield was 87%, and the diastereomer ratio was dr>99: 1.
R_{f} = 0.60 (40% AcOEt in hexane).

¹H NMR (400 MHz, CDCl₃, 24 °C): δ 7.02 (br d, 1H, J = 6.0 Hz), 5.67 (br d, 1H, J = 7.8 Hz), 4.43 (br s, 1H), 4.36 (quin, 1H, J = 7.1 Hz), 2.87-2.83 (m, 1H), 2.56 (dd, 1H, J = 17.0, 6.0 Hz), 1.427 (s, 9H), 1.423 (s, 9H), 1.41 (s, 9H), 1.32 (d, 3H, J = 7.1 Hz); ¹³C{¹H} NMR (100 MHz, CDCl₃, 24 °C): δ 171.7, 171.3, 170.3, 155.5, 81.9, 81.7, 80.3, 50.7, 48.9, 37.5, 28.3, 28.1, 28.0, 18.5.

### *Example d14: synthesis of Boc-L-Gluf^{t}Bu)-L-Ala-OtBu

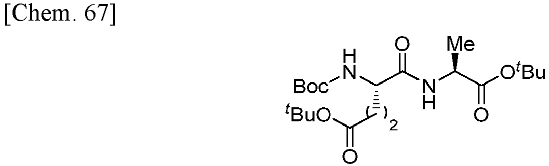

The reaction was carried out in accordance with the General Synthesis Procedure, using Boc-L-Glu(^{t}Bu) as the first amino acid AA¹ and L-Ala-OtBu as the second amino acid AA², whereby the title compound Boc-L-Glu(^{t}Bu)-L-Ala-OtBu was isolated as a white solid. The yield was 96%, and the diastereomer ratio was dr>99: 1.
R_{f} = 0.40 (40% AcOEt in hexane).

¹H NMR (400 MHz, CDCl₃, 24 °C): δ 6.81(br s, 1H), 5.35 (br d, 1H, J = 7.6 Hz), 4.37 (quin, 1H, J = 7.1 Hz), 4.12-4.06 (m, 1H), 2.39-2.25 (m, 2H), 2.07-1.99 (m, 1H), 1.90-1.80 (m, 1H), 1.41 (s, 9H), 1.40 (s, 9H), 1.38 (s, 9H), 1.30 (d, 3H, J = 7.3 Hz); ¹³C{¹H} NMR (100 MHz, CDCl₃, 24 °C): δ 172.7, 171.8, 171.1, 155.6, 81.9, 80.8, 79.9, 53.8, 48.8, 31.8, 28.3, 28.1, 28.07, 28.00, 18.4.

### *Example d15: synthesis of Boc-L-Asn(trt)-L-Ala-OtBu

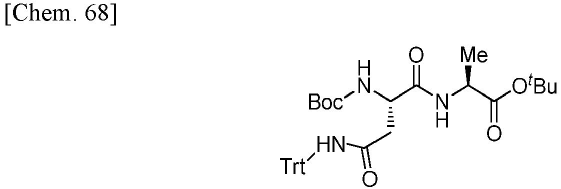

The reaction was carried out in accordance with the General Synthesis Procedure, using Boc-L-Asn(trt) as the first amino acid AA¹ and L-Ala-OtBu as the second amino acid AA², whereby the title compound Boc-L-Asn(trt)-L-Ala-OtBu was isolated as a white solid. The yield was 74%, and the diastereomer ratio was dr>99: 1.
R_{f}= 0.55 (40% AcOEt in hexane).

¹H NMR (400 MHz, CDCl₃, 24 °C): δ 7.28-7.16 (m, 15H), 6.91 (br s, 1H), 6.19 (br d, 1H, J = 7.3 Hz), 4.46-4.44 (m, 1H), 4.33 (quin, 1H, J = 7.1 Hz), 3.09 (d, 1H, J = 16.1 Hz), 2.69 (dd, 1H, J = 15.5, 4.8 Hz), 1.45 (s, 9H), 1.43 (s, 9H), 1.27 (d, 3H, J = 7.1 Hz); ¹³C{¹H} NMR (100 MHz, CDCl₃, 24 °C): δ 171.5, 170.8, 170.6, 155.8, 144.4, 128.7, 128.0, 127.1, 81.8, 80.2, 70.8, 51.3, 49.1, 38.0, 28.4, 28.0, 18.3.

### *Example d16: synthesis of Boc-L-Gln(trt)-L-Ala-OtBu

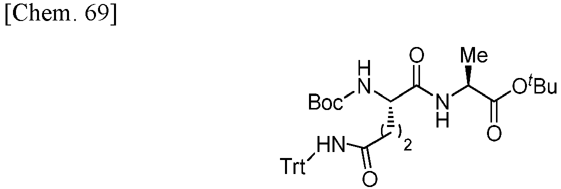

The reaction was carried out in accordance with the General Synthesis Procedure, using Boc-L-Gln(trt) as the first amino acid AA¹ and L-Ala-OtBu as the second amino acid AA², whereby the title compound Boc-L-Gln(trt)-L-Ala-OtBu was isolated as a white solid. The yield was 77%, and the diastereomer ratio was dr>99: 1.
R_{f} = 0.23 (40% AcOEt in hexane).

¹H NMR (400 MHz, CDCl₃, 24 °C): δ 7.31-7.22 (m, 15H), 6.56 (br d, 1H, J = 7.3 Hz), 5.42 (br d, 1H, J = 5.7 Hz), 4.32 (quin, 1H, J = 7.3 Hz), 4.02 (q, 1H, J = 7.1 Hz), 2.48 (t, 2H, J = 6.2 Hz), 2.06-1.91 (m, 2H), 1.43 (s, 18H), 1.28 (d, 3H, J = 7.1 Hz); ¹³C{¹H} NMR (100 MHz, CDCl₃, 24 °C): δ 171.9, 171.1, 155.8, 144.7, 128.8, 128.0, 127.0, 81.9, 79.9, 70.6, 53.4, 48.8, 33.6, 29.8, 28.4, 28.0, 18.0.

### *Example d17: synthesis of Boc-L-Lys(Z)-L-Ala-OtBu

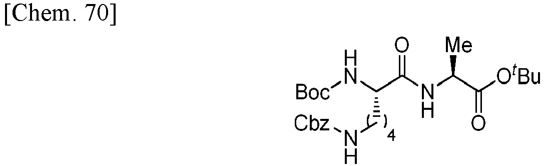

The reaction was carried out in accordance with the General Synthesis Procedure, using Boc-L-Lys(Z) as the first amino acid AA¹ and L-Ala-OtBu as the second amino acid AA², whereby the title compound Boc-L-Lys(Z)-L-Ala-OtBu was isolated as a white solid. The yield was 93%, and the diastereomer ratio was dr>99: 1.
Rᵣ=0.31 (40% AcOEt in hexane).

¹H NMR (400 MHz, CDCl₃, 24 °C): δ 7.34-7.28 (m, 5H), 6.57 (br s, 1H), 5.18 (br s, 1H), 5.08 (s, 2H), 5.01 (br s, 1H), 4.41 (quin, 1H, J = 7.1 Hz), 4.13-4.07 (m, 1H), 3.17 (t, 2H, J = 6.0 Hz), 1.84-1.79 (m, 1H), 1.66-1.56 (m, 1H), 1.54-1.48 (m, 2H), 1.43 (s, 9H), 1.42 (s, 9H), 1.38-1.36 (m, 2H), 1.34 (d, 3H, J = 7.1 Hz); ¹³C{¹H} NMR (100 MHz, CDCl₃, 24 °C): δ 172.0, 171.5, 156.7, 136.7, 128.6, 128.2, 128.1, 82.1, 80.0, 66.7, 54.2, 48.7, 40.5, 32.2, 29.4, 28.4, 28.0, 22.4, 18.5.

### *Example d18: synthesis of Boc-L-Arg(Z)₂-L-Ala-OtBu

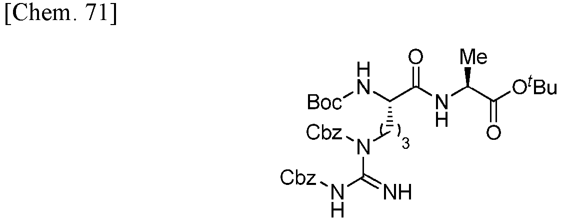

The reaction was carried out in accordance with the General Synthesis Procedure, using Boc-L-Arg(Z)₂ as the first amino acid AA¹ and L-Ala-OtBu as the second amino acid AA², whereby the title compound Boc-L-Arg(Z)₂-L-Ala-OtBu was isolated as a white solid. The yield was 69%, and the diastereomer ratio was dr>99: 1.
R_{f} = 0.42 (40% AcOEt in hexane).

¹H NMR (400 MHz, CDCl₃, 24 °C): δ 9.43 (br s, 1H), 9.27 (br s, 1H), 7.41-7.26 (m, 10H), 6.76 (br d, 1H, J = 7.1 Hz), 5.48 (br d, 1H, J = 8.0 Hz), 5.24 (s, 2H), 5.02-5.09 (m, 2H), 4.33 (quin, 1H, J = 7.1 Hz), 4.24-4.22 (m, 1H), 4.09-4.02 (m, 1H), 3.94-3.88 (m, 1H), 1.81-1.57 (m, 4H), 1.42 (s, 18H), 1.19 (d, 3H, J = 7.1 Hz); ¹³C{¹H} NMR (100 MHz, CDCl₃, 24 °C): δ 171.7, 171.4, 163.8, 160.8, 155.9, 155.6, 136.9, 134.7, 128.98, 128.94, 128.5, 128.4, 127.94, 127.90, 81.7, 79.8, 69.0, 67.0, 53.9, 48.7, 44.2, 28.4, 28.0, 24.8, 18.1.

### *Example d19: synthesis of Boc-L-His(Bzl)-L-Ala-OtBu

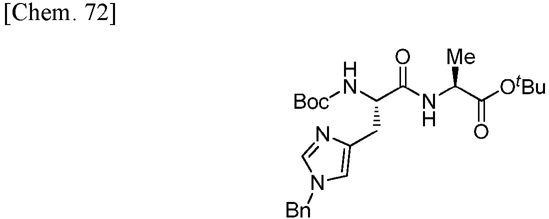

The reaction was carried out in accordance with the General Synthesis Procedure, using Boc-L-His(Bzl) as the first amino acid AA¹ and L-Ala-OtBu as the second amino acid AA², whereby the title compound Boc-L-His(Bzl)-L-Ala-OtBu was isolated as a white solid. The yield was 91%, and the diastereomer ratio was dr>99: 1.
R_{f} = 0.42 (40% AcOEt in hexane).

¹H NMR (400 MHz, CDCl₃, 24 °C): δ 7.42 (br s, 1H), 7.36-7.28 (m, 3H), 7.17-7.12 (m, 3H), 6.71 (s, 1H), 6.18 (br s, 1H), 5.06-4.97 (m, 2H), 4.37-4.32 (m, 2H), 3.08 (d, 1H, J = 12.1 Hz), 2.90 (dd, 1H, J = 14.6, 6.0 Hz), 1.43 (s, 9H), 1.42 (s, 9H), 1.27 (d, 3H, J = 7.1 Hz); ¹³C{¹H} NMR (100 MHz, CDCl₃, 24 °C): δ 171.9, 171.1, 155.8, 138.6, 136.7, 136.0, 129.1, 128.4, 127.5, 117.3, 81.7, 79.8, 54.7, 51.0, 48.7, 30.3, 28.4, 28.0, 18.5.

### *Example d20: synthesis of Boc-L-Trp-L-Ala-OtBu

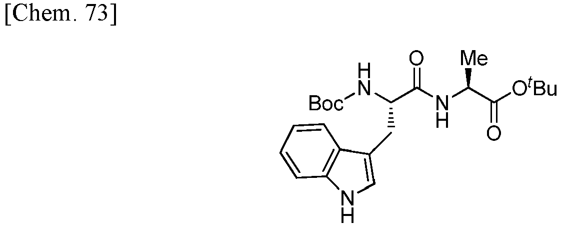

The reaction was carried out in accordance with the General Synthesis Procedure, using Boc-L-Trp as the first amino acid AA¹ and L-Ala-OtBu as the second amino acid AA², whereby the title compound Boc-L-Trp-L-Ala-OtBu was isolated as a white solid. The yield was 99%, and the diastereomer ratio was dr>99: 1.
R_{f} = 0.46 (40% AcOEt in hexane).

¹H NMR (400 MHz, CDCl₃, 24 °C): δ 8.30 (br d, 1H, J = 17.1 Hz), 7.34 (d, 1H, J = 7.8 Hz), 7.19-7.15 (m, 1H), 7.12-7.08 (m, 1H), 7.05 (br s, 1H), 6.45 (br s, 1H), 5.15 (br s, 1H), 4.44 (br s, 1H), 4.34-4.30 (m, 1H), 3.30 (br s, 1H), 3.18 (dd, 1H, J = 14.4, 6.8 Hz), 1.40 (s, 18H), 1.24 (d, 3H, J = 7.1 Hz); ¹³C{¹H} NMR (100 MHz, CDCl₃, 24 °C): δ 171.7, 171.1, 155.5, 136.3, 127.6, 123.3, 122.3, 119.7, 119.0, 111.2, 110.6, 82.0, 80.1, 55.2, 48.8, 28.3, 28.0, 18.6.

### *Example d21: synthesis of Boc-L-Pro-L-Ala-OtBu

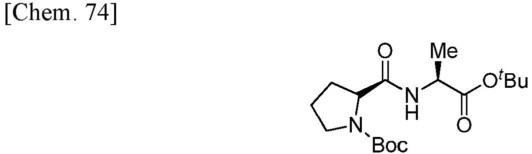

The reaction was carried out in accordance with the General Synthesis Procedure, using Boc-L-Pro as the first amino acid AA¹ and L-Ala-OtBu as the second amino acid AA², whereby the title compound Boc-L-Pro-L-Ala-OtBu was isolated as a white solid. The yield was 99%, and the diastereomer ratio was dr>99: 1.
R_{f}= 0.33 (40% AcOEt in hexane).

### *Example d22: synthesis of Boc-L-Ala-L-Gly-OtBu

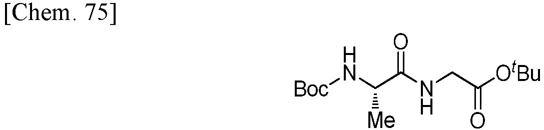

The reaction was carried out in accordance with the General Synthesis Procedure, using Boc-L-Ala as the first amino acid AA¹ and L-Gly-OtBu as the second amino acid AA², whereby the title compound Boc-L-Ala-L-Gly-OtBu was isolated as a white solid. The yield was 86%, and the diastereomer ratio was er>99: 1.
R_{f} = 0.21 (40% AcOEt in hexane).

### *Example d23: synthesis of Boc-L-Ala-L-Val-OtBu

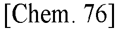

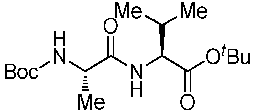

The reaction was carried out in accordance with the General Synthesis Procedure, using Boc-L-Ala as the first amino acid AA¹ and L-Val-OtBu as the second amino acid AA², whereby the title compound Boc-L-Ala-L-Val-OtBu was isolated as a white solid. The yield was 86%, and the diastereomer ratio was er>99:1.
R_{f} = 0.65 (40% AcOEt in hexane).

¹H NMR (400 MHz, CDCl₃, 24 °C): δ 6.55 (br d, 1H, J = 8.0 Hz), 5.00 (br s, 1H), 4.42-3.39 (m, 1H), 4.18-4.16 (m, 1H), 2.17-2.13 (m, 1H), 1.47 (s, 9H), 1.43 (s, 9 H), 1.35 (d, 3H, J = 7.2 Hz), 0.91 (d, 3 H, J = 6.8 Hz), 0.89 (d, 3 H, J = 6.8 Hz); ¹³C{¹H} NMR (100 MHz, CDCl₃, 24 °C): δ 172.5, 170.9, 155.5, 82.0, 80.1, 57.4, 50.2, 31.5, 28.4, 28.1, 18.9, 18.0, 17.5.

### *Example d24: synthesis of Boc-L-Ala-L-Ile-OtBu

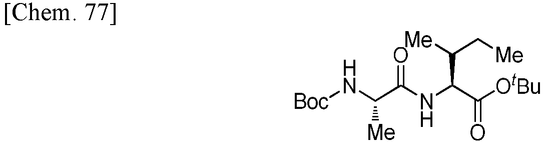

The reaction was carried out in accordance with the General Synthesis Procedure, using Boc-L-Ala as the first amino acid AA¹ and L-Ile-OtBu as the second amino acid AA², whereby the title compound Boc-L-Ala-L-Ile-OtBu was isolated as a white solid. The yield was 98%, and the diastereomer ratio was dr>99: 1.
R_{f} = 0.62 (40% AcOEt in hexane).

¹H NMR (400 MHz, CDCl₃, 24 °C): δ 6.58 (br d, 1H, J = 7.7 Hz), 5.0 (br d, 1H, J = 7.3 Hz), 4.44 (dd, 1H, J = 8.6, 4.7 Hz), 4.16-4.08 (m, 1H), 1.91-1.81 (m, 1H), 1.45 (s, 9H), 1.43 (s, 9 H), 1.34 (d, 3H, J = 7.1 Hz), 1.29-1.36 (m, 2 H), 0.93-0.97 (m, 6 H); ¹³C{¹H} NMR (100 MHz, CDCl₃, 24 °C): δ 172.2, 170.8, 155.5, 81.9, 80.0, 56.8, 50.1, 38.2, 28.4, 28.1, 25.2, 18.1, 15.4, 11.8.

### *Example d25: synthesis of Boc-L-Ala-L-Leu-OtBu

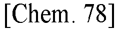

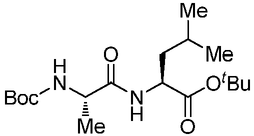

The reaction was carried out in accordance with the General Synthesis Procedure, using Boc-L-Ala as the first amino acid AA¹ and L-Leu-OtBu as the second amino acid AA², whereby the title compound Boc-L-Ala-L-Leu-OtBu was isolated as a white solid. The yield was 99%, and the diastereomer ratio was dr>99: 1.
R_{f} = 0.57 (40% AcOEt in hexane).

¹H NMR (400 MHz, CDCl₃, 24 °C): δ 6.55 (br s, 1H), 5.13 (br d, 1H, J = 6.2 Hz), 4.46-4.41 (m, 1H), 4.16-4.5 (m, 1H), 1.60-1.44 (m, 3H), 1.42 (s, 9H), 1.40 (s, 9 H), 1.32 (d, 3H, J = 7.1 Hz), 0.89 (d, 6 H, J = 6.1 Hz); ¹³C{¹H} NMR (100 MHz, CDCl₃, 24 °C): δ 172.3, 172.0, 155.5, 81.8, 80.0, 51.4, 49.9, 41.8, 28.3, 28.0, 24.9, 22.9, 22.0, 18.3.

### *Example d26: synthesis of Boc-L-Ala-L-Phg-OtBu

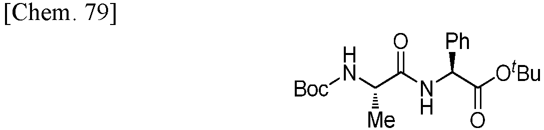

The reaction was carried out in accordance with the General Synthesis Procedure, using Boc-L-Ala as the first amino acid AA¹ and L-Phg-OtBu as the second amino acid AA², whereby the title compound Boc-L-Ala-L-Phg-OtBu was isolated as a white solid. The yield was 95%, and the diastereomer ratio was dr = 93.1:6.9.
R_{f} = 0.63 (40% AcOEt in hexane).

¹H NMR (400 MHz, CDCl₃, 24 °C): δ 7.32-7.27 (m, 5 H), 7.18 (br s, 1H), 5.41 (d, 1H, J = 7.3 Hz), 5.00 (br s, 1H), 4.22 (br s, 1H), 1.41 (s, 9H), 1.38 (s, 9H), 1.34 (d, 3 H, J = 7.1 Hz); ¹³C{¹H} NMR (100 MHz, CDCl₃, 24 °C): δ 171.8, 169.7, 155.5, 137.2, 128.8, 128.2, 127.0, 82.7, 80.2, 57.0, 50.0, 28.3, 27.9, 18.1.

### *Example d27: synthesis of Boc-L-Ala-L-Phe-OtBu

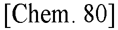

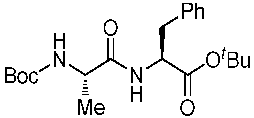

The reaction was carried out in accordance with the General Synthesis Procedure, using Boc-L-Ala as the first amino acid AA¹ and L-Phe-OtBu as the second amino acid AA², whereby the title compound Boc-L-Ala-L-Phe-OtBu was isolated as a white solid. The yield was 99%, and the diastereomer ratio was dr>99: 1.
R_{f} = 0.48 (40% AcOEt in hexane).

### *Example d28: synthesis of Boc-L-Ala-L-Tyr(^{t}Bu)-OtBu

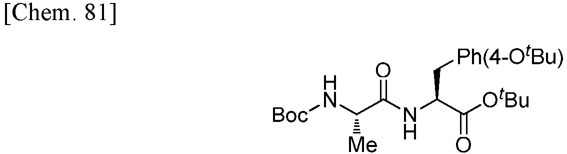

The reaction was carried out in accordance with the General Synthesis Procedure, using Boc-L-Ala as the first amino acid AA¹ and L-Tyr(^{t}Bu)-OtBu as the second amino acid AA², whereby the title compound Boc-L-Ala-L-Tyr(^{t}Bu)-OtBu was isolated as a white solid. The yield was 94%, and the diastereomer ratio was dr>99: 1.
R_{f} = 0.48 (40% AcOEt in hexane).

### *Example d29: synthesis of Boc-L-Ala-L-Ser(^{t}Bu)-OtBu

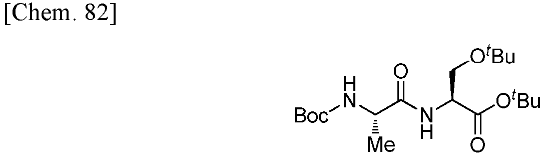

The reaction was carried out in accordance with the General Synthesis Procedure, using Boc-L-Ala as the first amino acid AA¹ and L-Ser(^{t}Bu)-OtBu as the second amino acid AA², whereby the title compound Boc-L-Ala-L-Ser(^{t}Bu)-OtBu was isolated as a white solid. The yield was 97%, and the diastereomer ratio was dr>99: 1.
Rᵣ=0.57 (40% AcOEt in hexane).

¹H NMR (400 MHz, CDCl₃, 24 °C): δ 6.60 (br d, 1 H, J = 6.4 Hz), 5.11 (br s, 1H), 4.54-4.52 (m, 1H), 4.19 (br s, 1H), 3.76 (dd, 1H, J = 8.7, 3.0 Hz), 3.50 (dd, 1H, J = 8.7, 2.7 Hz), 1.44 (s, 9H), 1.42 (s, 9 H), 1.37 (d, 3 H, J = 7.0 Hz), 1.12 (s, 9 H); ¹³C{¹H} NMR (100 MHz, CDCl₃, 24 °C): δ 172.3, 169.3, 155.2, 81.9, 79.8, 73.1, 62.1, 53.2, 50.1, 28.4, 28.1, 27.4, 19.1.

### *Example d30: synthesis of Boc-L-Ala-L-Thr(^{t}Bu)-OtBu

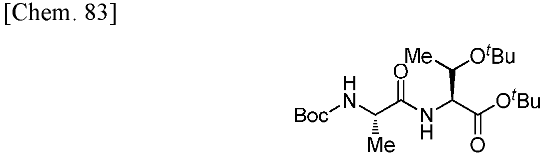

The reaction was carried out in accordance with the General Synthesis Procedure, using Boc-L-Ala as the first amino acid AA¹ and L-Thr(^{t}Bu)-OtBu as the second amino acid AA², whereby the title compound Boc-L-Ala-L-Thr(^{t}Bu)-OtBu was isolated as a white solid. The yield was 95%, and the diastereomer ratio was dr>99: 1.
R_{f} = 0.63 (40% AcOEt in hexane).

### *Example d31: synthesis of Boc-L-Ala-L-Cys(trt)-OtBu

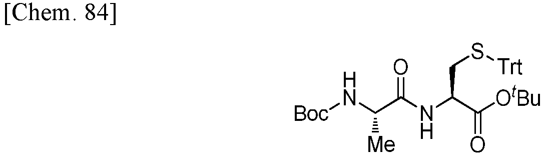

The reaction was carried out in accordance with the General Synthesis Procedure, using Boc-L-Ala as the first amino acid AA¹ and L-Cys(trt)-OtBu as the second amino acid AA², whereby the title compound Boc-L-Ala-L-Cys(trt)-OtBu was isolated as a white solid. The yield was 99%, and the diastereomer ratio was dr>99: 1.
R_{f} = 0.77 (40% AcOEt in hexane).

¹H NMR (400 MHz, CDCl₃, 24 °C): δ 7.39-7.36 (m, 6H, J = 6.4 Hz), 7.28-7.24 (m, 6H), 7.22-7.18 (m, 3H), 6.45 (br d, 1H, J = 6.1 Hz), 5.02 (br s, 1H), 4.45-4.41 (m, 1H), 4.13-4.05 (m, 1H), 2.60 (dd, 1H, J = 12.1, 5.5 Hz), 2.53 (dd, 1H, J = 12.0, 4.5 Hz), 1.43 (s, 9H), 1.41 (s, 9 H), 1.33 (d, 3 H, J = 7.1 Hz); ¹³C{¹H} NMR (100 MHz, CDCl₃, 24 °C): δ 172.1, 169.1, 155.3, 144.4, 129.6, 128.1, 126.9, 82.7, 80.1, 66.6, 51.7, 50.1, 34.0, 28.4, 28.0, 18.8.

### *Example d32: synthesis of Boc-L-Ala-L-Met-OtBu

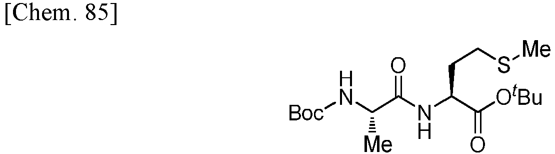

The reaction was carried out in accordance with the General Synthesis Procedure, using Boc-L-Ala as the first amino acid AA¹ and L-Met-OtBu as the second amino acid AA², whereby the title compound Boc-L-Ala-L-Met-OtBu was isolated as a white solid. The yield was 99%, and the diastereomer ratio was dr>99: 1.
R_{f}= 0.54 (40% AcOEt in hexane).

¹H NMR (400 MHz, CDCl₃, 24 °C): δ 6.70 (br d, 1H, J = 7.8 Hz), 5.00 (br s, 1H), 4.57-4.52 (m, 1H), 4.15-4.08 (m, 1H), 2.54-2.41 (m, 2H), 2.16-2.09 (m, 1H), 2.08 (s, 3H), 2.03-1.89 (m, 1H), 1.45 (s, 9H), 1.43 (s, 9H), 1.35 (d, 3H, J = 7.1 Hz); ¹³C{¹H} NMR (100 MHz, CDCl₃, 24 °C): δ 172.4, 170.8, 155.5, 82.5, 80.2, 52.2, 50.2, 32.1, 29.9, 28.4, 28.1, 18.3, 155.5.

### *Example d33: synthesis of Boc-L-Ala-L-Asp(^{t}Bu)-OtBu

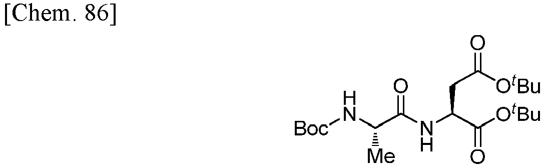

The reaction was carried out in accordance with the General Synthesis Procedure, using Boc-L-Ala as the first amino acid AA¹ and L-Asp(^{t}Bu)-OtBu as the second amino acid AA², whereby the title compound Boc-L-Ala-L-Asp(^{t}Bu)-OtBu was isolated as a white solid. The yield was 96%, and the diastereomer ratio was dr>99: 1.
R_{f} = 0.75 (40% AcOEt in hexane).

¹H NMR (400 MHz, CDCl₃, 24 °C): δ 6.83 (br d, 1H, J = 8.0 Hz), 5.08 (br s, 1H), 4.65 (q, 1H, J = 4.1 Hz), 4.17 (br s, 1H), 2.88 (dd, 1H, J = 17.1, 4.3 Hz), 2.69 (dd, 1H, J = 17.1, 4.3 Hz), 1.44 (s, 9H), 1.434 (s, 9H), 1.431 (s, 9H), 1.37 (d, 3H, J = 7.1 Hz); ¹³C{¹H} NMR (100 MHz, CDCl₃, 24 °C): δ 172.4, 170.4, 169.6, 155.2, 82.4, 81.7, 80.0, 50.2, 49.1, 37.4, 28.4, 28.1, 28.0, 19.1.

### *Example d34: synthesis of Boc-L-Ala-L-Glu(^{t}Bu)-OtBu

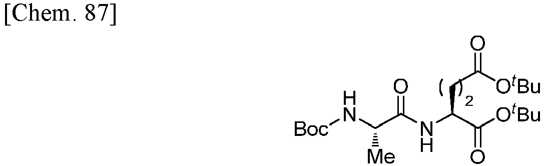

The reaction was carried out in accordance with the General Synthesis Procedure, using Boc-L-Ala as the first amino acid AA¹ and L-Glu(^{t}Bu)-OtBu as the second amino acid AA², whereby the title compound Boc-L-Ala-L-Glu(^{t}Bu)-OtBu was isolated as a white solid. The yield was 99%, and the diastereomer ratio was dr>99: 1.
R_{f} = 0.67 (40% AcOEt in hexane).

¹H NMR (400 MHz, CDCl₃, 24 °C): δ 6.84 (br s, 1H), 5.24 (br d, 1H, J = 6.0 Hz), 4.38 (td, 1H, J = 8.2, 4.8 Hz), 4.12 (br s, 1H), 2.37-2.13 (m, 2H), 2.08-1.96 (m, 1H), 1.86-1.76 (m, 1H), 1.38 (s, 9H), 1.35 (s, 9H), 1.35 (s, 9H), 1.28 (d, 3H, J = 7.1 Hz); ¹³C{¹H} NMR (100 MHz, CDCl₃, 24 °C): δ 172.5, 172.1, 170.8, 155.4, 82.1, 80.6, 79.8, 52.1, 50.0, 31.3, 28.3, 28.0, 27.9, 27.5, 18.4.

### *Example d35: synthesis of Boc-L-Ala-L-Asn-OtBu

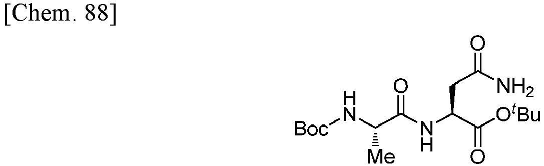

The reaction was carried out in accordance with the General Synthesis Procedure, using Boc-L-Ala as the first amino acid AA¹ and L-Asn-OtBu as the second amino acid AA², whereby the title compound Boc-L-Ala-L-Asn-OtBu was isolated as a white solid. The yield was 99%, and the diastereomer ratio was dr>99: 1.
R_{f}= 0.25 (5% MeOH in CHCl₃).

### *Example d36: synthesis of Boc-L-Ala-L-Lys(Z)-OtBu

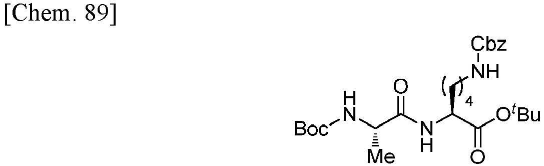

The reaction was carried out in accordance with the General Synthesis Procedure, using Boc-L-Ala as the first amino acid AA¹ and L-Lys(Z)-OtBu as the second amino acid AA², whereby the title compound Boc-L-Ala-L-Lys(Z)-OtBu was isolated as a white solid. The yield was 96%, and the diastereomer ratio was dr>99: 1.
R_{f} = 0.72 (70% AcOEt in hexane).

¹H NMR (400 MHz, CDCl₃, 24 °C): δ 7.34-7.28 (m, 5H), 6.69 (br s, 1H), 5.11 (br s, 1H), 5.08 (s, 2H), 4.46-4.41 (m, 1H), 4.16-4.10 (m, 1H), 3.22-3.15 (m, 2H), 1.86-1.78 (m, 1H), 1.65-1.55 (m, 1H), 1.51-1.48 (m, 2H), 1.44 (s, 9H), 1.43 (s, 9H), 1.38-1.24 (m, 5H); ¹³C{¹H} NMR (100 MHz, CDCl₃, 24 °C): δ 172.8, 171.2, 156.6, 155.6, 136.6, 128.5, 128.2, 128.1, 127.9, 82.0, 80.0, 66.6, 52.4, 50.0, 40.6, 32.0, 29.1, 28.4, 28.0, 22.1, 18.3.

### [Example Group E: Synthesis of Boc-L-Ala-L-Asn-OtBu via Amidation Reaction using Silane Compound (A)]

### *General Synthesis Procedure :

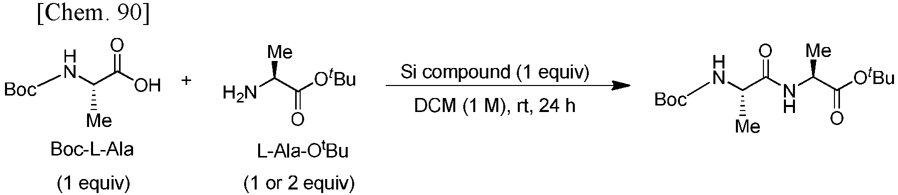

A 5.0-mL screw-cap vial heated and dried in a glove box was charged, under an argon atmosphere, with a stirrer (samarium-cobalt), Boc-L-Ala(0.5mmol, 1 equivalent), and L-Ala-OtBu. This mixture was combined with a silane compound (0.5mmol, 1 equivalent) (generation of hydrogen gas was observed.), and then mixed and dissolved in dry DCM (0.5 mL, 1M), and the vial was sealed. The vial was removed from the glove box and stirred vigorously under an argon atmosphere at room temperature for 24 hours. The reaction was monitored with TLC analysis. Once the reaction completed, the reaction mixture was diluted with CHCl₃ (3.0mL), and transferred by a pipette to a silica gel column. The vial and pipette used were washed with CHCl₃ (5.0mL). The reaction mixture was purified with flash column chromatography using 10 to 100% AcOEt in hexane or 0 to 10% MeOH in CHCl₃ as an eluate, whereby the title compound Boc-L-Ala-L-Ala-OtBu was obtained.

### *Example e1: synthesis using Si[OCH₂CF₃]₄

The reaction was carried out in accordance with the General Synthesis Procedure except that L-Ala-OtBu was used in an amount of 1 equivalent (0.5 mmol), and tris(1,1,1,3,3,3-hexafluoroisopropoxy)silane (HSi[OCH(CF₃)_{2]3}) was used as a silane compound, whereby the title compound Boc-L-Ala-L-Ala-OtBu was obtained. The yield was 78%, and the diastereomer ratio was dr>99: 1.

### *Example e2: synthesis using HSi[OCH₂CF₂CHF₂]₃

The reaction was carried out in accordance with the General Synthesis Procedure except that L-Ala-OtBu was used in an amount of 1 equivalent (0.5 mmol), and tris(3,3,2,2-tetrafluoro n-propoxy)silane (HSi[OCH₂CF₂CHF₂]₃) was used as a silane compound, whereby the title compound Boc-L-Ala-L-Ala-OtBu was obtained. The yield was 53%, and the diastereomer ratio was dr>99: 1.

### *Example e3: synthesis using HSi[OCH₂CCl₃]₃

The reaction was carried out in accordance with the General Synthesis Procedure except that L-Ala-OtBu was used in an amount of 1 equivalent (0.5 mmol), and tris(2,2,2-trichloroethoxy)silane (HSi[OCH₂CCl₃]₃) was used as a silane compound, whereby the title compound Boc-L-Ala-L-Ala-OtBu was obtained. The yield was 53%, and the diastereomer ratio was dr>99: 1.

### *Example e4: synthesis using HSi[OCH₂CF_{3]3}

The reaction was carried out in accordance with the General Synthesis Procedure except that L-Ala-OtBu was used in an amount of 2 equivalents (0.5 mmol), and tris(2,2,2-trifluoroethoxy)silane (HSi[OCH₂CF_{3]3}) was used as a silane compound, whereby the title compound Boc-L-Ala-L-Ala-OtBu was obtained. The yield was 53%, and the diastereomer ratio was dr>99: 1.

### *Example e5: synthesis using Si[OCH₂CF₃]₄

The reaction was carried out in accordance with the General Synthesis Procedure except that L-Ala-OtBu was used in an amount of 2 equivalents (0.5 mmol), and tetrakis(2,2,2-trifluoroethoxy)silane (Si[OCH₂CF_{3]4}) was used as a silane compound, whereby the title compound Boc-L-Ala-L-Ala-OtBu was obtained. The yield was 66%, and the diastereomer ratio was dr>99: 1.

### *Example e6: synthesis using Si[OCH(CF₃)₂]₄

The reaction was carried out in accordance with the General Synthesis Procedure except that L-Ala-OtBu was used in an amount of 2 equivalents (0.5 mmol), and tetrakis(1,1,1,3,3,3-hexafluoroisopropoxy)silane (Si[OCH(CF₃)₂]₄) was used as a silane compound, whereby the title compound Boc-L-Ala-L-Ala-OtBu was obtained. The yield was 85%, and the diastereomer ratio was dr>99: 1.

### [Example Group F: Amidation Reaction using Silane Compound (A) in the presence of Tris(haloalkoxy)aminosilane Catalyst 1]

### *Example f1: synthesis of Boc-L-Ala-L-Ala-OtBu via amidation reaction 1

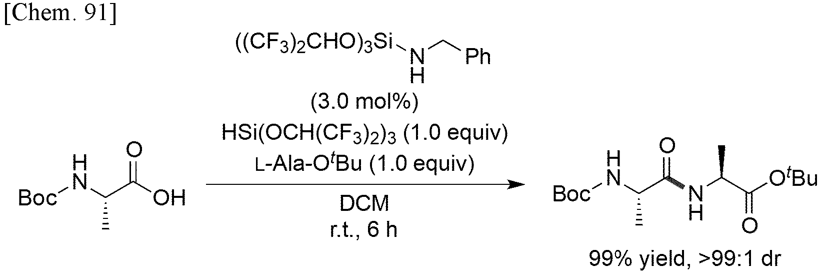

In a glove box, a heat-dried 5.0-mL screw-cap vial was charged with a magnetic stirring rod (Sm-Co), benzylamine (53.6mg, 0.50mmol), DCM(0.50mL), and HSi[OCH(CF₃)₂]₃(265.1mg, 0.50mmol), and the vial was sealed and removed from the glove box. The resulting mixture was stirred vigorously under an argon atmosphere at room temperature for 4 hours, whereby a catalyst solution containing BnNHSi[OCH(CF₃)₂]₃ was prepared. In a glove box, a flame-dried 5.0 mL screw cap vial was charged with a mixture of the catalyst solution containing BnNHSi[OCH(CF₃)₂]₃ (0.015mL, 0.015mmol), Boc-L-Ala-OH(94.6mg, 0.50mmol), DCM(0.50mL), HSi[OCH(CF₃)₂]₃(265.1mg, 0.50mmol), and L-Ala-OtBu(72.6mg, 0.50mmol), and the vial was sealed and removed from the glove box. The resulting mixture was stirred vigorously under an argon atmosphere at room temperature for 6 hours, and the reaction mixture was diluted with CHCl₃ (3.0mL), and then transferred into a SiO₂ column with a pipette. The vial and pipette used were washed with CHCl₃ (12mL). The reaction mixture was purified with flash column chromatography (20 to 100% AcOEt in hexane), whereby Boc-L-Ala-L-Ala-OtBu was isolated as a colorless liquid. The yield was 99% (156.9mg), and the diastereomer ratio was dr>99: 1.

### *Example f2: synthesis of Boc-L-Ala-L-Ala-OtBu via amidation reaction 2

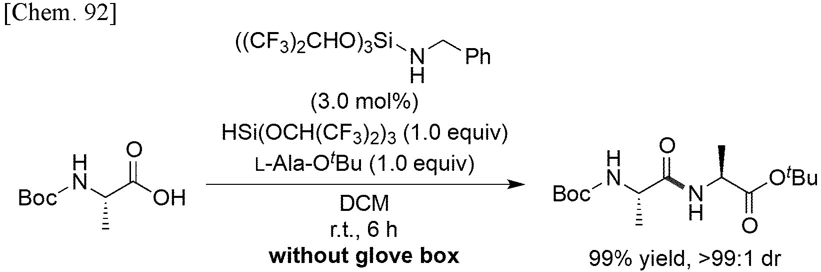

In a glove box, a heat-dried 5.0-mL screw-cap vial was charged with a magnetic stirring rod (Sm-Co), benzylamine (53.6mg, 0.50mmol), DCM (0.50mL), and HSi[OCH(CF₃)₂]₃(265.1mg, 0.50mmol), and the vial was sealed and removed from the glove box. The resulting mixture was stirred vigorously under an argon atmosphere at room temperature for 4 hours, whereby a catalyst solution containing BnNHSi[OCH(CF₃)₂]₃ was prepared. A flame-dried 5.0mL screw-cap vial was charged with ETFE septa, a magnetic stirring bar (Sm-Co), and Boc-L-Ala-OH (94.6mg, 0.50mmol). After replacing the air in the vial with nitrogen, L-Ala-OtBu (72.6mg, 0.50mmol), DCM (0.50mL), HSi[OCH(CF₃)₂]₃ (265.1mg, 0.50mmol), and the catalyst solution containing BnNHSi[OCH(CF₃)₂]₃ (0.015mL, 0.015mmol) were put into the vial, which was then sealed with a screw cap. The resulting mixture was stirred vigorously under a nitrogen atmosphere at room temperature for 6 hours, and the reaction mixture was diluted with CHCl₃ (3.0mL), and transferred into SiO₂ column with a pipette. The vial and pipette used were washed with CHCl₃ (12mL). The reaction mixture was purified with flash column chromatography (20 to 100% AcOEt in hexane), whereby the title compound Boc-L-Ala-L-Ala-OtBu was isolated as a colorless liquid. The yield was 98% (155.0mg), and the diastereomer ratio was dr>99: 1.

### *Example f3: synthesis of Boc-L-Met-L-Ala-OtBu via amidation reaction

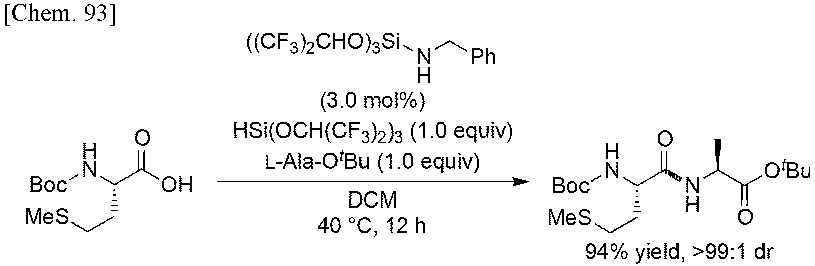

The reaction was carried out in the same manner as in Example f1 except that Boc-L-Met-OH (124.7mg, 0.50mmol) was used instead of Boc-L-Ala-OH, and the mixing of the reaction solution was carried out at 40°C for 12h, whereby Boc-L-Met-L-Ala-OtBu was isolated as a colorless liquid. The yield was 94% (176.5mg), and the diastereomer ratio was dr>99: 1.

### *Example f4: synthesis of Boc-L-Val-L-Ala-OtBu via amidation reaction

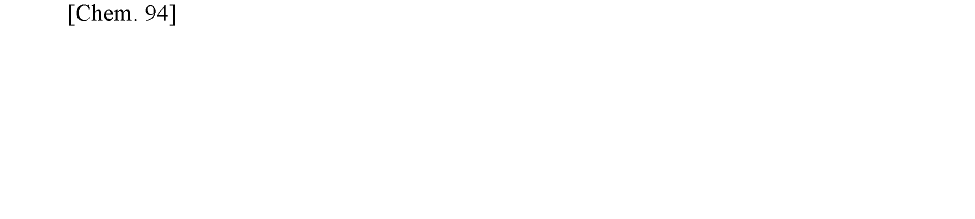

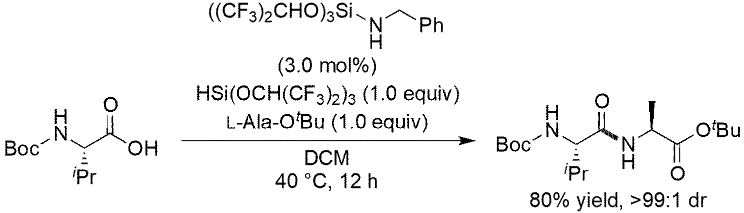

The reaction was carried out in the same manner as in Example f1 except that Boc-L-Val-OH (108.6mg, 0.50mmol) was used instead of Boc-L-Ala-OH, the used amount of DCM was changed to 1.0mL, and the mixing of the reaction solution was carried out at 40°C for 12h, whereby Boc-L-Val-L-Ala-OtBu was obtained as a white solid. The yield was 80% (137.1mg), and the diastereomer ratio was dr>99: 1.

### *Example f5: synthesis of Fmoc-L-Ser(tBu)-L-Lys(Boc)-OBn via amidation reaction

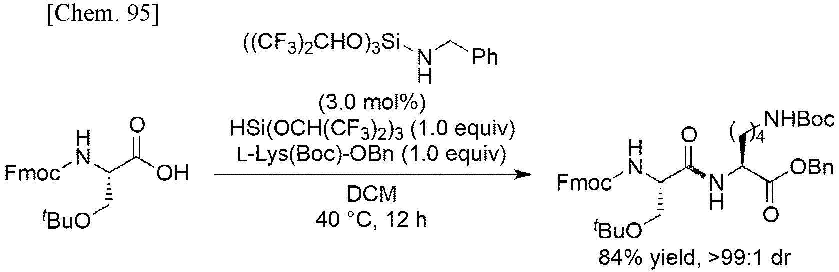

The reaction was carried out in the same manner as in Example f1 except that Fmoc-L-Ser(tBu)-OH (191.7mg, 0.50mmol) was used instead of Boc-L-Ala-OH, the used amount of DCM was changed to 1.0mL, L-Lys(Boc)-OBn (168.2mg, 0.50mmol) was used instead of L-Ala-OtBu, and the mixing of the reaction solution was carried out at 40°C for 12h, whereby Fmoc-L-Ser(tBu)-L-Lys(Boc)-OBn was obtained as a white solid. The yield was 84% (295.8mg), and the diastereomer ratio was dr>99: 1.

### *Example f6: synthesis of Boc-L-Ala-L-Val-OtBu via amidation reaction

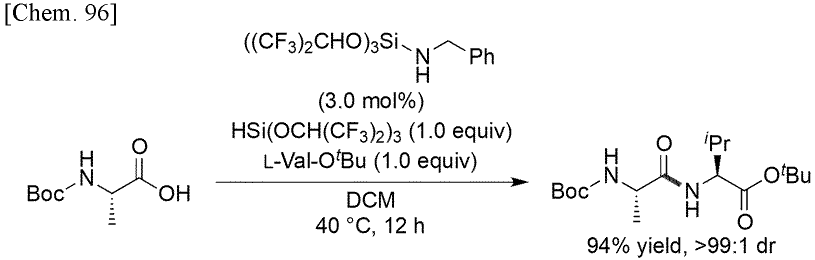

The reaction was carried out in the same manner as in Example f1 except that L-Val-OtBu (86.6mg, 0.50mmol) was used instead of L-Ala-OtBu, and the mixing of the reaction solution was carried out at 40°C for 12h, whereby Boc-L-Ala-L-Val-OtBu was obtained as a white solid. The yield was 94% (162.2mg), and the diastereomer ratio was dr>99: 1.

### *Example f7: synthesis of Fmoc-L-Arg(Boc)₂-L-Thr(tBu)-OtBu via amidation reaction

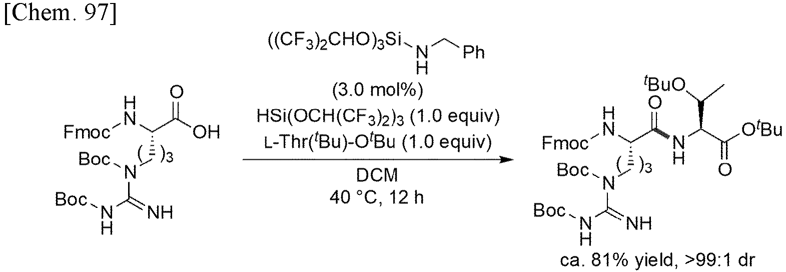

The reaction was carried out in the same manner as in Example f1 except that Fmoc-L-Arg(Boc)₂-OH (298.3mg (HPLC purity 92.4%), about 0.47mmol. The main byproduct is Fmoc-L-Arg(Boc)-OH. Purchased from Watanabe Chemical Industry Co.) was used instead of Boc-L-Ala-OH, the used amount of DCM was changed to 1.0mL, L-Thr(tBu)-OtBu(115.7mg, 0.50mmol) was used instead of L-Ala-OtBu, and the mixing of the reaction solution was carried out at 40°C for 12h, whereby Fmoc-L-Arg(Boc)₂-L-Thr(tBu)-OtBu was obtained as a white solid. The yield was about 81% (307.7mg), and the diastereomer ratio was dr>99: 1.

### [Example Group G: Amidation Reactions using Silane Compound (A) in the presence of Tris(haloalkoxy)aminosilane Catalyst 2]

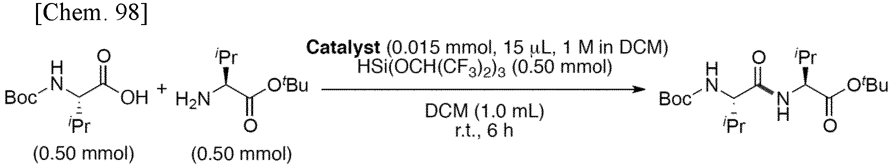

In a glove box, a flame-dried 5.0mL screw-cap vial was charged with a mixture of 1 MDCM solution of each of various aminosilane catalysts shown in Tables 1 to 3 below (referred to as "Catalyst" in the reaction formula above and in Tables 1 to 3 below) (0.015mL, 0.015mmol), HSi[OCH(CF₃)₂]₃ (265.1mg, 0.50mmol), Boc-L-Val-OH (108.6mg, 0.50mmol), L-Val-OtBu (86.6mg, 0.50mmol), and DCM(1.0mL), and the vial was sealed and removed from the glove box. The resulting mixture was stirred vigorously under an argon atmosphere at room temperature for 6 hours, and the reaction mixture was diluted with CHCl₃ (3.0mL), and transferred into a SiO₂ column with a pipette. The vial and pipette used were washed with CHCl₃ (12mL). The reaction mixture was purified with flash column chromatography (20 to 100% AcOEt in hexane), whereby the target compound Boc-L-Val-L-Val-OtBu was obtained. The yield and the diastereomer ratios of each target compound obtained for each of the various aminosilane catalysts used are shown in Tables 1 to 3 below.

**[Table 1]**

| Entry | Aminosilane catalyst | Yield (%) | Diastereomer ratio d.r. |
|---|---|---|---|
| 0 | None | 53 | >99:1 |
| 1 | 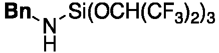 | 66 | >99:1 |
| 2 | 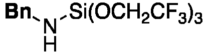 | 49 | >99:1 |
| 3 | 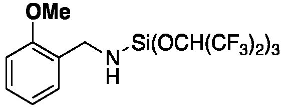 | 68 | >99:1 |
| 4 | 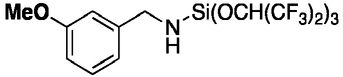 | 67 | >99:1 |
| 5 | 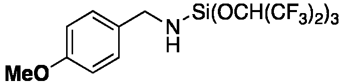 | 75 | >99:1 |
| 6 | 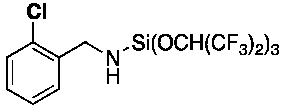 | 62 | >99:1 |
| 7 | 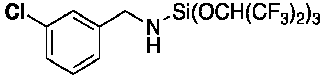 | 65 | >99:1 |
| 8 | 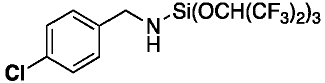 | 57 | >99:1 |
| 9 | 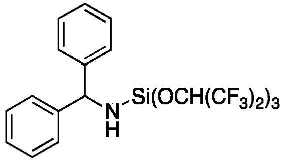 | 80 | >99:1 |
| 10 | 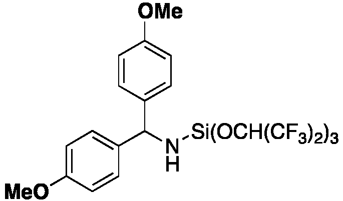 | | |

**[Table 2]**

| Entry | Aminosilane catalyst | Yield (%) | Diastereomer ratio d.r. |
|---|---|---|---|
| 11 | 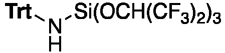 | 65 | >99:1 |
| 12 | 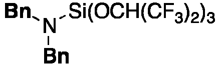 | 58 | >99:1 |
| 13 | 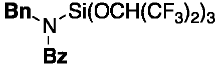 | 49 | >99:1 |
| 14 | 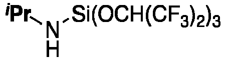 | 71 | >99:1 |
| 15 | 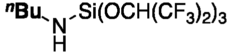 | 61 | >99:1 |
| 16 | 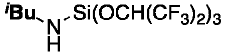 | 66 | >99:1 |
| 17 | 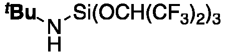 | 70 | >99:1 |
| 18 | 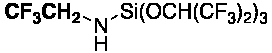 | 71 | >99:1 |
| 19 | 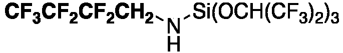 | 73 | >99:1 |
| 20 | 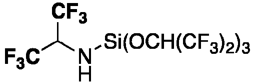 | | |
| 21 | 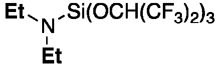 | 65 | >99:1 |
| 22 | 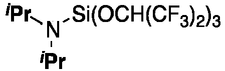 | 62 | >99:1 |

**[Table 3]**

| Entry | Aminosilane catalyst | yield (%) | Diastereomer ratio d.r. |
|---|---|---|---|
| 23 | 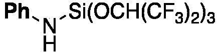 | 48 | >99:1 |
| 24 | 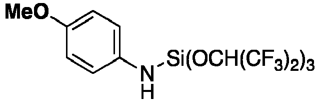 | 60 | >99:1 |
| 25 | 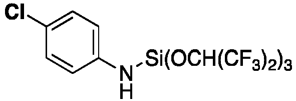 | 65 | >99:1 |
| 26 | 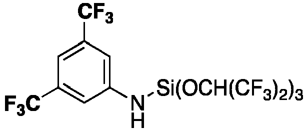 | 52 | >99:1 |
| 27 | 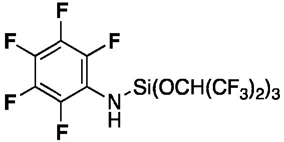 | 66 | >99:1 |
| 28 | 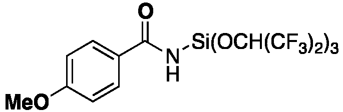 | 40 | >99:1 |
| 29 | 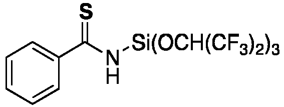 | 42 | >99:1 |
| 30 | 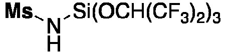 | 38 | >99:1 |
| 31 | 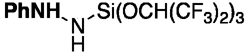 | 66 | >99:1 |
| 32 | 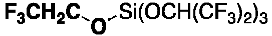 | 58 | >99:1 |
| 33 | 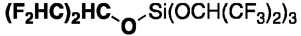 | 58 | >99:1 |
| 34 | 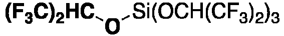 | 57 | >99:1 |

### [Example Group H: Amidation Reactions using Silane Compound (A) in the presence of Cesium Fluoride and Imidazole (without protective group)]

### *Example h1: synthesis of H-L-Phe-L-Ala-OtBu (in the presence of cesium fluoride and imidazole)

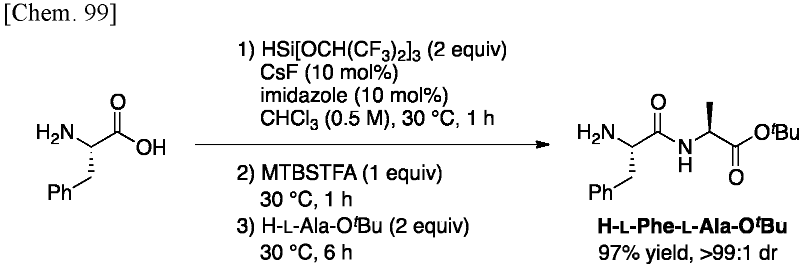

A 5-mL screw-cap vial heated and dried in a glove box was charged, under an argon atmosphere, with a stirrer (samarium-cobalt), L-phenylalanine (H-L-Phe-OH, 165.2mg, 1.0mmol), tris(hexafluoroisopropoxy)silane (HSi(OCH(CF₃)₂)₃, 530.2mg, 1.0mmol), cesium fluoride (CsF, 10 mol%), imidazole (10 mol%), and chloroform (0.5 mL), and sealed with a septum and a screw cap. The reaction vial was removed from the glove box, placed in an oil bath heated to 30°C, and then stirred vigorously for 1 hours. The screw cap was removed, and N-tert-butyldimethylsilyl-N-methyl trifluoroacetamide (MTBSTFA, 120.7mg, 0.5mmol) was added through the septum using a microsyringe, and the vial was sealed with the screw cap again, in an oil bath heated to 30°C stirred vigorously for 1 hours. The screw cap was removed again, through the septum using a microsyringe L-alanine -tert-butyl ester (H-L-Ala-Ot-Bu, 145.2mg, 1.0mmol) was added, and the vial was sealed with the screw cap again, in an oil bath heated to 30°C stirred vigorously for 6 hours. The reaction vial was removed from the oil bath and left to stand until it reached to room temperature, and the reaction mixture was diluted with a 1% methanol/chloroform mixture (3.0 mL), and the product was transferred to a previously prepared silica gel column (1% methanol/chloroform mixture) using a Pasteur pipette. This procedure was repeated three more times, and the screw cap, septum, and the Pasteur pipette used was washed with the same mixture. The reaction product was purified with silica gel column chromatography (1-5% methanol/chloroform mixture), whereby the desired dipeptide (H-L-Phe-L-Ala-Ot-Bu, 97%, >99:1dr) was obtained as a white solid.

### *Reference Example h1: synthesis of H-L-Phe-L-Ala-OtBu (in the absence of cesium fluoride and imidazole)

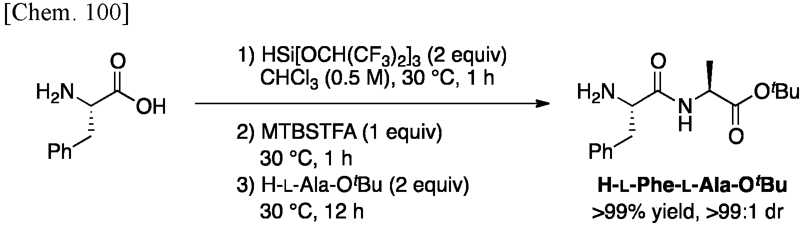

The synthesis was carried out in the same manner as in Example h1 except that cesium fluoride and imidazole were not used, and the heating time of the solution after the addition of H-L-Ala-Ot-Bu under stirring was changed from 6 hours to 12 hours, whereby the desired dipeptide (H-L-Phe-L-Ala-Ot-Bu, 99%, >99:1dr) was obtained as a white solid.

### *Example h2: synthesis of H-L-Leu-L-Ala-OtBu (in the presence of cesium fluoride and imidazole)

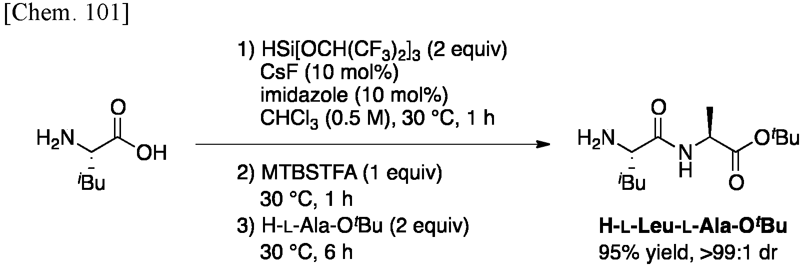

The synthesis was carried out in the same manner as in Example h1 except that L-leucine (H-L-Leu-OH, 1.0mmol) was used instead of L-phenylalanine, whereby the desired dipeptide (H-L-Leu-L-Ala-OtBu, 95%, >99:1dr) was obtained as a white solid.

### *Reference Example h2: synthesis of H-L-Leu-L-Ala-OtBu (in the absence of cesium fluoride and imidazole)

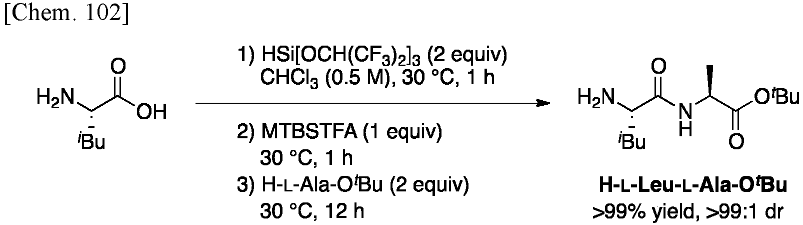

The synthesis was carried out in the same manner as in Example h2 except that cesium fluoride and imidazole were not used, and the heating time of the solution after the addition of H-L-Ala-Ot-Bu under stirring was changed from 6 hours to 12 hours, whereby the desired dipeptide (H-L-Leu-L-Ala-Ot-Bu, 99%, >99:1dr) was obtained as a white solid.

### *Example h3: synthesis of H-L-Ala-L-Ala-OtBu (in the presence of cesium fluoride and imidazole)

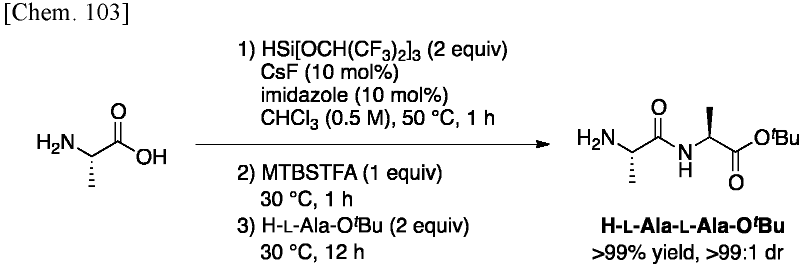

The synthesis was carried out in the same manner as in Example h1 except that L-alanine (H-L-Ala-OH, 1.0mmol) was used instead of L-phenylalanine, the temperature of the first heating for 1 hours was changed from 30°C to 50°C, and the heating time of the solution after the addition of H-L-Ala-Ot-Bu under stirring was changed from 6 hours to 12 hours, whereby the desired dipeptide (H-L-Ala-L-Ala-OtBu, 99%, >99:1dr) was obtained as a white solid.

### *Example h4: synthesis of H-L-Met-L-Ala-OtBu (in the presence of cesium fluoride and imidazole)

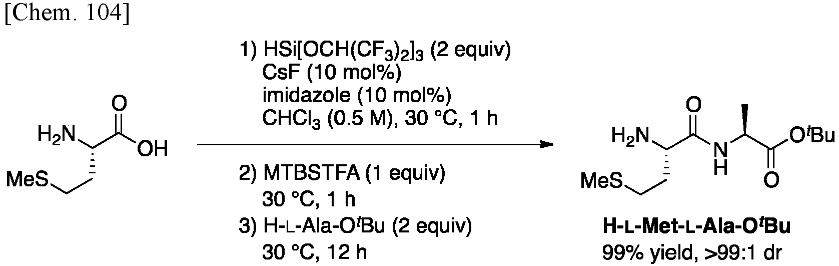

The synthesis was carried out in the same manner as in Example h1 except that L-methionine (H-L-Met-OH, 1.0mmol) was used instead of L-phenylalanine, and the heating time of the solution after the addition of H-L-Ala-Ot-Bu under stirring was changed from 6 hours to 12 hours, whereby the desired dipeptide (H-L-Met-L-Ala-OtBu, 99%, >99:1dr) was obtained as a white solid.

### [Example Group I: Amidation Reactions using Silane Compound (B) (1)]

The amide compounds were produced according to the method described in each of the following Example sections.

In the following examples, unless otherwise stated, diastereomeric or enantiomeric ratios were determined with ¹H-NMR analysis (measuring instrument: JEOL 400SS, measuring condition: 400MHz, solvent: CDCl₃).

### *Example i1: synthesis of H-Phe-Ala-Ot-Bu 1

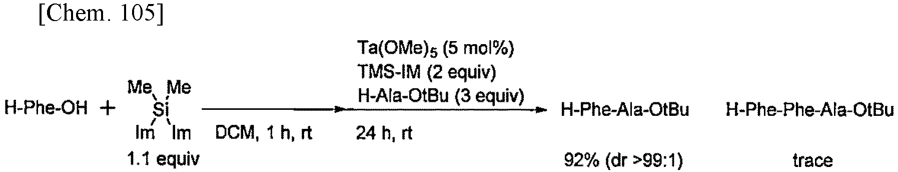

A 12-mL test tube was charged with phenylalanine (H-Phe-OH, 0.25mmol, 41.3mg) and dimethylsilyldiimidazole (DMSDIM, 0.275mmol, 52.8mg) suspended in dichloromethane (DCM, 1mL) in a glove box, and stirred under an argon atmosphere at room temperature. After one hour, Ta(OMe)₅(12.5µmol, 4.2mg), trimethylsilylimidazole (TMSIM, 0.50mmol, 73.4µL) and alanine tert-butyl ester (0.75mmol, 108.8mg) was added in a glove box, and was stirred at room temperature for 24 hours. The product was diluted with chloroform (5mL), and purified with silica gel chromatography (CHCl₃:MeOH=100:1), whereby title dipeptide H-Phe-Ala-Ot-Bu was obtained. The resulting quantity was 67.2mg, the yield was 92%, and the diastereomer ratios were dr>99:1. A trace of tripeptide H-Phe-Phe-Ala-Ot-Bu byproduct was observed.

¹H NMR (400 MHz, CDCl₃): δ 7.78 (br d, J =7.6 Hz, 1H, NH), 7.33-7.21 (m, 5H, PhH), 4.50-4.42 (quin, J =7.4 Hz, 1H, CHCH₃), 3.62 (dd, J =3.9 Hz, 9.2 Hz, 1H, CH₂PH), 3.22 (dd, J =3.9 Hz, 9.2 Hz, 1H, CH₂PH), 2.72 (m, 1H, CHCH₂), 1.59 (br s, 2H, NH₂CHPh), 1.45 (s, 9H, C(CH₃)₃), 1.36 (d, J =7.1 Hz, 3H, CHCH3). ¹³C NMR (100 MHz, CDCl3): δ 173.8, 172.2, 137.7, 129.3, 128.6, 126.7, 81.8, 56.2, 48.2, 40.9, 27.9, 18.6.

### *Example i2: synthesis of H-Phe-Ala-Ot-Bu 2

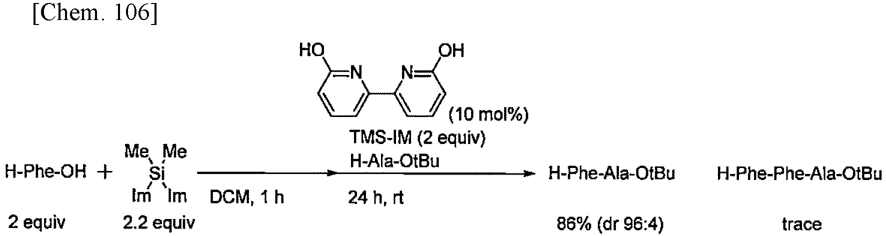

A 12-mL test tube was charged with phenylalanine (H-Phe-OH, 0.50mmol, 82.6mg), dimethylsilyldiimidazole (DMSDIM, 0.55mmol, 105.6mg) suspended in dichloromethane (DCM, 1mL) in a glove box, and stirred under an argon atmosphere at room temperature. After one hour, 2,2'-bipyridine-6,6'-diol (25µmol, 4.7mg), trimethylsilylimidazole (TMSIM, 0.50mmol, 73.4µL) and alanine tert-butyl ester (H-Ala-OtBu, 0.25mmol, 36.3mg) was added in a glove box, and was stirred at room temperature for 24 hours. The product was diluted with chloroform (5mL), and purified with silica gel chromatography (CHCl₃:MeOH=100:1), whereby title dipeptide H-Phe-Ala-Ot-Bu was obtained. The resulting quantity was 62.8mg, the yield was 86%, and the diastereomer ratios were dr96:4. A trace of tripeptide H-Phe-Phe-Ala-Ot-Bu byproduct was observed.

### *Example i3: synthesis of H-Phe-Ala-Ot-Bu 3

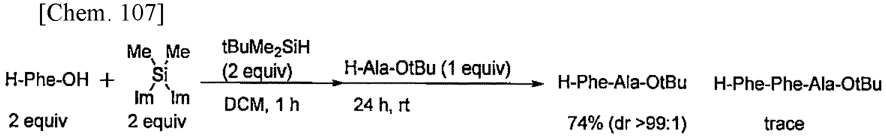

A 5-mL vial was charged with phenylalanine (H-Phe-OH, 0.50mmol, 82.6mg), dimethylsilyldiimidazole (DMSDIM, 0.55mmol, 105.6mg) and tert-butyl dimethyl silane (tBuMe₂SiH, 0.50mmol, 82.1µL) suspended in dichloromethane (DCM, 0.1mL) in a glove box, and stirred under an argon atmosphere at room temperature. After one hour, alanine tert-butyl ester (H-Ala-OtBu, 0.25mmol, 36.3mg) was added in a glove box, and was stirred at room temperature for 24 hours. The product was diluted with chloroform (5mL), and purified with silica gel chromatography (CHCl₃:MeOH=100:1), whereby title dipeptide H-Phe-Ala-Ot-Bu was obtained. The resulting quantity was 54.1mg, the yield was 74%, and the diastereomer ratios were dr>99: 1. A trace of tripeptide H-Phe-Phe-Ala-Ot-Bu byproduct was observed.

### *Example i4: synthesis of H-Phe-Ala-Ot-Bu 4

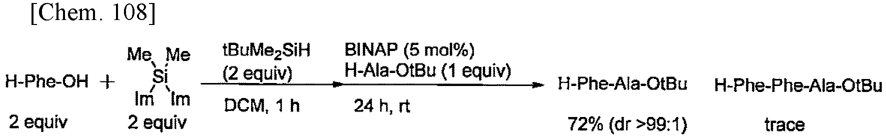

A 5-mL vial was charged with phenylalanine (H-Phe-OH, 0.50mmol, 82.6mg), dimethylsilyldiimidazole (DMSDIM, 0.55mmol, 105.6mg) and tert-butyl dimethyl silane (tBuMe₂SiH, 0.50mmol, 82.1µL) suspended in dichloromethane (DCM, 0.1mL) in a glove box, and stirred under an argon atmosphere at room temperature. After one hour, in a glove box 2,2'-bis(diphenyl phosphino)-1,1'-binaphthyl (BINAP, 12.5µmol, 7.8mg) and alanine tert-butyl ester (H-Ala-OtBu, 0.25mmol, 36.3mg) was added, and was stirred at room temperature for 24 hours. The product was diluted with chloroform (5mL), and purified with silica gel chromatography (CHCl₃:MeOH=100:1), whereby title dipeptide H-Phe-Ala-Ot-Bu was obtained. The resulting quantity was 52.6mg, the yield was 72%, and the diastereomer ratios were dr>99: 1. A trace of tripeptide H-Phe-Phe-Ala-Ot-Bu byproduct was observed.

### [Example Group J: Amidation Reactions using Silane Compound (B) (2)] *General Synthesis Procedure:

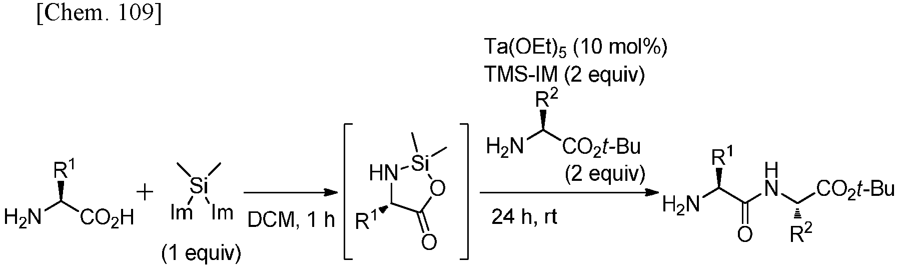

A 12-mL test tube was charged with a first amino acid AA¹ (0.5mmol, 1 equivalent) and dimethylsilyldiimidazole (DMSDIM, 0.5mmol, 1 equivalent) and suspended in dichloromethane (DCM, 1mL) in a glove box, and stirred under an argon atmosphere at room temperature. After one hour, Ta(OEt)₅ (10 mol%), trimethylsilylimidazole (TMSIM, 1.0mmol, 2 equivalents), and L-Ala-OtBu (1.0mmol, 2.0 equivalents) was added in a glove box, and was stirred at room temperature for 24 hours. The product was diluted with chloroform (5mL), and purified with silica gel chromatography (CHCl₃:MeOH=100:1), whereby an analytically pure product was obtained. In the reaction formula above, R¹ represents the side chain of the first amino acid AA¹.

### *Example j 1: synthesis of L-Phe-L-Ala-OtBu

The reaction was carried out in accordance with the General Synthesis Procedure except that L-phenylalanine (L-Phe) was used as the first amino acid AA¹, whereby the title compound L-Phe-L-Ala-OtBu was obtained. The yield was 95%, and the diastereomer ratio was dr>20:1.

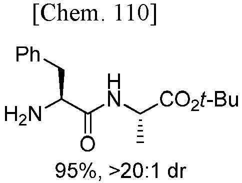

### *Example j2: synthesis of L-Val-L-Ala-OtBu

The reaction was carried out in accordance with the General Synthesis Procedure except that L-valine (L-Val) was used as the first amino acid AA¹, and the reaction temperature was changed to 50°C, whereby the title compound L-Val-L-Ala-OtBu was obtained. The yield was 72%, and the diastereomer ratio was dr>20: 1.

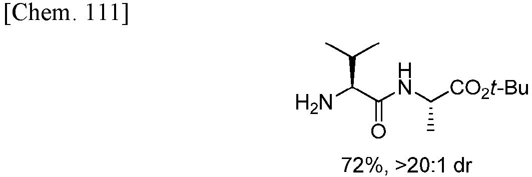

### *Example j3: synthesis of L-Ile-L-Ala-OtBu

The reaction was carried out in accordance with the General Synthesis Procedure except that L-isoleucine (L-Ile) was used as the first amino acid AA¹, and the reaction temperature was changed to 50°C, whereby the title compound L-Ile-L-Ala-OtBu was obtained. The yield was 78%, and the diastereomer ratio was dr>20: 1.

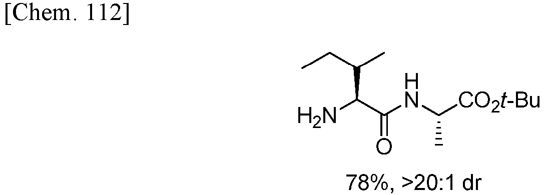

### *Example j4: synthesis of L-Leu-L-Ala-OtBu

The reaction was carried out in accordance with the General Synthesis Procedure except that L-leucine (L-Leu) was used as the first amino acid AA¹, and the reaction temperature was changed to 50°C, whereby the title compound L-Leu-L-Ala-OtBu was obtained. The yield was 89%, and the diastereomer ratio was dr>20: 1.

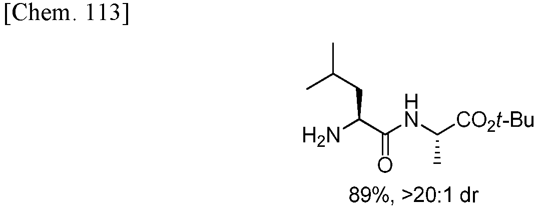

### *Example j5: synthesis of L-Met-L-Ala-OtBu

The reaction was carried out in accordance with the General Synthesis Procedure except that L-methionine (L-Met) was used as the first amino acid AA¹, whereby the title compound L-Met-L-Ala-OtBu was obtained. The yield was 86%, and the diastereomer ratio was dr>20:1.

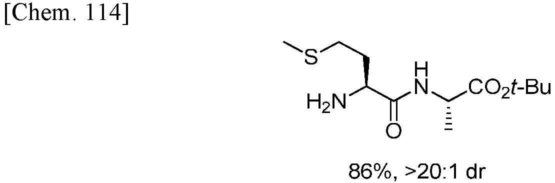

### *Example j6: synthesis of Bn-L-Cys-L-Ala-OtBu

The reaction was carried out in accordance with the General Synthesis Procedure except that benzyl -L-cysteine (Bn-L-Cys) was used as the first amino acid AA¹, whereby the title compound Bn-L-Cys-L-Ala-OtBu was obtained. The yield was 56%, and the diastereomer ratio was dr>20:1.

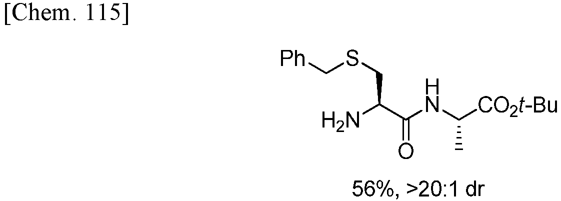

### *Example j7: synthesis of Boc-L-Trp-L-Ala-OtBu

The reaction was carried out in accordance with the General Synthesis Procedure except that L-tryptophan whose side chain's amino group was protected with a Boc group (Boc-L-Trp) was used as the first amino acid AA¹, whereby the title compound Boc-L-Trp-L-Ala-OtBu was obtained. The yield was 91%, and the diastereomer ratio was dr>20: 1.

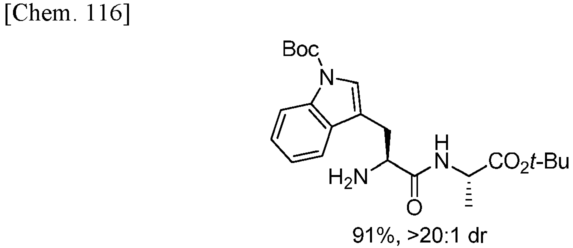

### *Example j8: synthesis of tBuO-L-Tyr-L-Ala-OtBu

The reaction was carried out in accordance with the General Synthesis Procedure except that L-tyrosine whose side chain's hydroxyl group was protected with a t-butyl group (tBuO-L-Tyr) was used as the first amino acid AA¹, whereby the title compound tBuO-L-Tyr-L-Ala-OtBu was obtained. The yield was 90%, and the diastereomer ratio was dr>20: 1.

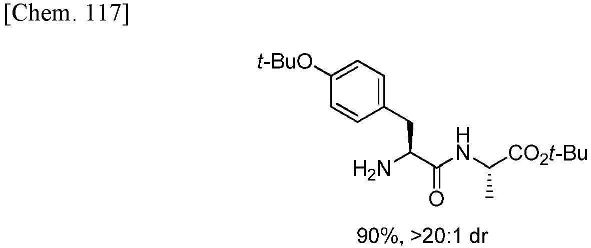

### *Example j9: synthesis of tBuO-L-Ser-L-Ala-OtBu

The reaction was carried out in accordance with the General Synthesis Procedure except that L-serine whose side chain's hydroxyl group was protected with a t-butyl group (tBuO-L-Ser) was used as the first amino acid AA¹, whereby the title compound tBuO-L-Ser-L-Ala-OtBu was obtained. The yield was 71%, and the diastereomer ratio was dr>20: 1.

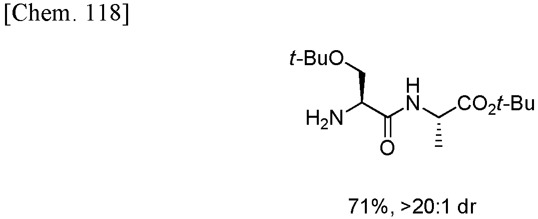

### *Example j 10: synthesis of tBuO-L-Thr-L-Ala-OtBu

The reaction was carried out in accordance with the General Synthesis Procedure except that L-threonine whose side chain's hydroxyl group was protected with a t-butyl group (tBuO-L-Thr) was used as the first amino acid AA¹, whereby the title compound tBuO-L-Thr-L-Ala-OtBu was obtained. The yield was 95%, and the diastereomer ratio was dr>20: 1.

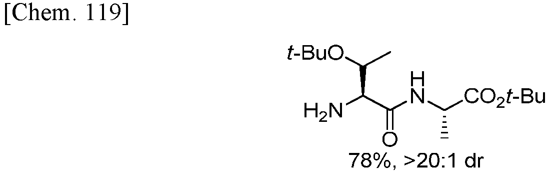

### *Example j 11: synthesis of PG-L-Lys-L-Ala-OtBu

The reaction was carried out in accordance with the General Synthesis Procedure except that L-lysine whose side chain's amino group was protected with a protective group PG (PG-L-Lys) was used as the first amino acid AA¹, whereby the title compounds PG-L-Phe-L-Ala-OtBu were obtained. The protective group PG was changed from a Boc group, Cbz group, and Trt group. For Boc-L-Phe-L-Ala-OtBu, the yield was 53%, and the diastereomer ratio was dr>20:1; for Cbz-L-Phe-L-Ala-OtBu, the yield was 55%, and the diastereomer ratio was dr>20:1; and for Trt-L-Phe-L-Ala-OtBu, the yield was 71%, and the diastereomer ratio was dr>20:1.

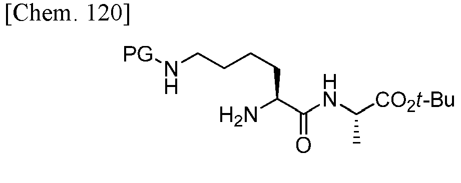

| | | |
|---|---|---|
| PG: | Boc | 53%, >20:1 dr |
| | Cbz | 55%, >20:1 dr |
| | Trt | 71%, >20:1 dr |

### *Example j 12: synthesis of tBuO-L-Glu-L-Ala-OtBu

The reaction was carried out in accordance with the General Synthesis Procedure except that L-glutamic acid whose side chain's carbonic acid group was protected with a t-butyl group (tBuO-L-Glu) was used as the first amino acid AA¹, whereby the title compound tBuO-L-Glu-L-Ala-OtBu was obtained. The yield was 88%, and the diastereomer ratio was dr>20:1.

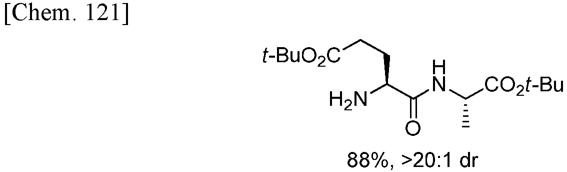

### *Example j 13: synthesis of tBuO-L-Asp-L-Ala-OtBu

The reaction was carried out in accordance with the General Synthesis Procedure except that L-asparaginic acid whose side chain's carbonic acid group was protected with a t-butyl group (tBuO-L-Asp) was used as the first amino acid AA¹, whereby the title compound tBuO-L-Asp-L-Ala-OtBu was obtained. The yield was 90%, and the diastereomer ratio was dr>20:1.

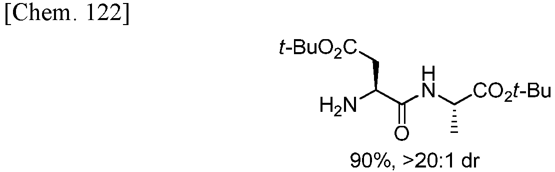

### *Example j 14: synthesis of β-Ala-L-Ala-OtBu

The reaction was carried out in accordance with the General Synthesis Procedure except thatβ-alanine (β-Ala) was used as the first amino acid AA¹, whereby the title compound β-Ala-L-Ala-OtBu was obtained. The yield was 53%.

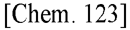

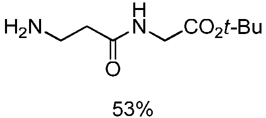

### INDUSTRIAL APPLICABILITY

The present invention can be widely used for the synthesis of amide compounds in various industrial fields, and therefore has high utility value.

## Claims

1. A method of producing, from a compound represented by general formula (1-1) and a compound represented by general formula (1-2), an amide compound represented by general formula (1-3), comprising:
causing a reaction between the compound represented by general formula (1-1) and the compound represented by general formula (1-2) in the presence of a silane compound represented by general formula (B). 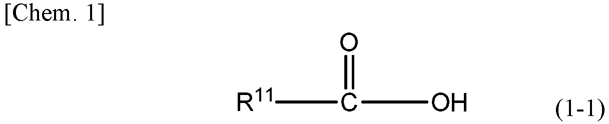
In general formula (1-1),
R¹¹ represents a monovalent hydrocarbon group or heterocyclic group that may have one or more substituents or a monovalent group formed by linking, optionally via a linking group, two or more multivalent hydrocarbon and/or heterocyclic groups that each may have one or more substituents. 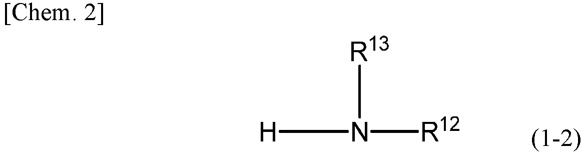
In general formula (1-2),
R¹² represents a monovalent hydrocarbon group or heterocyclic group that may have one or more substituents or a monovalent group formed by linking, optionally via a linking group, two or more multivalent hydrocarbon and/or heterocyclic groups that each may have one or more substituents, and
R¹³ represents a hydrogen atom, carboxyl group, or hydroxyl group, or a monovalent hydrocarbon group or heterocyclic group that may have one or more substituents and may be bound to the nitrogen atom via a linking group, or
R¹² and R¹³ may be bound to each other to form, together with the nitrogen atom to which R¹² and R¹³ bind, a hetero ring that may have one or more substituents. 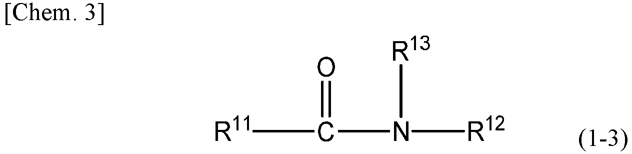
In general formula (1-3), each symbol represents the same definition as that of the same symbol in general formulae (1-1) and (1-2) above. 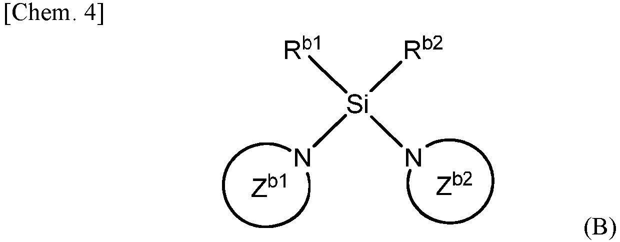
In general formula (B),
R^{b1}and R^{b2}, independently of each other, represent a hydrogen atom or an alkyl group or alkoxy group having 1 to 10 carbon atoms or an aryl group, aralkyl group, alkyl aryl group, aryloxy group, aralkyloxy group, or alkyl aryloxy group having 6 to 12 carbon atoms, wherein the alkyl, alkoxy, aryl, aralkyl, alkyl aryl, aryloxy, aralkyloxy, or alkyl aryloxy group may have one or more substituents, and
Z^{b1}and Z^{b2}, independently of each other, represent a 5- to 7-membered heterocyclic group that contains at least one nitrogen atom as a ring-constituting atom and may have one or more substituents.

2. A method of producing, from a compound represented by general formula (2-1), an amide compound represented by general formula (2-2), comprising:
causing an intramolecular reaction in the compound represented by general formula (2-1) in the presence of a silane compound represented by general formula (B). 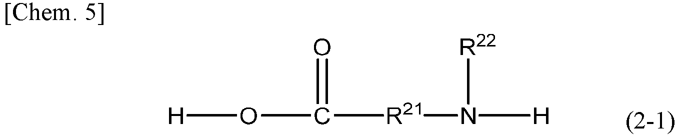
In general formula (2-1),
R²¹ represents a divalent hydrocarbon group or heterocyclic group that may have one or more substituents or a divalent group formed by linking, optionally via a linking group, two or more multivalent hydrocarbon and/or heterocyclic groups that each may have one or more substituents, and
R²² represents a hydrogen atom, carboxyl group, or hydroxyl group, or a monovalent hydrocarbon group or heterocyclic group that may have one or more substituents and may be bound to the nitrogen atom via a linking group, or
R²¹ and R²² may be bound to each other to form, together with the nitrogen atom to which R²¹ and R²² bind, a hetero ring that may have one or more substituents. 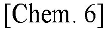 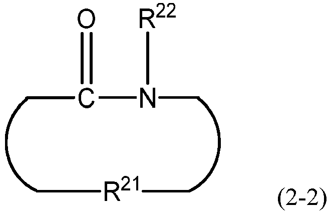
In general formula (2-2), each symbol represents the same definition as that of the same symbol in general formula (2-1) above. 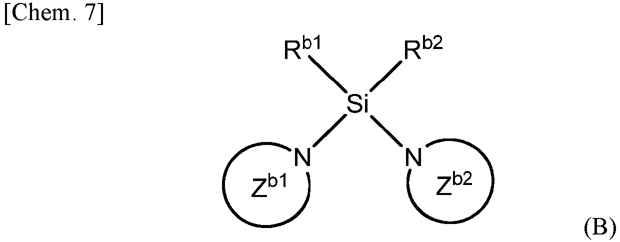
In general formula (B),
R^{b1}and R^{b2}, independently of each other, represent a hydrogen atom or an alkyl group or alkoxy group having 1 to 10 carbon atoms or an aryl group, aralkyl group, alkyl aryl group, aryloxy group, aralkyloxy group, or alkyl aryloxy group having 6 to 12 carbon atoms, wherein the alkyl, alkoxy, aryl, aralkyl, alkyl aryl, aryloxy, aralkyloxy, or alkyl aryloxy group may have one or more substituents, and
Z^{b1}and Z^{b2}, independently of each other, represent a 5- to 7-membered heterocyclic group that contains at least one nitrogen atom as a ring-constituting atom and may have one or more substituents.

3. A method of producing, from a compound represented by general formula (3-1) and a compound represented by general formula (3-2), an amide compound represented by general formula (3-3), comprising:
causing a reaction between the compound represented by general formula (3-1) and the compound represented by general formula (3-2) in the presence of a silane compound represented by general formula (B). 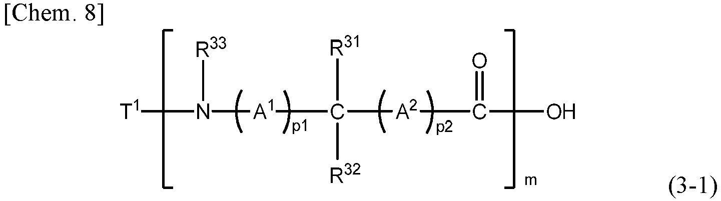
In general formula (3-1),
R³¹ and R³², independently of each other, represent a hydrogen atom, halogen atom, hydroxyl group, carboxyl group, nitro group, cyano group, thiol group, or, a monovalent hydrocarbon group or heterocyclic group that may have one or more substituents and may be bound to the carbon atom via a linking group, and
R³³ represents a hydrogen atom, carboxyl group, hydroxyl group, or, a monovalent hydrocarbon group or heterocyclic group that may have one or more substituents and may be bound to the nitrogen atom via a linking group, or
R³¹ and R³³ may be bound to each other to form, together with the carbon atom to which R³¹ binds and the nitrogen atom to which R³³ binds, a hetero ring that may have one or more substituents,
A¹ and A², independently of each other, represent a divalent aliphatic hydrocarbon group that may have one or more substituents having 1 to 3 carbon atoms,
T¹ represents a hydrogen atom or a monovalent substituent,
p1 and p2, independently of each other, represent an integer of 0 or 1, and
m represents an integer of equal to or greater than 1 corresponding to the number of the structure units parenthesized with [ ], provided that when m is equal to or greater than 2, then the two or more structure units in [ ] may be either identical to each other or different from each other. 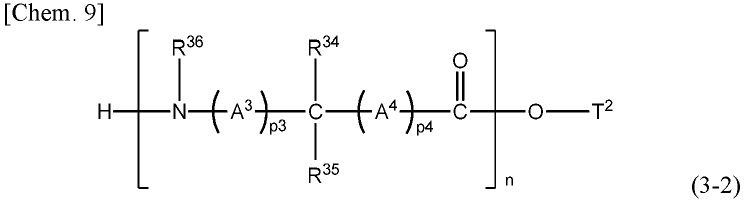
In general formula (3-2),
R³⁴and R³⁵, independently of each other, represent a hydrogen atom, halogen atom, hydroxyl group, carboxyl group, nitro group, cyano group, thiol group, or, a monovalent hydrocarbon group or heterocyclic group that may have one or more substituents and may be bound to the carbon atom via a linking group,
R³⁶ represents a hydrogen atom, carboxyl group, hydroxyl group, or, a monovalent hydrocarbon group or heterocyclic group that may have one or more substituents and may be bound to the nitrogen atom via a linking group,
R³⁴ and R³⁶ may be bound to each other to form, together with the carbon atom to which R³⁴ binds and the nitrogen atom to which R³⁶ binds, a hetero ring that may have one or more substituents,
A³and A⁴, independently of each other, represent a divalent aliphatic hydrocarbon group that may have one or more substituents having 1 to 3 carbon atoms,
T² represents a hydrogen atom or a monovalent substituent,
p3 and p4, independently of each other, represent an integer of 0 or 1, and
n represents an integer of equal to or greater than 1 corresponding to the number of the structure units parenthesized with [ ], provided that when n is equal to or greater than 2, then the two or more structure units in [ ] may be either identical to each other or different from each other. 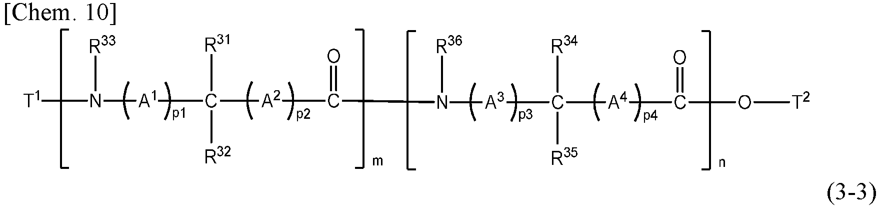
In general formula (3-3), each symbol represents the same definition as that of the same symbol in general formulae (3-1) and (3-2) above. 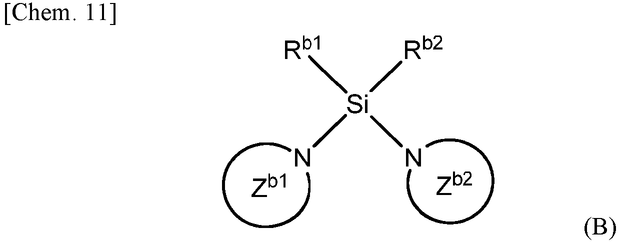
In general formula (B),
R^{b1}and R^{b2}, independently of each other, represent a hydrogen atom or an alkyl group or alkoxy group having 1 to 10 carbon atoms or an aryl group, aralkyl group, alkyl aryl group, aryloxy group, aralkyloxy group, or alkyl aryloxy group having 6 to 12 carbon atoms, wherein the alkyl, alkoxy, aryl, aralkyl, alkyl aryl, aryloxy, aralkyloxy, or alkyl aryloxy group may have one or more substituents, and
Z^{b1}and Z^{b2}, independently of each other, represent a 5- to 7-membered heterocyclic group that contains at least one nitrogen atom as a ring-constituting atom and may have one or more substituents.

4. The method according to any one of claims 1 to 3, wherein the reaction is carried out in the presence of, in addition to the silane compound represented by general formula (B), a second silane compound which is different from the silane compound represented by general formula (B).

5. The method according to claim 4, wherein the second silane compound is at least one compound selected from compounds represented by general formulae (C1) to (C4). 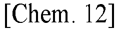 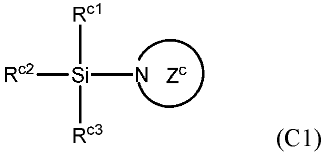 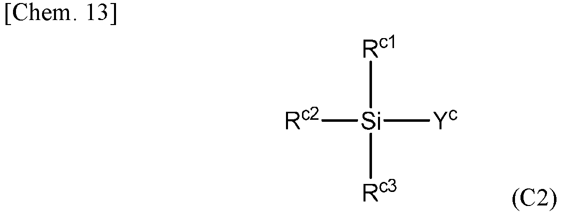 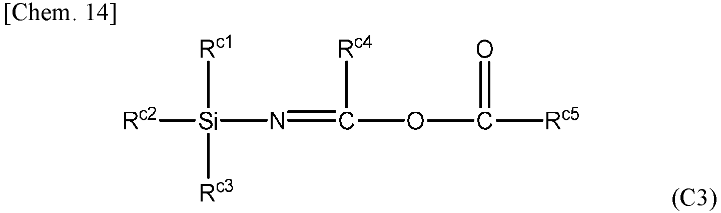 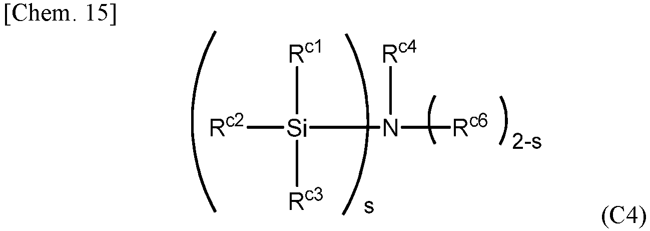
In general formulae (C1) to (C4),
R^{c1} to R^{c3}, independently of each other, represent a hydrogen atom or a linear- or branched-chain alkyl group or alkoxy group that contains 1 to 10 carbon atoms and may have one or more substituents, provided that 0 or 1 of R^{c1} to R^{c3} is a hydrogen atom,
R^{c4} and R^{c5}*,* independently of each other, represent a linear- or branched-chain alkyl group or alkoxy group that contains 1 to 10 carbon atoms and may have one or more substituents,
Z^{c} represents a 5- to 10-membered heterocyclic group that contains at least one nitrogen atom as a ring-constituting atom and may have one or more substituents,
Y^{c} represents a hydrogen atom or halogen group,
R^{c6} represents a linear- or branched-chain alkyl group, alkoxy group, or alkyl carbonyl group that contains 1 to 10 carbon atoms and may have one or more substituents, and
s represents an integer of 1 or 2, provided that when s is 2, then R^{c6} is absent.

6. The method according to any one of claims 1 to 5, wherein the reaction is in the presence of an aminosilane catalyst.

7. The method according to claim 6, wherein the aminosilane catalyst is at least one compound selected from compounds represented by general formula (D). 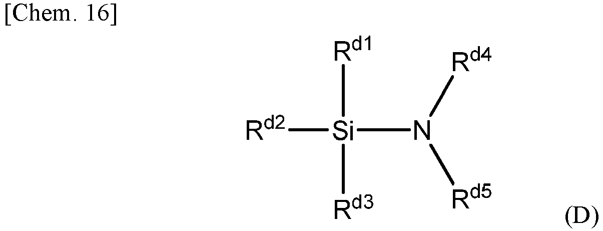
In general formula (D),
R^{d1} to R^{d3}, independently of each other, represent a hydrogen atom or a linear- or branched-chain alkyl group or alkoxy group that may have one or more substituents, provided that 0 or 1 of R^{d1} to R^{d3} is a hydrogen atom, and
R^{d4} and R^{d5}, independently of each other, represent a hydrogen atom or an alkyl group, aryl group, alkyl aryl group, or aryl alkyl group that may have one or more substituents, provided that 0 or 1 of R^{d4} and R^{d5} is a hydrogen atom.

8. The method according to any one of claims 1 to 7, wherein the reaction is carried out in the presence of a Lewis acid catalyst.

9. The method according to claim 8, wherein the Lewis acid catalyst is a metal compound containing at least one metal selected from the group consisting of titanium, zirconium, hafnium, tantalum, and niobium.

10. The method according to any one of claims 1 to 9, wherein the reaction is carried out in the presence of a phosphorus compound.

11. The method according to claim 10, wherein the phosphorus compound is either a phosphine compound or phosphate compound.

12. The method according to any one of claims 1 to 11, wherein the reaction is carried out as a batch reaction or a flow reaction.
